# EUROPEAN PATENT APPLICATION

(11) **EP 4 837 534 A1**
(43) Date of publication of application: **11.11.2026**
(21) Application number: 25779221.8
(22) Date of filing: 21.03.2025
(51) Int. Cl.: A61B 34/10

(54) **INTERVENTIONAL MEDICAL SYSTEM AND CONTROL METHOD THEREFOR**

(30) Priority: 27.03.2024 CN 202410361767; 27.03.2024 CN 202410361493
(71) Applicant: Wuhan United Imaging Surgical Co., Ltd., Wuhan, Hubei 430206 (CN)
(72) Inventor: ZHANG, Qiao, Wuhan, Hubei 430206 (CN); SHI, Danjing, Wuhan, Hubei 430206 (CN); GAO, Hao, Wuhan, Hubei 430206 (CN); DU, Leran, Wuhan, Hubei 430206 (CN); SONG, Weifan, Wuhan, Hubei 430206 (CN); ZHANG, Yang, Wuhan, Hubei 430206 (CN)
(74) Representative: Wang, Bo
(86) International application number: PCT/CN2025/084137
(87) International publication number: WO 2025/201205

(57) **Abstract**

The present disclosure relates to a system for interventional medicine and a control method thereof. The system includes an interventional surgical robot and a processor. The interventional surgical robot includes a manipulator arm and an end effector. The processor is configured to obtain surgical planning information in an interventional mode, and plan a movement path of the end effector in the interventional mode based on the surgical planning information. The interventional mode is an online mode or an offline mode. The surgical planning information at least includes a plurality of operation positions.

## Description

### CROSS-REFERENCE TO RELATED APPLICATIONS

This application claims priority to Chinese Patent Application No. 202410361767.0, filed on March 27, 2024 and Chinese Patent Application No. 202410361493.5, filed on March 27, 2024, the entire contents of each of which are hereby incorporated by reference.

### TECHNICAL FIELD

The present disclosure generally relates to the field of medicine, and in particular to systems for interventional medicine and methods for controlling the systems for interventional medicine.

### BACKGROUND

Interventional surgery is a relatively common technique in modern surgery, particularly in the field of minimally invasive surgery. For example, in interventional puncture surgery, a puncture needle is inserted into a soft tissue lesion target under guidance of imaging and other methods to complete operations such as drug delivery, biopsy, local anesthesia, radiotherapy, and ablation therapy. Interventional surgery is widely used in the diagnosis and treatment of organs and tissues such as the prostate, lung, liver, kidney, and spine.

Traditional interventional surgery has drawbacks such as requiring multiple scans for verification, long surgical duration, high technical requirements for doctors, and poor repeatability. Furthermore, traditional interventional surgery also has many problems such as direct exposure of doctors to radiation and limited operating space. Currently, an end effector carried by an interventional surgical robot can assist or replace a doctor in performing interventional surgery. However, interventional surgery typically requires performing interventional operations at a plurality of operation positions (also referred to as interventional positions, i.e., needle tracts). Planning a movement path of the end effector is relatively difficult. Furthermore, a medical instrument (for example, a puncture needle) may remain in a patient's body after an operation.

Therefore, it is desirable to provide systems for interventional medicine and methods for controlling the systems for interventional medicine to improve the success rate of the interventional surgical robot assisting in or performing interventional surgery.

### SUMMARY

One or more embodiments of the present disclosure provide a system for interventional medicine. The system for interventional medicine includes an interventional surgical robot and a processor. The interventional surgical robot includes a manipulator arm and an end effector. The processor is configured to obtain surgical planning information in an interventional mode. The interventional mode is an online mode or an offline mode. The surgical planning information at least includes a plurality of operation positions. The processor is further configured to plan a movement path of the end effector in the interventional mode based on the surgical planning information.

One or more embodiments of the present disclosure provide a method implemented on a system for interventional medicine. The system for interventional medicine includes an interventional surgical robot and a processor. The interventional surgical robot includes a manipulator arm and an end effector. The method includes obtaining surgical planning information in an interventional mode. The interventional mode is an online mode or an offline mode. The surgical planning information at least includes a plurality of operation positions. The method further includes planning a movement path of the end effector in the interventional mode based on the surgical planning information.

One or more embodiments of the present disclosure provide a control method for a system for interventional medicine. The system for interventional medicine includes an interventional surgical robot and a processor. The interventional surgical robot includes a manipulator arm and an end effector. The method includes determining an interventional mode of the system for interventional medicine; configuring the end effector based on the interventional mode; determining surgical planning information in the interventional mode; and planning a movement path of the end effector in the interventional mode based on the surgical planning information.

One or more embodiments of the present disclosure provide a method for path planning of an interventional surgical robot. The interventional surgical robot includes a manipulator arm and an end effector connected to an end of the manipulator arm. The method includes obtaining a plurality of operation positions from surgical planning information, and determining a plurality of subregions for path planning extending along a first direction. The plurality of subregions for path planning fully cover the plurality of operation positions. Each of the plurality of subregions for path planning covers at least a portion of the plurality of operation positions. The method further includes planning a movement path of the end effector based on the subregions for path planning.

One or more embodiments of the present disclosure provide a system for interventional medicine. A data connection exists between the system for interventional medicine and a medical imaging system. The medical imaging system includes a scanning device and a scanning table. The system for interventional medicine includes an interventional surgical robot and a processor. The interventional surgical robot includes a manipulator arm and an end effector. The processor is configured to obtain first position information of the scanning device, second position information of the scanning table, and surgical planning information. The surgical planning information includes a plurality of operation positions and a plurality of working channels corresponding to the plurality of operation positions. The processor is further configured to plan a movement path of the end effector based on the first position information, the second position information, and the surgical planning information. The processor is further configured to determine target surgical planning information in response to a successful planning of the movement path of the end effector.

One or more embodiments of the present disclosure provide a system for interventional medicine. A data connection exists between the system for interventional medicine and a medical imaging system. The medical imaging system includes a scanning device and a scanning table. The system for interventional medicine includes an interventional surgical robot and a processor. The interventional surgical robot includes a manipulator arm and an end effector. The processor is configured to obtain first position information of the scanning device, second position information of the scanning table, and target surgical planning information to be executed. The target surgical planning information includes a plurality of target operation positions and a plurality of target working channels corresponding to the plurality of target operation positions. The processor is further configured to sequentially plan and execute an execution subpath between each set of adjacent target operation positions. At least a portion of each of the execution subpaths is executed within a scanning chamber of the scanning device. The sequentially planning and executing the execution subpath between each set of adjacent target operation positions includes: in response to an inclination of a target working channel corresponding to a target operation position included in the execution subpath relative to a second direction, controlling the scanning device, the scanning table, and the manipulator arm to operate in coordination to execute the execution subpath.

One or more embodiments of the present disclosure provide a computer-readable storage medium. The storage medium stores computer instructions. When a computer reads the computer instructions in the storage medium, the computer executes the method for the system for interventional medicine in the embodiments of the present disclosure.

### BRIEF DESCRIPTION OF THE DRAWINGS

The present disclosure is further described in an illustrative manner by way of exemplary embodiments. The exemplary embodiments are described in detail with reference to the accompanying drawings. The embodiments are not limiting. In the embodiments, the same reference numerals denote the same structures.
FIG. 1 is a schematic diagram of an exemplary system for interventional medicine according to some embodiments of the present disclosure.
FIG. 2 and FIG. 3 are schematic diagrams of exemplary systems for interventional medicine according to some embodiments of the present disclosure.
FIG. 4 is a block diagram of an exemplary system for interventional medicine according to some embodiments of the present disclosure.
FIG. 5 is a flowchart of an exemplary process for path planning of an interventional surgical robot in a system for interventional medicine according to some embodiments of the present disclosure.
FIG. 6 is a flowchart of an exemplary process for path planning of an interventional surgical robot in a system for interventional medicine according to some embodiments of the present disclosure.
FIG. 7A and FIG. 7B are schematic diagrams of an exemplary sorting of a plurality of operation positions according to some embodiments of the present disclosure.
FIG. 8 is a schematic diagram of an exemplary sorting of a plurality of operation positions according to some embodiments of the present disclosure.
FIG. 9 is a schematic diagram of an exemplary end effector of an interventional surgical robot corresponding to the plurality of operation positions in FIG. 8 according to some embodiments of the present disclosure.
FIG. 10A is a schematic diagram of an exemplary sorting of a plurality of operation positions according to some embodiments of the present disclosure.
FIG. 10B is a schematic diagram of an exemplary end effector of an interventional surgical robot corresponding to the plurality of operation positions in FIG. 10A according to some embodiments of the present disclosure.
FIG. 11 is a flowchart of an exemplary process for subpath planning in a system for interventional medicine according to some embodiments of the present disclosure.
FIG. 12 is a schematic diagram of an exemplary overall path planning in a system for interventional medicine according to some embodiments of the present disclosure.
FIG. 13 is a schematic diagram of an exemplary end effector retraction of an interventional surgical robot according to some embodiments of the present disclosure.
FIG. 14 is a schematic diagram of an exemplary front view when an end effector of an interventional surgical robot performs end effector retraction according to some embodiments of the present disclosure.
FIG. 15 is a schematic diagram of an exemplary retreat direction when an end effector of an interventional surgical robot performs end effector retraction according to some embodiments of the present disclosure.
FIG. 16 is a flowchart of an exemplary process for execution subpath planning in a system for interventional medicine according to some embodiments of the present disclosure.
FIG. 17 is a flowchart of an exemplary process for overall path planning in a system for interventional medicine according to some embodiments of the present disclosure.
FIG. 18 is a flowchart of an exemplary overall workflow of a system for interventional medicine during an interventional surgical procedure according to some embodiments of the present disclosure.
FIG. 19 is a schematic diagram of an exemplary overall path planning in a system for interventional medicine according to some embodiments of the present disclosure.
FIG. 20 is a flowchart of an exemplary process for path planning of an interventional surgical robot in a system for interventional medicine according to some embodiments of the present disclosure.
FIG. 21 is a flowchart of an exemplary process for path planning of an interventional surgical robot in a system for interventional medicine according to some embodiments of the present disclosure.
FIG. 22A is a schematic diagram of an exemplary path planning failure caused by a collision between a manipulator arm end model and a patient model according to some embodiments of the present disclosure.
FIG. 22B is a schematic diagram of an exemplary adjustment of a needle path after the path planning failure in FIG. 22A according to some embodiments of the present disclosure.
FIG. 23A is a schematic diagram of an exemplary path planning failure caused by a collision between a manipulator arm end model and a scanning device model according to some embodiments of the present disclosure.
FIG. 23B is a schematic diagram of an exemplary adjustment of a needle path after the path planning failure in FIG. 23A according to some embodiments of the present disclosure.
FIG. 24A is a schematic diagram of an exemplary path planning failure caused by a collision between a manipulator arm end model and a surgical instrument model according to some embodiments of the present disclosure.
FIG. 24B is a schematic diagram of an exemplary adjustment of a needle path after the path planning failure in FIG. 24A according to some embodiments of the present disclosure.
FIG. 25 is a schematic diagram illustrating an exemplary needle path inclined in a craniocaudal direction without tilting a computed tomography (CT) gantry according to some embodiments of the present disclosure.
FIG. 26 is a schematic diagram of an exemplary calculation of a CT gantry inclination angle according to some embodiments of the present disclosure.
FIG. 27 is a schematic diagram of an exemplary calculation of a vertical table movement amount of a CT table according to some embodiments of the present disclosure.
FIG. 28 is a schematic diagram of an exemplary calculation of a horizontal table movement amount of a CT table according to some embodiments of the present disclosure.
FIG. 29 is a flowchart of an exemplary control method for a system for interventional medicine according to some embodiments of the present disclosure.

### DETAILED DESCRIPTION

In order to more clearly illustrate the technical solutions of the embodiments of the present disclosure, the accompanying drawings to be used in the description of the embodiments are briefly introduced below. It is apparent that the accompanying drawings in the following description are merely some examples or embodiments of the present disclosure. For those skilled in the art, the present disclosure can also be applied to other similar scenarios based on these accompanying drawings without making inventive efforts. Unless obviously obtained from the context or the context illustrates otherwise, the same numeral in the drawings refers to the same structure or operation.

It should be understood that the terms 'system', 'device', 'unit', and/or 'module' as used herein are a manner for distinguishing different components, elements, parts, portions, or assemblies at different levels. However, if other expressions can achieve the same purpose, the terms can be replaced by other expressions.

As shown in the present disclosure and the claims, unless the context clearly indicates otherwise, the terms 'a', 'an', and/or 'the' do not specifically refer to a singular form, but may also include a plural form. In general, the terms "comprise," "includes," and/or "comprising," "include," "includes," and/or "including," merely prompt to include steps and elements that have been clearly identified, and these steps and elements do not constitute an exclusive listing. The methods or devices may also include other steps or elements.

Flowcharts are used in the present disclosure to illustrate operations performed by a system according to embodiments of the present disclosure. It should be understood that preceding or subsequent operations are not necessarily performed in the exact order. Rather, the various steps may be processed in reverse order or simultaneously. Meanwhile, other operations may also be added to these processes, or one or more operations may be removed from these processes.

Interventional surgery refers to introducing a surgical instrument (for example, a puncture needle) into a patient's body for diagnosing and performing local treatment on the patient. In some embodiments, interventional multi-needle puncture surgery is performed within a scanning chamber (also referred to as a scanning bore) of a scanning device under the guidance of medical imaging, for example, within a scanning chamber of a scanning device such as a Computed Tomography (CT) device, a Magnetic Resonance Imaging (MRI) device, or the like.

The interventional mode refers to a manner for performing interventional surgery. The interventional mode includes an online mode and an offline mode. The online mode refers to that interventional surgery requires real-time guidance from a medical imaging device, that is, during the interventional surgical procedure, under the guidance of real-time scanned images from the medical imaging device, a doctor actively controls a puncture or a manipulator arm controls the puncture. The offline mode refers to that interventional surgery does not require real-time guidance from a medical imaging device, that is, during the interventional surgical procedure, a doctor performs a manual puncture with the aid of an offline end effector, and the medical imaging device is used for pre-surgical simulation, that is, for path planning of the surgery. Taking a CT device as an example, in the online mode or the offline mode, after performing a CT scan on a patient's lesion position, it is necessary to perform a pre-surgical simulation to determine a needle path position and to plan a path for a surgical robot. In the online mode, during the surgical procedure, the end effector of the manipulator arm holds a puncture needle and moves to an interventional puncture position (entry point) according to a planned path. The CT device performs real-time exposure. A doctor outside a CT room observes an entire needle insertion process through real-time exposure of the CT device. The doctor actively controls the end effector (for example, an auxiliary positioner, an end gripper, or the like) at the end of the manipulator arm to cause the puncture needle to perform a puncture, or the manipulator arm automatically controls the end effector to cause the puncture needle to perform a puncture. In the offline mode, during the surgical procedure, a doctor follows a planned path and controls the puncture needle to perform a puncture with the aid of the end effector at the end of the manipulator arm. In the online mode, the end effector at the end of the manipulator arm is controlled to perform a puncture with the puncture needle. That is to say, in the online mode and the offline mode, the puncture needle and the end effector can move together between different puncture positions. Therefore, path planning for a surgical robot is essentially planning a movement path of the end effector of the manipulator arm. However, because the effect of pre-surgical simulation is difficult to ensure, during actual surgery, it may easily lead to a collision between an end of a manipulator arm and other objects, for example, a collision with objects such as a patient, an existing puncture needle, a scanning device, or the like. Further, during real-time exposure, in some situations, a puncture trajectory of the puncture needle cannot be observed, for example, in situations where a needle path is tilted in a head-foot direction when performing surgery such as chest or abdominal puncture. This leads to an increase in surgical difficulty, thereby affecting the surgical effect.

Embodiments of the present application provide a system for interventional medicine, comprising an interventional surgical robot and a processor. The interventional surgical robot includes a manipulator arm and an end effector. The processor is configured to obtain surgical planning information in the interventional mode. The interventional mode is the online mode or the offline mode, and the surgical planning information at least includes a plurality of operation positions. The processor is further configured to plan a movement path of the end effector in the interventional mode based on the surgical planning information.

It should be noted that the method for path planning for the end effector of the interventional surgical robot in the embodiments of the present disclosure may be applicable to real-time interventional surgery (online mode) and may also be applicable to offline interventional surgery (offline mode). By performing path planning for the surgical robot in advance before surgery based on the plurality of operation positions (puncture points) in the surgical planning information, it can avoid collision between the end effector and other objects during the surgical procedure, and can also avoid mutual collision among a plurality of puncture needles, thereby improving the effect of interventional multi-needle puncture surgery.

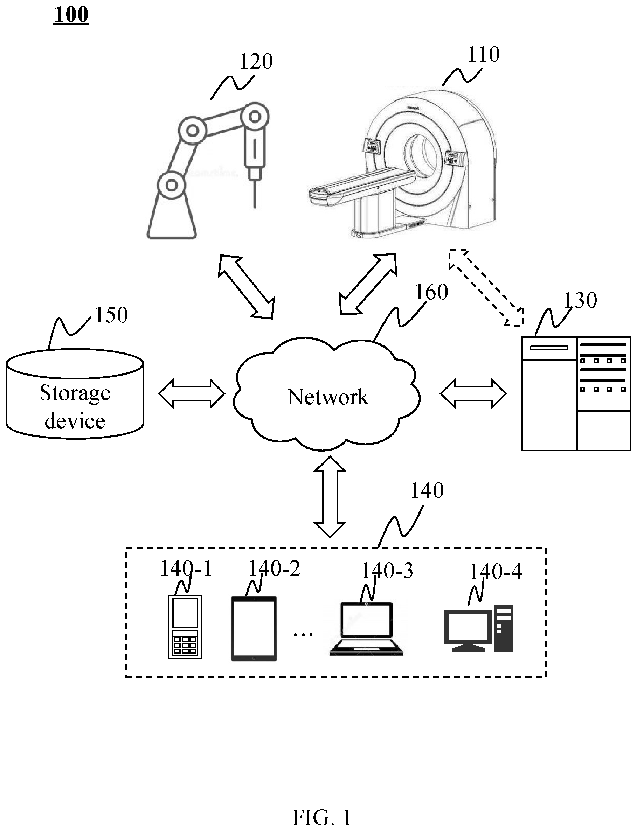

FIG. 1 is a schematic diagram illustrating an exemplary system for interventional medicine according to some embodiments of the present disclosure. In some embodiments, a system for interventional medicine 100 (referred to as the system 100 for short) includes a medical imaging system 110, an interventional surgical robot 120, a processing device 130, a terminal 140, a storage device 150, and a network 160.

The medical imaging system 110 refers to a system that can reproduce internal structures of a human body as images. In some embodiments, the medical imaging system 110 includes a scanning device and a scanning table. The scanning device is any medical imaging device that can image or treat a designated body part of a target object (for example, a patient, a phantom), for example, a CT device, an MR device, a Positron Emission Tomography (PET) device, a Single-Photon Emission Computed Tomography (SPECT) device, an ultrasonic device, or the like. The scanning table may be used to place a patient. In some embodiments, the scanning device obtains medical images by scanning a patient, wherein the medical images include medical ray images, ultrasound scanned images, or the like. The medical ray images include CT images, MR images, PET images, or the like. The present disclosure will illustrate hereinafter using that the scanning device is a CT device as an example. In some embodiments, the medical imaging system 110 exchanges data and/or information with other components in the system 100 (for example, the interventional surgical robot 120, the processing device 130, the storage device 150, the terminal 140) via the network 160. In some embodiments, the medical imaging system 110 is directly connected to other components in the system 100. In some embodiments, one or more components in the system 100 (for example, the processing device 130, the storage device 150) are included in the medical imaging system 110.

The interventional surgical robot 120 refers to a medical robot that can perform surgical treatment on a patient. The interventional surgical robot 120 includes various types of surgical robots for performing interventional surgery, for example, a percutaneous puncture surgical robot, or the like. In some embodiments, the interventional surgical robot 120 is applied to a medical field that requires complex operations, for example, real-time interventional surgery (interventional surgery in the online mode) and offline interventional surgery (interventional surgery in the offline mode), or the like. In some embodiments, the interventional surgical robot 120 is provided with a camera device to obtain images of the robot and an environment where the robot is located, and transmit the images to the processing device 130 and/or the terminal 140. In some embodiments, the interventional surgical robot 120 includes a manipulator arm. The manipulator arm is used for positioning between a surgical instrument and a patient, and the manipulator arm is connected to the surgical instrument via an end effector. The end effector is a device for positioning a surgical instrument and/or connecting the manipulator arm and the surgical instrument, for example, an end gripper, an end assistor, or the like. In some embodiments, the end effector fixes the surgical instrument at an end of the manipulator arm in a surgical robot system, such that a position of the surgical instrument is fixed relative to the manipulator arm. The surgical instrument is an instrument used for performing a surgical procedure on a patient, and may include various types, for example, a puncture needle, a scalpel, a laser emitter, or the like. In some embodiments, since the surgical instrument can be connected to the end effector, the end portion of the manipulator arm, the end effector, and the surgical instrument connected to the manipulator arm via the end effector are regarded as an entirety, and the entirety is collectively referred to as the manipulator arm end. In some embodiments, when the surgical instrument is connected to the end effector, the movement path of the end effector may also represent the movement path of the surgical instrument.

The processing device 130 can process data and/or information obtained from other devices or components of the system. The processing device 130 performs the surgical path planning method described in some embodiments of the present disclosure based on these data, information, and/or processing results, to complete one or more functions described in some embodiments of the present disclosure. For example, the processing device 130 plans the movement path of the end effector based on surgical planning information, and/or position information of the scanning device in the medical imaging system 110 and position information of the scanning table, to determine a target surgical plan. For example, in response to a failure in path planning, the processing device 130 outputs a prompt message to a user. For example, when performing surgical procedures such as a chest puncture, an abdominal puncture, or the like, for a craniocaudal inclined needle path, the processing device 130, by tilting the gantry of the scanning device, enables the user to see the puncture needle insertion trajectory during real-time exposure by the scanning device. In some embodiments, the processing device 130 sends the processed data, for example, the target surgical plan, a sorting result of the target operation positions, or the like, to the storage device 150 for storage. In some embodiments, the processing device 130 obtains pre-stored data and/or information from the storage device 150, for example, surgical planning information, position information of components, or the like, for performing the path planning method shown in some embodiments of the present disclosure. In some embodiments, the processing device 130 includes one or more sub-processing devices (for example, a single-core processing device or a multi-core multi-chip processing device). Merely by way of example, the processing device 130 includes a central processing unit (CPU), an application specific integrated circuit (ASIC), an application specific instruction set processor (ASIP), a graphics processing unit (GPU), a digital signal processor (DSP), a field-programmable gate array (FPGA), a reduced instruction set computer (RISC), a microprocessor, or the like, or any combination thereof.

A terminal 140 can control operations of a surgical procedure. In some embodiments, a doctor issues operation instructions to the medical imaging system 110 via the terminal 140, to enable the medical imaging system 110 to complete specified operations, for example, irradiating and imaging a specified body part of the patient. The doctor issues operation instructions to the interventional surgical robot 120 via the terminal 140, to enable the interventional surgical robot 120 to complete specified operations, for example, executing the target surgical plan and performing a surgical procedure on the patient. In some embodiments, the terminal 140 instructs the processing device 130 to perform the path planning method of the interventional surgical robot 120 as described in some embodiments of the present disclosure. In some embodiments, the terminal 140 includes a display component, and the processing device 130 outputs a prompt message to the user through the display component. For example, information on success and/or failure of surgical path planning, or the like. In some embodiments, the terminal 140 is one or any combination of devices with input and/or output functions, such as a mobile device 140-1, a tablet computer 140-2, a laptop computer 140-3, a desktop computer 140-4, or the like.

A storage device 150 can store data or information generated by other devices. In some embodiments, the storage device 150 stores data and/or information acquired by the medical imaging system 110, for example, scanning images of target objects such as a phantom, the patient, or the like. In some embodiments, the storage device 150 stores data and/or information processed by the processing device 130, for example, the target surgical plan, parameters for gantry tilting of the scanning device, or the like. The storage device 150 includes one or more storage components, and each storage component may be an independent device or a part of other devices. The storage device 150 may be local or may be implemented via the cloud.

A network 160 can connect various components of the system and/or connect the system with external resource portions. The network 160 enables communication between the various components, and with other parts outside the system, to facilitate the exchange of data and/or information. In some embodiments, one or more components of the system 100 (for example, the medical imaging system 110, the interventional surgical robot 120, the processing device 130, the storage device 150, and the terminal 140) send data and/or information to other components via the network 160. In some embodiments, the network 160 is any one or more of a wired network or a wireless network.

In some embodiments, the system for interventional medicine includes the interventional surgical robot 120 and the processing device 130, and the system for interventional medicine establishes a data connection with the medical imaging system 110 via the network 160. During an interventional surgical procedure, based on medical images acquired by the scanning device in the medical imaging system 110 and the surgical planning information, or the like, the processing device 130 performs the path planning method for the interventional surgical robot as described in some embodiments of the present disclosure, to plan and execute the movement path of the end effector of the interventional surgical robot 120 in an interventional mode.

It should be noted that the above description is provided merely for illustrative purposes and is not intended to limit the scope of the present disclosure. For a person of ordinary skill in the art, a plurality of changes and modifications may be made under the guidance of the content of the present disclosure. The features, structures, methods, and other features of the exemplary embodiments described in the present disclosure may be combined in various manners to obtain additional and/or alternative exemplary embodiments. For example, the processing device 130 may be based on a cloud computing platform, for example, a public cloud, a private cloud, a community cloud, a hybrid cloud, or the like. However, these changes and modifications do not depart from the scope of the present disclosure.

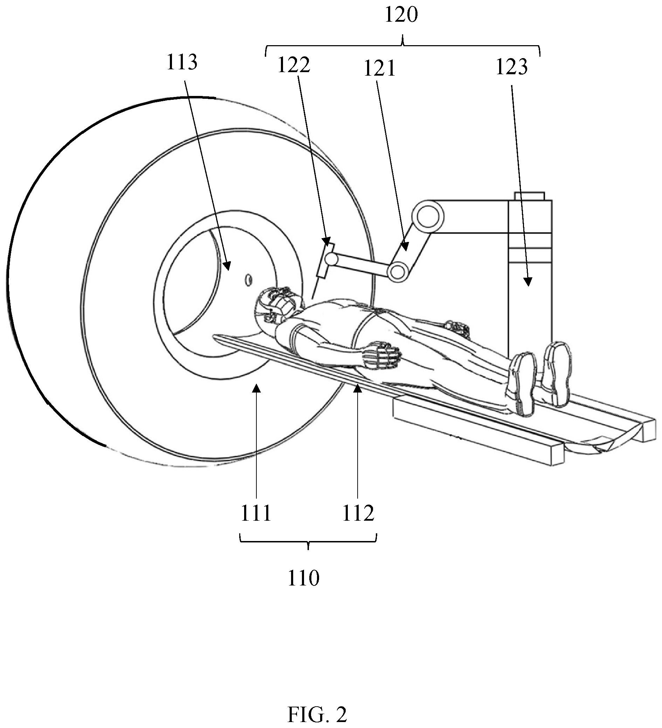

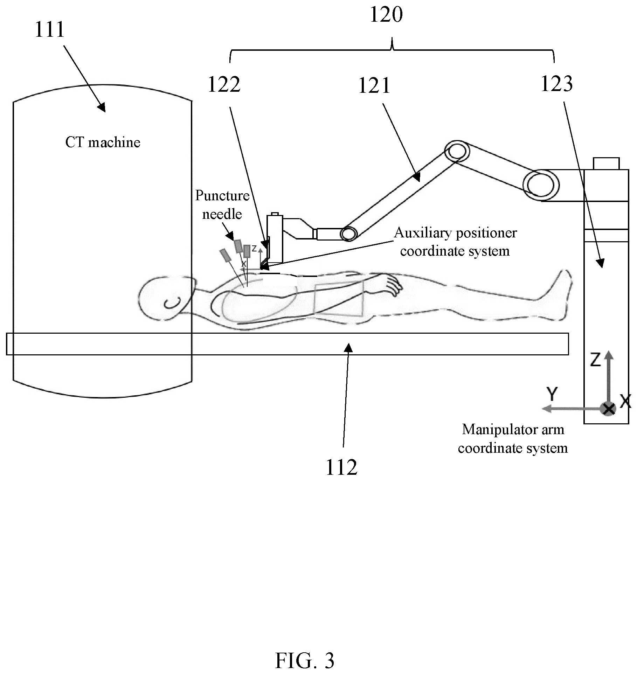

FIG. 2 and FIG. 3 are schematic diagrams illustrating an exemplary system for interventional medicine according to some embodiments of the present disclosure.

As shown in FIG. 2 and FIG. 3, in some embodiments, the medical imaging system 110 in FIG. 1 includes a scanning device 111 and a scanning table 112. Merely by way of example, in FIG. 2, the scanning device 111 is a CT machine, and the scanning table 112 is a surgical table (a surgical table corresponding to the CT machine may also be referred to as a CT bed), and the CT machine performs scanning, imaging, and/or treating of the patient placed on the surgical table.

As shown in FIG. 2, the scanning device 111 includes a gantry (not shown in FIG. 2) and a scanning chamber 113. The scanning chamber is located in the middle of the gantry, and is a hole-like structure enclosed by the inner side of the gantry. The scanning table 112 is used to carry the patient. When the patient is being scanned, the patient is placed on the scanning table 112, and a scanning part of the patient is placed within the scanning chamber by rotation of the gantry and movement of the scanning table 112. In some embodiments, the scanning chamber includes a chamber of a medical imaging device or a chamber formed by a combination of a plurality of medical imaging devices.

As shown in FIG. 2 and FIG. 3, the interventional surgical robot 120 includes a manipulator arm 121, an end effector 122, and a manipulator arm base 123. The manipulator arm 121 is connected to the manipulator arm base 123, and the end effector 122 is connected to the end of the manipulator arm 121. The manipulator arm base 123 may be a fixed base or a movable base (for example, a trolley). In some embodiments, the manipulator arm 121 includes at least two successively actively connected links. When the manipulator arm 121 receives data or instructions sent by other devices or other components of the system 100, the joints connecting the links on the manipulator arm 121 move to cause the manipulator arm 121 to move to a position indicated by the instructions, thereby moving the end effector 122 to the position indicated by the instructions. In some embodiments, the end of the manipulator arm 121 is a link at the farthest end of the manipulator arm 121 (the end closer to the patient's side), which is used to hold the end effector 122. The end effector 122 is connected to a puncture needle, and the interventional surgical robot 120 performs an interventional puncture surgical procedure on the patient on the surgical table through the puncture needle fixed to the end of the end effector 122. In some embodiments, the interventional surgical robot 120 performs a surgical procedure on the patient based on the target surgical plan formulated by the processing device 130, for example, an interventional multi-needle puncture surgical procedure, or the like.

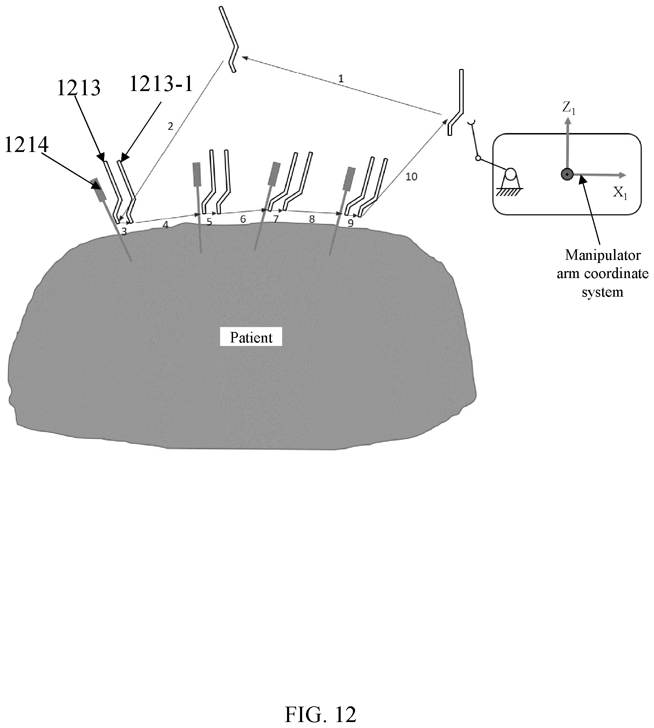

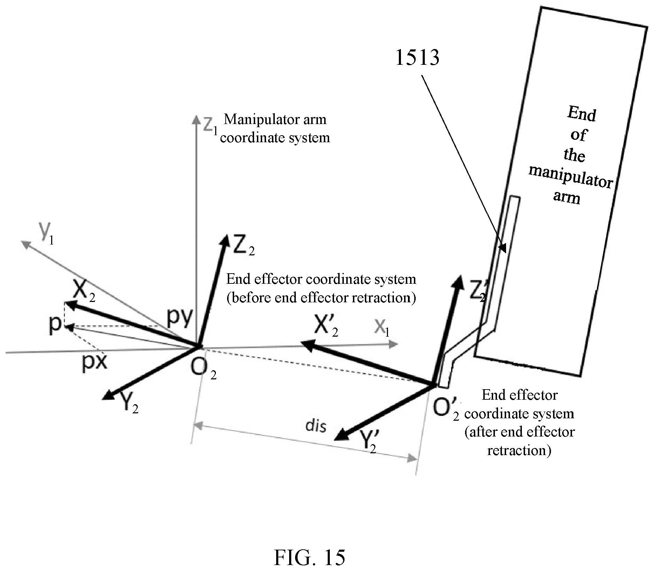

As shown in FIG. 3, the end effector 122 is an auxiliary positioner. An auxiliary positioner coordinate system (that is, the end effector coordinate system when the end effector is the auxiliary positioner) is established with an origin of the end effector 122 (for example, a point O₂ in FIG. 15). The position and attitude of the end effector 122 are represented by the end effector coordinate system and modeling parameters of the end effector 122. In some embodiments, the origin of the end effector 122 is the end position on the end effector 122. For more details on the end effector coordinate system, refer to the related description in FIG. 12.

Merely by way of example, as shown in FIG. 3, a process of the interventional multi-needle puncture surgical procedure is as follows. A patient lies on the scanning table 112. The scanning table 112 carries the patient and moves into the scanning chamber of the scanning device 111. A CT scan is performed on a surgical site of the patient on the scanning table 112. After the scan, a user (for example, a doctor) determines a plurality of puncture positions and needle paths on an interface. The plurality of puncture positions and the needle paths are sorted. A needle path simulation verification is performed. After the needle path simulation verification is successful, the scanning table 112 moves to a planned position. The gantry of the scanning device 111 is tilted to a planned angle corresponding to the planned position. The end effector 122 on the interventional surgical robot 120 holds a puncture needle and moves to a corresponding puncture needle path position according to a planned path. CT real-time exposure guides the user to perform the interventional puncture surgical procedure. In some embodiments, during an interventional surgical procedure, the end effector 122 on the interventional surgical robot 120 moves to a first puncture position (i.e., an operation position) for auxiliary positioning, so that the user can perform an interventional action (for example, inserting a puncture needle from the body surface of the patient to a lesion position with a preset posture through the guidance of an auxiliary positioner). After completing an interventional action, the end effector 122 retracts a certain distance and then moves to a next puncture position, until the interventional actions at all of the puncture positions are completed. During the surgical procedure, the manipulator arm 121 and the end effector 122 of the interventional surgical robot 120 need to avoid the scanning device 111, the body of the patient, and surgical instruments on the body surface of the patient (for example, a puncture needle), or the like.

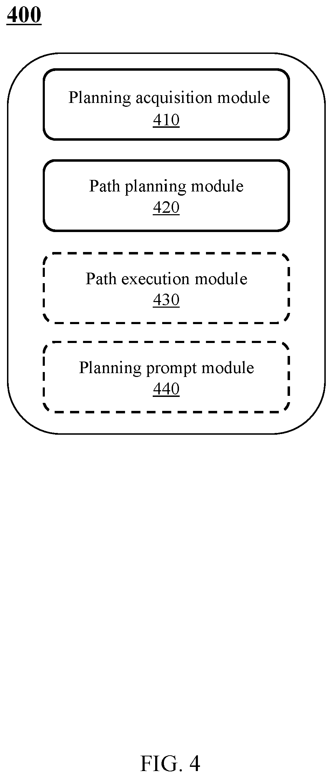

FIG. 4 is a block diagram illustrating an exemplary system for interventional medicine according to some embodiments of the present disclosure. As shown in FIG. 4, a system for interventional medicine 400 (referred to as the system 400) includes a planning acquisition module 410 and a path planning module 420. In some embodiments, the modules of the system 400 are implemented by a processing device 130.

The planning acquisition module 410 is configured to obtain surgical planning information in an interventional mode. The interventional mode is an online mode or an offline mode. The surgical planning information at least includes a plurality of operation positions. For more content regarding how to obtain surgical planning information, refer to step S510.

The path planning module 420 is configured to plan a movement path of an end effector (for example, the end effector 122) in the interventional mode based on the surgical planning information. For more content regarding how to plan the movement path of the end effector in the interventional mode, refer to step S520.

In some embodiments, the system 400 further includes a path execution module 430. In response to successful planning of the movement path of the end effector, the path execution module 430 sequentially plans and executes an execution subpath between each set of adjacent target operation positions according to the planned movement path of the end effector in the interventional mode. For more content regarding how to plan and execute the execution subpath, refer to step S550. In response to a failure in planning the movement path of the end effector, the path execution module 430 re-plans the movement path of the end effector. For more content regarding how to re-plan the movement path of the end effector, refer to step S540.

In some embodiments, the system 400 further includes a planning prompt module 440. In response to a failure or success in planning the movement path of the end effector, the planning prompt module 440 outputs a prompt message. For more content regarding how to output the prompt message, refer to step S530.

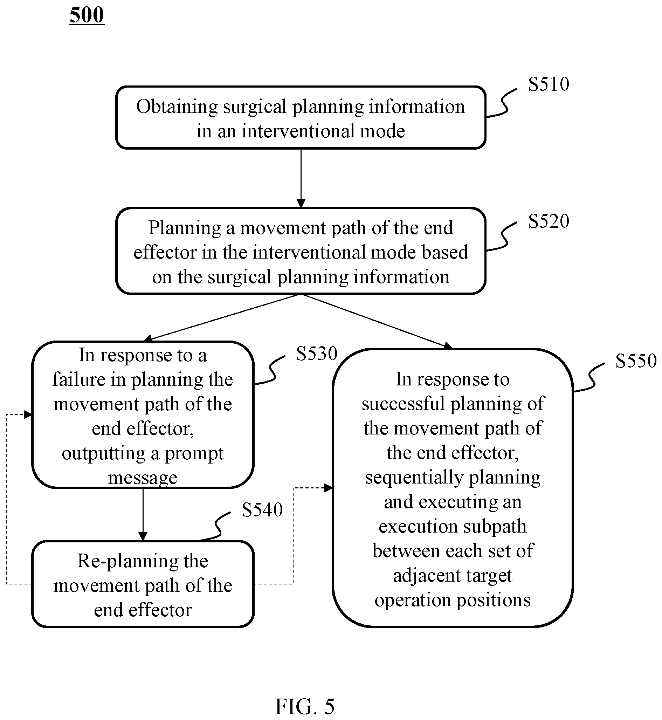

FIG. 5 is a flowchart illustrating an exemplary process for path planning of an interventional surgical robot in a system for interventional medicine according to some embodiments of the present disclosure. As shown in FIG. 5, the process 500 includes the following steps. In some embodiments, the processing device 130 or the modules of the system 400 plan and execute the movement path of an end effector (for example, the end effector 122) in the interventional mode in a system for interventional medicine (for example, the system 100) by executing at least a part of the steps in a process 200.

Step S510: obtaining surgical planning information in an interventional mode. In some embodiments, step S510 is executed by the planning acquisition module 410.

The surgical planning refers to a plan of a surgical procedure that needs path planning. The surgical procedure may comprise various types of surgical procedures, for example, vascular surgical procedures, puncture surgical procedures, or the like. The surgical planning information refers to information that can represent the surgical planning, for example, a type of the surgical procedure, a time of the surgical procedure, an executor of the surgical procedure, a surgical object, a surgical site, patient condition information, a lesion position, or the like. In some embodiments, the patient condition information includes a name of the patient, a gender of the patient, a height of the patient, a weight of the patient, a medical history of the patient, and a lesion position to be treated by the present interventional surgical procedure, or the like. The interventional mode refers to a manner of execution of an interventional surgical procedure. In some embodiments, the interventional mode includes an online mode or an offline mode. It can be understood that the surgical planning and the surgical planning information correspond to a specific interventional mode. That is, for the online mode and the offline mode, there are their respective corresponding surgical planning and surgical planning information, and the surgical planning and the surgical planning information may be the same or different.

An operation position refers to a position where a surgical operation is performed on the body of a target object (i.e., a surgical object, for example, a patient) during a surgical procedure execution. For example, the operation position may be a needle insertion point in a puncture surgical procedure (a point where a surgical instrument contacts the skin of the patient), or the like. In some embodiments, the surgical planning information at least includes a plurality of operation positions. For example, a plurality of needle insertion points. A working channel refers to a surgical path of a surgical instrument corresponding to an operation position. For example, the working channel may be a needle path in a puncture surgical procedure comprising a needle insertion point and a target point, or the like. The needle path refers to a surgical instrument channel formed between a needle insertion point of a surgical instrument and a lesion target point that the surgical instrument needs to reach. In some embodiments, the number of working channels may correspond to the number of operation positions, for example, one-to-one, many-to-one, or the like. In some embodiments, in the online mode, the surgical planning information further includes a plurality of working channels corresponding to the plurality of operation positions. Unless otherwise specified, a surgical procedure in the present disclosure refers to a multi-needle puncture surgical procedure, and an operation position refers to a needle insertion point (a puncture position).

The end effector at the end of the manipulator arm (for example, the end effector 122) needs to perform a corresponding surgical procedure at the plurality of operation positions included in the surgical planning information, along the corresponding working channels. Therefore, it is necessary to determine a movement order of the end effector between the operation positions, which is the movement path. This can ensure a maximum movement space of the manipulator arm. The movement path of the end effector determines a movement order of a surgical instrument connected to the end effector (for example, a puncture order of a puncture needle). Since the end effector is driven by the movement of the manipulator arm, in some embodiments, the movement path of the end effector is determined by movement paths of various joint angles of the manipulator arm. In some embodiments, the processing device 130 obtains the plurality of operation positions in the surgical planning information.

In some embodiments, the surgical planning information further includes information such as at least one of the following information: three-dimensional information of a medical imaging system; an initial position of a scanning device and a scanning table at a start of the surgical procedure; and a position that the scanning device and the scanning table should reach during the surgical procedure execution, or the like.

A first position refers to a position of a scanning device (for example, the scanning device 111). First position information refers to information that represents the first position of the scanning device. The first position information includes a variety of information, for example, a horizontal and vertical position of the gantry of the scanning device, a tilt angle of the gantry of the scanning device, or the like. A second position refers to a position of a scanning table. Second position information refers to information that represents the second position of the scanning table. The second position information includes a variety of information, for example, a vertical position (bed height) of the scanning table, a horizontal position of the scanning table, or the like. In some embodiments, the first position includes an initial position and/or a real-time position of the scanning device. In some embodiments, the second position includes the initial position and/or the real-time position of the scanning table. In some embodiments, the processing device 130 further obtains the first position of the scanning device and the second position of the scanning table, so as to determine starting positions of the scanning device and the scanning table in a planned movement path based on the first position and the second position. For example, the processing device 130 may directly use the first position of the scanning device and the second position of the scanning table as the starting positions. As another example, the processing device 130 may add a preset offset to each of the first position of the scanning device and the second position of the scanning table as the starting positions.

In some embodiments, the surgical planning information includes a surgical initial position, that is, the positions of the scanning device, the scanning table, or the like at the start of a surgical procedure. The first position and the second position may be the same as or different from their respective surgical initial positions. In some embodiments, the first position and the second position include the surgical initial positions of the scanning device and the scanning table in the surgical planning information, and the processing device 130 obtains the surgical initial positions of the scanning device and the scanning table from the surgical planning information. In some embodiments, the first position and the second position are preset position information. For example, the scanning device and the scanning table each preset a fixed position as their respective surgical initial positions, and the processing device 130 obtains the preset position information by reading a configuration file, or the like.

In some embodiments, the first position and the second position include the current positions of the scanning device and the scanning table. The processing device 130 obtains the current positions of the scanning device and the scanning table in various manners. For example, the processing device 130 obtains the current positions of the scanning device and the scanning table via sensors on the scanning device and the scanning table, images captured by an external camera, or the like. In some embodiments, the processing device 130 obtains the current positions of the scanning device and the scanning table at any time (for example, throughout the entire duration of a surgical procedure or at certain moments during the surgical procedure) as the first position and the second position.

The three-dimensional information of a medical imaging system (for example, the medical imaging system 110) refers to three-dimensional information of the external contour of the medical imaging system. In some embodiments, the processing device 130 performs three-dimensional modeling on the medical imaging device by taking photos, three-dimensional scanning, or the like, to store the three-dimensional information of the medical imaging device into a storage device (for example, the storage device 150). When performing path planning for the interventional surgical robot, the three-dimensional information of the medical imaging system can serve as obstacle avoidance information for the manipulator arm and the end effector of the interventional surgical robot.

The processing device 130 obtains the interventional mode and the surgical planning information in various manners. For example, the information may be obtained from a storage device (for example, the storage device 150), or the like. The present disclosure is not limited thereto.

In some embodiments, in response to obtaining the interventional mode, the processing device 130 outputs a first pending confirmation information, wherein the first pending confirmation information indicates whether the end effector is configured.

In some embodiments, in response to obtaining the interventional mode, the processing device 130 determines and outputs recommended configuration information for the end effector, wherein the recommended configuration information includes a recommended end effector and a recommended configuration manner for the end effector. Specifically, the processing device 130 obtains medical information of a patient (for example, condition information, lesion positions, or the like), and determines the recommended configuration information based on the medical information and the interventional mode.

Step S520, planning a movement path of the end effector in the interventional mode based on the surgical planning information. In some embodiments, Step S520 is performed by a path planning module 420.

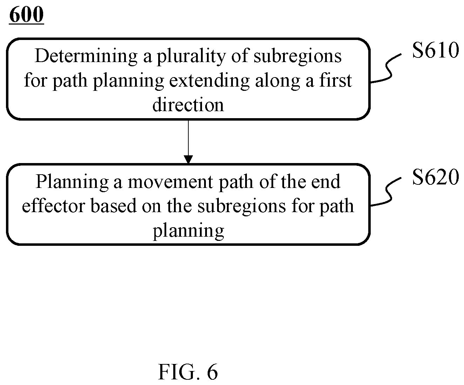

In the offline mode or the online mode, the processing device 130 determines a plurality of subregions for path planning extending along a first direction, based on the plurality of operation positions in the obtained surgical planning information. The plurality of subregions for path planning cover the plurality of operation positions, and each of the subregions for path planning covers at least a part of the plurality of operation positions. The processing device 130 determines a first sorting result of the plurality of operation positions based on the subregions for path planning, and plans the movement path of the end effector based on the first sorting result. For detailed information on how to determine the subregions for path planning and how to plan the movement path of the end effector based on the subregions for path planning, reference is made to the relevant content of FIG. 6.

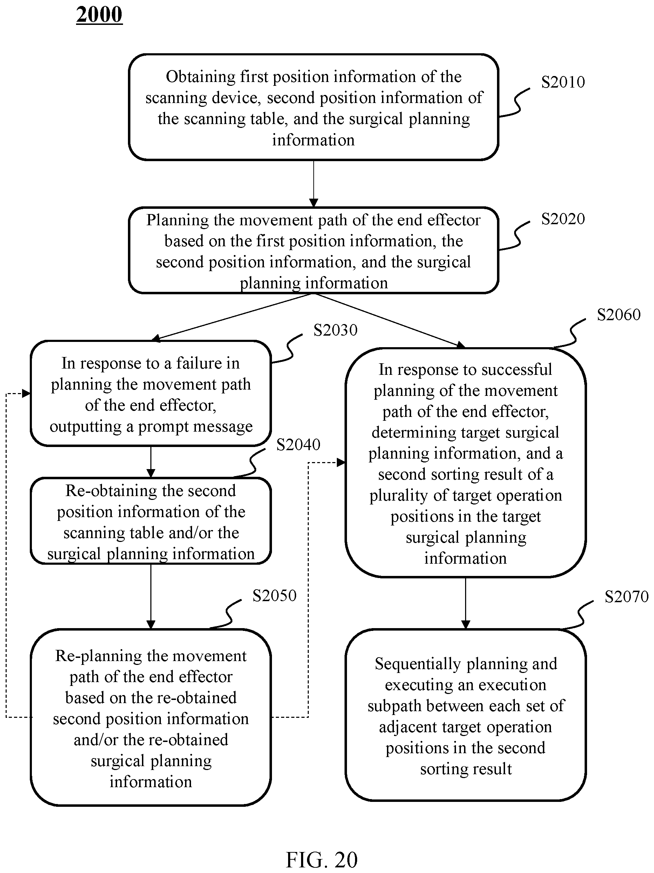

In some embodiments, in the online mode, the surgical planning information further includes a plurality of working channels corresponding to the plurality of operation positions. The processing device 130 obtains the first position information of the scanning device and the second position information of the scanning table, and plans the movement path of the end effector based on the first position information, the second position information, and the surgical planning information. For more information on how to plan the movement path of the end effector based on the first position information, the second position information, and the surgical planning information, reference is made to the relevant content of FIG. 20.

A patient model is a model for simulating a patient entity and its movement trajectories, and can be implemented in various manners, for example, by a physical phantom, a computer-simulated patient body, or the like. In some embodiments, the patient model is determined based on position information and appearance contour information of a patient at different times, or the like. A scanning device model is a model for simulating a scanning device entity and its movement trajectories, and can be implemented in various manners, for example, by scanning device movement simulation, computer-simulated scanning device movement, or the like. In some embodiments, the scanning device model is determined based on position information and appearance contour information of a whole or a part of the scanning device (for example, a scanning chamber) at different times, or the like. A manipulator arm end model is a model for simulating a manipulator arm end, a surgical instrument connected thereto, and movement trajectories of these objects including the manipulator arm end part, the end effector, and the surgical instrument. The surgical instrument is connected to the manipulator arm via the end effector. Therefore, the manipulator arm end part, the end effector, and the surgical instrument can be regarded as a whole. A collision model is a model for simulating whether a plurality of objects will collide during mutual movement, and is capable of simulating various types of objects, for example, the manipulator arm, the end effector, the patient, the scanning device, the surgical instrument connected to the manipulator arm, or the like. In some embodiments, the collision model includes the patient model, the scanning device model, and the manipulator arm end model.

In some embodiments, in the online mode, the processing device 130 determines the collision model based on the first position information and the second position information. For more information on how to determine the collision model based on the first position information and the second position information, reference is made to the relevant content of Step S2020.

In some embodiments, the processing device 130 further plans the movement path of the end effector in the interventional mode based on the collision model. During an interventional multi-needle puncture surgical procedure, puncture needles from the previous several punctures may remain on a patient's body surface. Therefore, these puncture needles need to be considered when planning the movement path of the end effector, to avoid collision interference between the end effector and these puncture needles when the end effector moves along the planned movement path. Thus, models corresponding to these puncture needles need to be added to the collision model at an appropriate timing. In some embodiments, in addition to the patient model, the scanning device model, and the manipulator arm end model, the collision model further includes a surgical instrument model of a surgical instrument inserted into a patient's body. The processing device 130 updates the collision model by adding surgical instrument models of certain operation positions to the collision model. For example, when planning a subpath of the movement path, the processing device 130 updates the collision model by adding a surgical instrument model corresponding to a preceding operation position among a set of adjacent operation positions to the collision model, and plans the subpath based on the updated collision model. For more information on how to plan the movement path based on the collision model, reference is made to the relevant content of Step S1120 and Step S2020.

Step S530, in response to a failure in planning the movement path of the end effector, outputting a prompt message. In some embodiments, Step S530 is performed by a planning prompt module 440.

After a planning failure of the movement path of the end effector, the processing device 130 outputs a prompt message to a user (for example, a doctor, or the like) in various manners. The prompt message is determined based on a cause of the planning failure, and includes at least one of a user failure cause and solution suggestions, or the like.

When planning the movement path, some abnormal situations may exist (for example, a collision between the end effector and a patient, the scanning device, a surgical instrument, or the like), which may lead to a planning failure. If the planning of the movement path of the end effector fails, the processing device 130 outputs a prompt message to a user (for example, a doctor, or the like) in various manners. The prompt message is determined based on a cause of the planning failure, and includes at least one of a user failure cause and solution suggestions, or the like. For more information on the prompt message, reference is made to Step S2030.

In some embodiments, the processing device 130 performs Step S530 based on the prompt message, human experience, or the like, to re-plan the movement path of the end effector. For example, a user (for example, a doctor) may manually drag the manipulator arm to a safe position, or the manipulator arm may be automatically dragged to a safe position by the processing device 130. Then, the surgical planning information is adjusted to obtain a new plurality of puncture positions, and the movement path of the end effector 122 is re-planned based on the adjusted surgical planning information until all subpath planning is successful.

In step S540, re-planning the movement path of the end effector. In some embodiments, step S540 is executed by the path planning module 420.

The processing device 130 re-plans the movement path of the end effector based on the prompt message in step S530. Alternatively, in response to a failure in planning the movement path of the end effector, the processing device 130 directly re-plans the movement path of the end effector. The manner of planning the path is the same as or similar to that in step S520, and will not be described again herein. In some embodiments, the processing device 130 adjusts the operation positions and the corresponding working channels in the planned movement path based on the prompt message, or a user manually adjusts the operation positions and the corresponding working channels in the planned movement path based on manual experience.

The processing device 130 re-acquires at least one of the second position information of the scanning table and the surgical planning information in a manner the same as or similar to that in step S510, wherein the reacquired surgical planning information includes at least one adjusted operation position and an adjusted working channel corresponding to the adjusted operation position. The processing device 130 re-plans the movement path of the end effector based on the reacquired second position information and/or the reacquired surgical planning information. For example, in the offline mode, the processing device 130 re-acquires the surgical planning information, and re-plans the movement path of the end effector based on the reacquired surgical planning information. As another example, in the online mode, the processing device 130 re-acquires the second position information of the scanning table and the surgical planning information, and re-plans the movement path of the end effector based on the reacquired second position information and the reacquired surgical planning information.

In response to successful re-planning of the path, the processing device 130 continues to execute step S550. If the re-planning of the path is not successful, the processing device 130 returns to execute step S530 to re-plan the path again.

Step S550, in response to successful planning of the movement path of the end effector, sequentially planning and executing an execution subpath between each set of adjacent target operation positions. In some embodiments, step S550 is executed by the path execution module 430.

When the movement path of the end effector is successfully planned, the processing device 130 determines target surgical planning information and a sorting result of the plurality of target operation positions in the target surgical planning information. This sorting result is also referred to as the second sorting result. After successful planning, the operation positions and the corresponding working channels in the surgical planning information are verified and can meet the requirements of a normal surgery. The processing device 130 determines the surgical planning information after successful planning as the target surgical planning information. The second sorting result of the plurality of target operation positions in the target surgical planning information is the sorting result of the plurality of operation positions in the surgical planning information (i.e., the first sorting result). The sorting result of the target operation positions determines the execution order of these target operation positions in a surgery. In some embodiments, the target surgical planning information includes surgical planning information to be executed. For example, the surgical planning information executed in an actual surgical phase or a surgical simulation phase.

The movement path further includes an execution path. The execution path includes one or more execution subpaths. In some embodiments, the execution path includes an execution subpath between each set of adjacent operation positions in the second sorting result, wherein each of the execution subpaths includes one or more path segments. The complete movement path of the end effector may comprise a plurality of continuous execution subpaths. Specifically, the processing device 130 may control the end effector of the manipulator arm to move along the execution subpath by instructions, or the like.

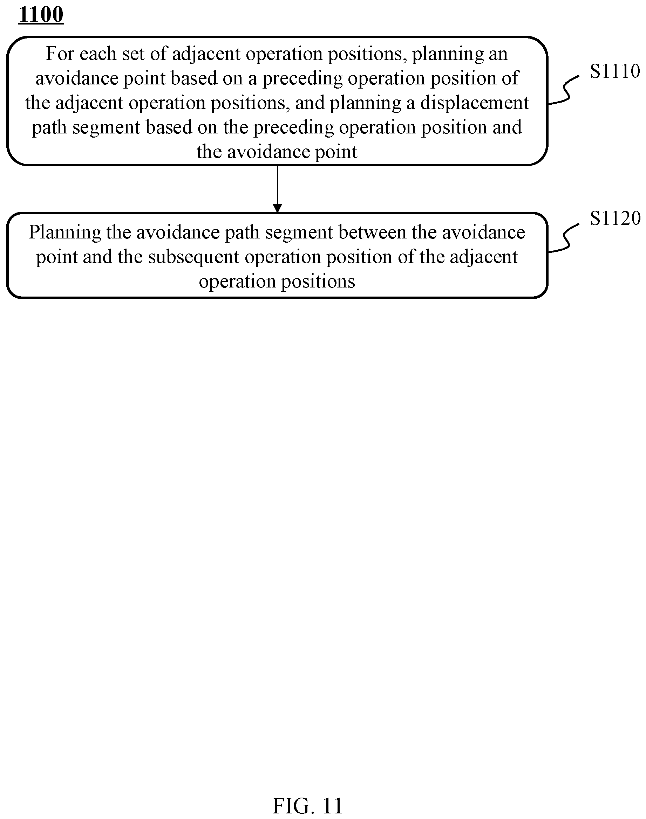

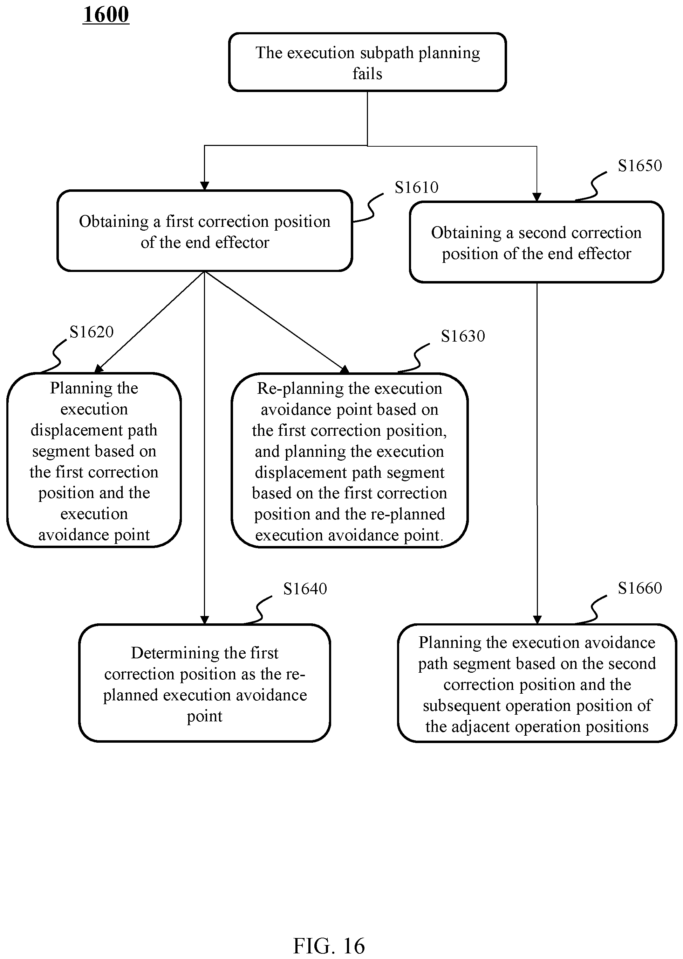

After determining the target surgical planning information, in a preset environment, the processing device 130 sequentially plans and executes an execution subpath between each set of adjacent target operation positions in the second sorting result based on the target surgical planning information. The preset environment includes at least one of an actual surgical phase, a surgical simulation phase, or the like. For example, in the offline mode, the processing device 130 sequentially plans and executes an execution subpath between each set of adjacent target operation positions in the second sorting result based on the target surgical planning information. As another example, in the online mode, the processing device 130 sequentially plans and executes an execution subpath between each set of adjacent target operation positions in the second sorting result based on the first position information, the second position information, and the target surgical planning information. For more details on how to plan and execute the execution subpath, refer to FIG. 11, FIG. 16, step S1870, step S2070, and steps S2120-S2160.

In some embodiments, the online mode includes at least one of a real-time control mode, a breakpoint control mode, or the like. In the online mode, the processing device 130 further plans the execution path based on images acquired by the scanning device.

In some embodiments, the online mode includes a real-time control mode, and the execution path is further planned based on a real-time image. The real-time image is an image acquired by keeping the scanning device continuously turned on during an interventional surgical procedure. For example, if it is a real-time control mode, then images captured by a CT machine provide real-time guidance during the entire puncture phase of a puncture surgery.

In some embodiments, the online mode includes a breakpoint control mode, and the execution path is further planned based on a breakpoint image. The breakpoint image is an image acquired by intermittently turning on the scanning device during an interventional surgical procedure. For example, if it is a breakpoint control mode, then during the entire puncture phase of a puncture surgery, a cycle of scanning and puncture is performed. That is, when a puncture operation is performed, the CT machine does not perform scanning; and when the CT machine performs scanning, the puncture operation is not performed.

In some embodiments, the online mode includes a real-time control mode and a breakpoint control mode. The real-time control mode and the breakpoint control mode are switchable. For example, in a puncture surgery, in the breakpoint control mode, a doctor may switch a cycle of scanning and puncture to real-time guidance by scanning during puncture at any time, that is, switch to the real-time control mode.

In some embodiments, steps S510-S540 are executed in a preoperative phase of a surgery, and step S550 is executed in an intraoperative phase of the surgery.

In some embodiments of the present disclosure, by planning and executing the movement path of the end effector of the interventional surgical robot based on surgical planning information in different interventional modes, the path can be planned in advance before a surgery, which improves the accuracy of path planning. At the same time, this avoids the end effector colliding with other objects (for example, a patient, an existing puncture needle, a scanning device, or the like) during the surgery. By using a prompt message to prompt a user with a failure reason and a solution suggestion when planning fails, the user can accurately and quickly determine the failure reason and timely make corresponding adjustments to the planning, thereby ensuring the effectiveness of the planned path.

FIG. 6 is a flowchart illustrating an exemplary process for path planning of an interventional surgical robot in a system for interventional medicine according to some embodiments of the present disclosure. As shown in FIG. 6, the process 600 includes the following steps. In some embodiments, the processing device 130 or the path planning module 420 implements step S520 by executing the process 600, that is, planning the movement path of the end effector in the interventional mode in a system for interventional medicine based on surgical planning information, wherein the process 600 is applicable to the offline mode and the online mode.

Step S610, determining a plurality of subregions for path planning extending along a first direction.

Since a multi-needle puncture surgery includes a plurality of puncture positions (operation positions) and these puncture positions are widely distributed, each of the puncture positions requires interventional operation with a surgical instrument. After the intervention is completed, the surgical instrument (for example, a puncture needle) may remain on the patient's body surface, then the surgical instrument after intervention may become an obstacle on the movement path of the end effector. Therefore, the processing device 130 may divide a plurality of subregions for path planning based on the plurality of operation positions. The subregions for path planning cover the plurality of operation positions, and each of the subregions for path planning covers at least a part of the plurality of operation positions.

In some embodiments, the processing device 130 establishes a CT bed coordinate system in the following manner. An origin is at a point on the CT bed. A short axis of the CT bed is an X-axis, with the positive X-direction facing right. A direction perpendicular to a surface of the CT bed is a Y-axis, with the positive Y-direction pointing from a lower surface of the CT bed to an upper surface of the CT bed. A long axis of the CT bed is a Z-axis, with the positive Z-direction being the direction in which the CT bed enters a scanning chamber. In some embodiments, the processing device 130 establishes a CT bed coordinate system in the following manner. By moving the CT bed into a scanning chamber, when a bed code value displayed on a CT device operation interface becomes 0, a CT isocenter and an origin of the CT bed coordinate system coincide. At this time, the position of the CT isocenter relative to the CT bed is the position of the origin of the CT bed coordinate system.

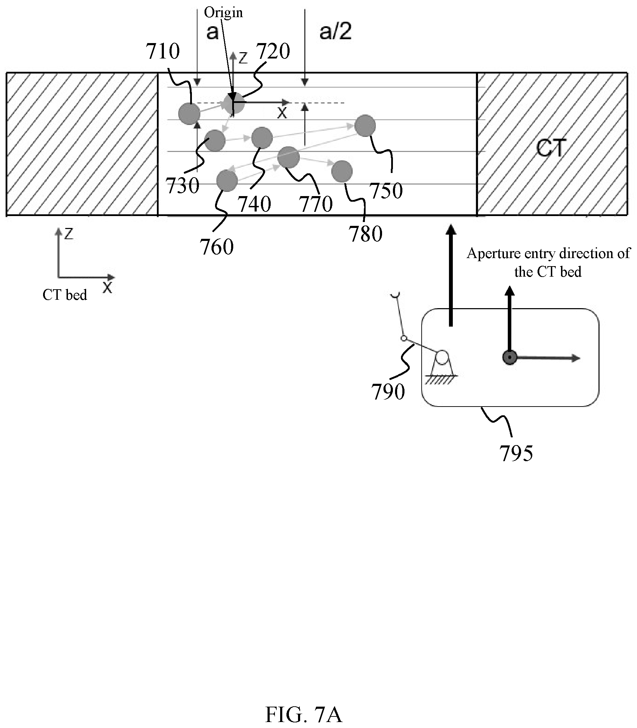

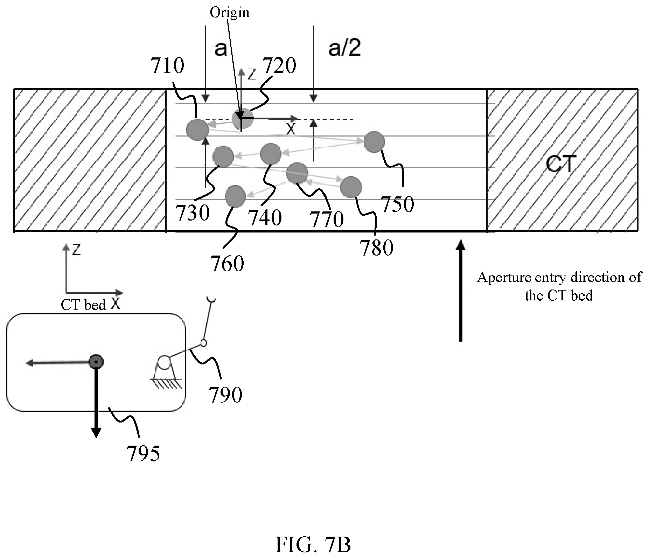

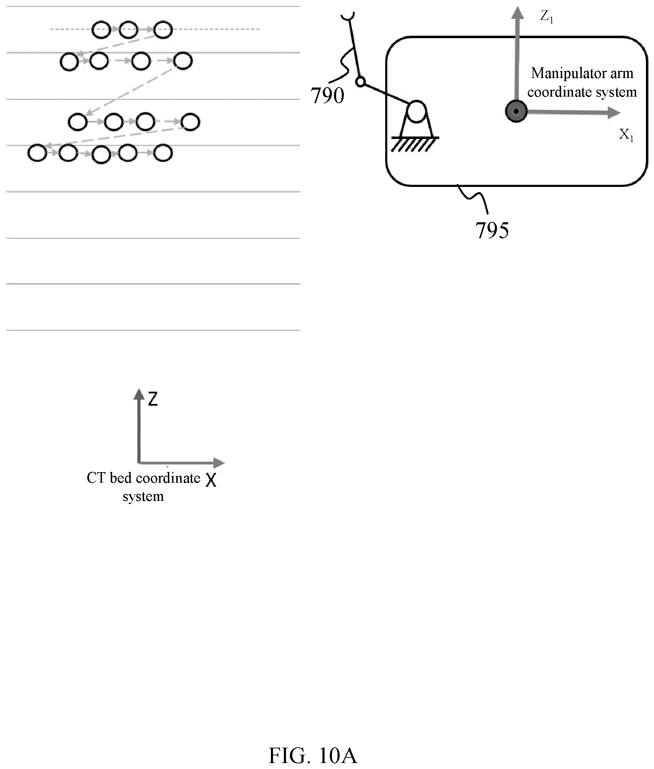

In some embodiments, the first direction is parallel to the short axis of the scanning table (for example, the scanning table 112). Merely by way of example, FIG. 7A, FIG. 7B, and FIG. 10A are schematic diagrams illustrating an exemplary sorting of the plurality of operation positions according to some embodiments of the present disclosure. As shown in FIGs. 7A, 7B, and 10A, the first direction is parallel to the short axis of the scanning table (the X-axis of the CT bed coordinate system), a puncture region is divided into a plurality of strip-shaped subregions for path planning, and dots in the puncture region represent operation positions. The plurality of subregions for path planning are a plurality of divided strip-shaped regions with the same width in the Z-axis direction of the CT bed coordinate system, and the width of each strip-shaped subregion for path planning may be configured to be a. In FIGs. 7A, 7B, and 10A, the plurality of strip-shaped subregions for path planning are referred to as multi-row subregions for path planning. The width w of the subregion for path planning may be manually set by a doctor based on the size of a surgical instrument, the shape of the end effector 122, and the density of the plurality of operation positions. In some embodiments, when the shape of the end effector 122 is I-shaped, it may be more suitable to set the first direction as the X-axis direction of the CT bed coordinate system. For specific reasons, refer to the relevant content in step S620. In some embodiments, the plurality of subregions for path planning may be a plurality of divided strip-shaped regions with different widths along the first direction, which may be suitable for a scenario where the plurality of operation positions are unevenly distributed. For example, regions where insertion points are relatively dense are divided into strip-shaped regions with a smaller width, and regions where insertion points are relatively sparse are divided into strip-shaped regions with a larger width.

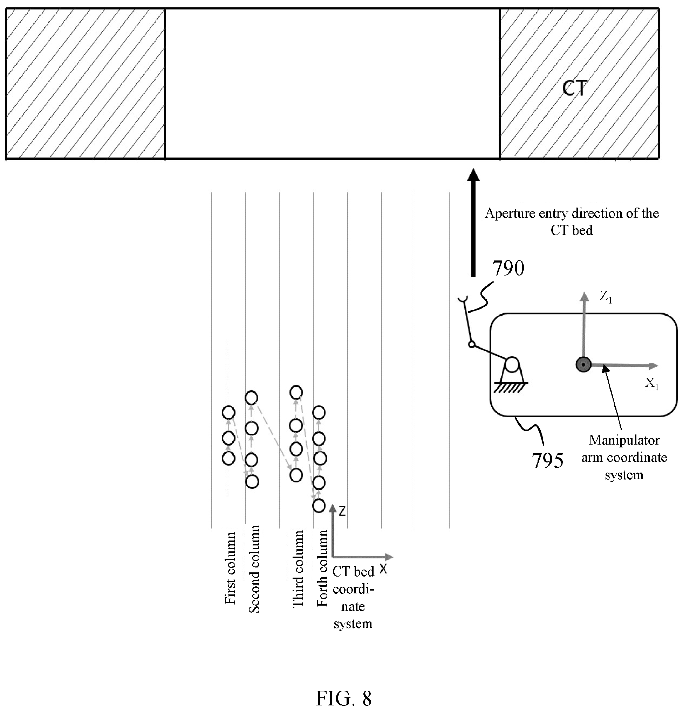

In some embodiments, the first direction is parallel to the long axis of the scanning table. Merely by way of example, FIG. 8 is a schematic diagram illustrating an exemplary sorting of a plurality of operation positions according to some embodiments of the present disclosure. As shown in FIG. 8, the first direction is parallel to the long axis of the scanning table (the Z-axis of the CT bed coordinate system), a puncture region is divided into a plurality of strip-shaped subregions for path planning, and dots in the puncture region represent operation positions. The plurality of subregions for path planning are a plurality of divided strip-shaped regions with the same width in the X-axis direction of the CT bed coordinate system, and the width of each strip-shaped subregion for path planning may be configured to be a, which may be suitable for a scenario where a plurality of working channels are relatively evenly distributed. In FIG. 8, the plurality of strip-shaped subregions for path planning are referred to as multi-column subregions for path planning. In some embodiments, when the shape of the end effector 122 is L-shaped, it may be more suitable to set the first direction as the Z-axis direction of the CT bed coordinate system. For specific reasons, refer to the relevant content in step S620.

Step S620, planning a movement path of the end effector based on the subregions for path planning.

The processing device 130 determines the first sorting result of the plurality of operation positions based on the subregions for path planning.

In some embodiments, the processing device 130 sorts the plurality of subregions for path planning along a preset direction to obtain a first intermediate sorting result, wherein the preset direction is parallel to a horizontal plane and perpendicular to the first direction. In some embodiments, the processing device 130 sorts the operation positions covered by each subregion for path planning to obtain a second intermediate sorting result. The sorting of the plurality of subregions for path planning and the sorting of the operation positions in each subregion for path planning may be performed sequentially or simultaneously. In some embodiments, the processing device 130 obtains the first sorting result based on the first intermediate sorting result and a plurality of second intermediate sorting results.

In some embodiments, the processing device 130 determines a sorting characteristic value of each subregion for path planning, and then sorts the subregions for path planning along the preset direction based on the sorting characteristic values of all the subregions for path planning. In some embodiments, a sorting characteristic value of a subregion for path planning includes an extreme value of operation position characteristic values in the subregion, for example, a maximum value or a minimum value. In some embodiments, a sorting characteristic value of a subregion for path planning is another value related to operation position characteristic values in the subregion, for example, an average value of all the characteristic values, or the like. In some embodiments, the processing device 130 determines a sorting characteristic value of a subregion for path planning through various manners. For example, the operation position characteristic value ranked first/last in the second intermediate sorting result of the subregion for path planning is used as the sorting characteristic value. As another example, an extreme value of operation position characteristic values in the subregion for path planning is directly obtained as the sorting characteristic value.

In some embodiments, the preset direction is determined based on a position of the subregion for path planning relative to a manipulator arm base. For example, as shown in FIG. 8, when the first direction is the long axis of the scanning table (i.e., the Z-axis direction of the CT bed coordinate system), and a subregion for path planning is on the left side of the manipulator arm base, then the preset direction may be a direction in which the X-coordinate value of the CT bed coordinate system increases (the positive X-axis direction). As another example, when the first direction is the long axis of the scanning table (i.e., the Z-axis direction of the CT bed coordinate system), and if a subregion for path planning is on the right side of the manipulator arm base, then the preset direction may be a direction in which the X-coordinate value of the CT bed coordinate system decreases (the negative X-axis direction). As another example, as shown in FIG. 8, when the first direction is the long axis of the scanning table (i.e., the Z-axis direction of the CT bed coordinate system), the subregions for path planning may be sorted in an order from far from a manipulator arm base 795 to close to the manipulator arm base 795, or the subregions for path planning may be sorted in an order from close to the manipulator arm base 795 to far from the manipulator arm base 795.

In some embodiments, the preset direction is determined based on a position of the subregion for path planning relative to the scanning device. For example, as shown in FIGs. 7A and 7B, when the first direction is the short axis of the scanning table (i.e., the X-axis direction of the CT bed coordinate system), the subregions for path planning may be sorted along the Z-axis direction of the CT bed coordinate system in an order from inside a scanning chamber aperture of the scanning device to outside the scanning chamber aperture, that is, the preset direction may be a negative Z-axis direction of the CT bed coordinate system. As another example, the preset direction may be a positive Z-axis direction of the CT bed coordinate system, that is, the subregions for path planning may be sorted along the Z-axis direction in an order from outside the scanning chamber aperture of the scanning device to inside the scanning chamber aperture.

In some embodiments, the end effector may comprise an end gripper (for example, a claw), the end gripper is used for holding a puncture needle, and the preset direction is determined based on an opening direction of the end gripper. Specifically, the processing device 130 determines opening directions of the end gripper at the plurality of operation positions, and determines the preset direction based on the opening directions. In some embodiments, the preset direction is a direction opposite to an opening direction of the end gripper at a majority of operation positions. For example, when the opening direction of the end gripper at a majority of insertion points is towards inside the scanning chamber aperture of the scanning device, the preset direction is a direction away from the aperture (i.e., towards outside the aperture). As another example, when the opening direction of the end gripper at a majority of insertion points is towards outside the scanning chamber aperture of the scanning device, the preset direction is a direction towards the aperture (i.e., towards inside the aperture).

The second intermediate sorting result refers to the sorting of all operation positions in a single region. In each of the plurality of subregions for path planning, the processing device 130 sorts the operation positions according to a preset sorting rule to obtain the second intermediate sorting result. The preset sorting rule includes a plurality of types. For example, the preset sorting rule includes any one of: sorting along the first direction, sorting based on characteristic values of operation positions, and sorting according to the shape of the end effector at the manipulator arm end, or the like. By sorting the operation positions covered by each subregion for path planning, it may be possible to avoid a collision between a surgical instrument previously punctured and remaining on a patient's body surface and the end effector, thereby improving the rationality and accuracy of path planning.

In some embodiments, in each subregion for path planning, the processing device 130 sorts the operation positions covered by the subregion for path planning along the first direction to obtain the second intermediate sorting result. For example, as shown in FIGs. 7A and 7B, when the first direction is parallel to the short axis of the scanning table, namely the X-axis of the CT bed coordinate system, in each subregion for path planning, the insertion points are sorted according to a direction in which the X-coordinate value increases (FIG. 7A) or a direction in which the X-coordinate value decreases (FIG. 7B).

In some embodiments, the processing device 130 sorts based on characteristic values of operation positions, wherein the characteristic values of operation positions may comprise various values capable of representing the operation positions, for example, coordinate values of the operation positions (for example, coordinate values along the first direction or the second direction, or the like), measures of importance of the operation positions, or the like.

In some embodiments, the processing device 130 determines the preset sorting rule according to the shape of the end effector at the manipulator arm end. For example, the processing device 130 determines a sorting rule according to the shape of an auxiliary positioner, an opening direction of the end gripper, or the like.

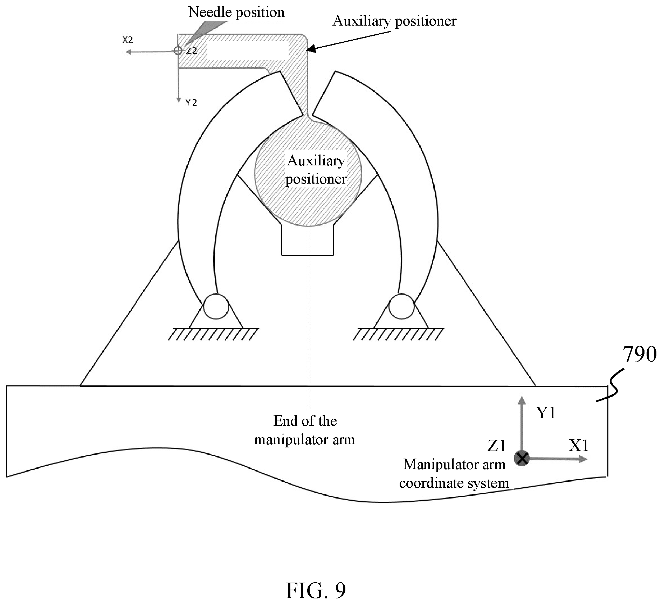

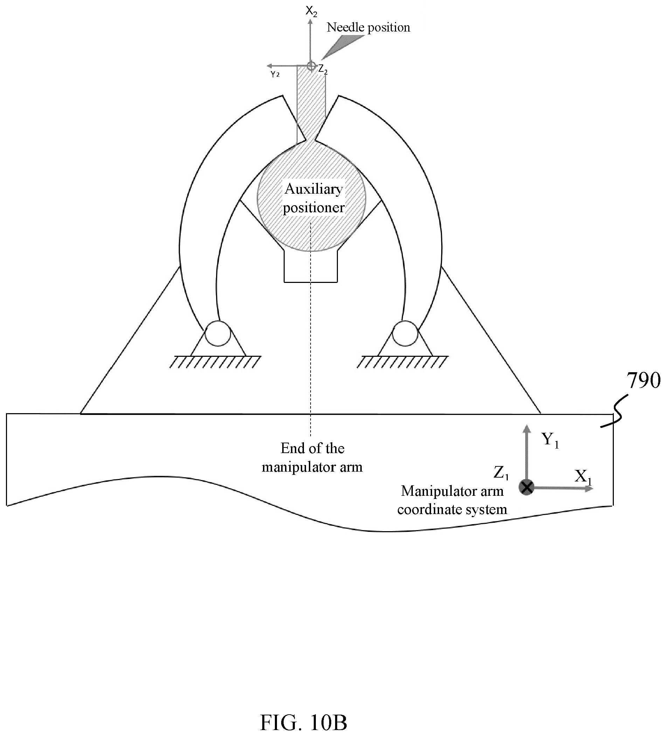

In some embodiments, the end effector is an auxiliary positioner, the auxiliary positioner can assist in puncturing with a puncture needle, and the preset sorting rule is determined according to the shape of the auxiliary positioner. Merely by way of example, FIG. 9 is a schematic diagram illustrating an exemplary end effector of an interventional surgical robot corresponding to the plurality of operation positions in FIG. 8 according to some embodiments of the present disclosure. As shown in FIG. 9, the shape of the end effector is L-shaped. When the end effector has an L-shape, as shown in FIG. 8, on each of the subregions for path planning in the Z-axis direction of the CT bed coordinate system, the needle insertion points are sorted in an order from outside to inside of the aperture of the scanning chamber of the scanning device (for example, when the positive Z-axis direction points into the aperture, the needle insertion points are sorted according to Z coordinate values from smallest to largest). Simultaneously, for the sorting of the plurality of subregions for path planning, the puncture positions on the adjacent subregions for path planning are sorted in an order from far from the manipulator arm base 795 to near the manipulator arm base 795 (that is, sorted according to X coordinate values from smallest to largest). This sorting manner is referred to as an N-like sorting manner. As another example, FIG. 10B is a schematic diagram of an end effector of an exemplary interventional surgical robot corresponding to the plurality of operation positions in FIG. 10A according to some embodiments of the present disclosure. As shown in FIG. 10B, the end effector has an I-shape. When the end effector has an I-shape, as shown in FIG. 10A, on each of the subregions for path planning in the X-axis direction of the CT bed coordinate system, the needle insertion points are sorted in an order from far from the manipulator arm base 795 to near the manipulator arm base 795 (for example, when the positive X-axis direction points to the base, the needle insertion points are sorted according to X coordinate values from smallest to largest). Simultaneously, for the sorting of the plurality of subregions for path planning, the puncture positions on the adjacent subregions for path planning are sorted in an order from inside to outside of the aperture of the scanning chamber of the scanning device (that is, sorted according to Z coordinate values from largest to smallest). This sorting manner may be referred to as a Z-like sorting manner.

In some embodiments, the end effector includes an end gripper (for example, a gripper), and the preset sorting rule is determined based on the opening direction of the end gripper. Specifically, the processing device 130 determines the opening directions of the end gripper at the plurality of operation positions, and sorting the operation positions covered by each of the subregions for path planning based on the opening directions. Taking the end gripper as a gripper as an example, the processing device 130 first determines the opening directions of the gripper at the plurality of operation positions, and sorting the operation positions covered by one or more subregions for path planning based on the opening directions. For example, a puncture region includes a plurality of needle insertion points, and each of the needle insertion points corresponds to an opening direction of a gripper. A plurality of needle insertion points on each of the subregions for path planning correspond to a plurality of opening directions of the gripper. If most of the opening directions of the gripper in the puncture region point towards the inner side of the aperture of the scanning chamber, then the preset sorting rule is to sort in an order from inside to outside of the aperture of the scanning chamber of the scanning device. For example, when the positive Z-axis direction points into the aperture of the scanning chamber, the needle insertion points are sorted according to Z coordinate values from largest to smallest. If most of the opening directions of the gripper in the puncture region point towards the outer side of the aperture of the scanning chamber, then the preset sorting rule is to sort in an order from outside to inside of the aperture of the scanning chamber of the scanning device. For example, when the positive Z-axis direction points into the aperture of the scanning chamber, the needle insertion points are sorted according to Z coordinate values from smallest to largest.

In some embodiments, the opening direction of the end gripper of the manipulator arm is determined in the following manner. First, the position of an origin and the Z₂-axis direction of an end effector coordinate system (for example, the coordinate system composed of X₂, Y₂, and Z₂ in FIGS. 9 and 10B, established with an endpoint of the end effector away from the manipulator arm 790 as an origin) at a needle insertion point are determined based on the needle insertion point and a target point. Second, in a plane passing through the origin of the end effector coordinate system and perpendicular to the Z₂ axis of the end effector coordinate system, a direction is arbitrarily taken as the Y₂-axis direction. Third, the X₂-axis direction of the end effector coordinate system is determined based on the Z₂-axis direction and the Y₂-axis direction of the end effector coordinate system. Fourth, inverse kinematics is used to verify the reasonableness of the end effector coordinate system (before end effector retraction). If it is unreasonable, then the Z₂ axis of the end effector coordinate system is rotated by the end of the manipulator arm, and a new end effector coordinate system orientation is continuously searched. If it is reasonable, then the position and attitude of the end effector coordinate system are determined, and the X₂ axis of the end effector coordinate system is the opening direction of an end gripper assembly.

In some embodiments, the processing device 130 may sort the operation positions according to the first intermediate sorting result and the plurality of second intermediate sorting results to obtain a sorting result of the plurality of operation positions, that is, the first sorting result. Merely by way of example, the processing device 130 arranges the second intermediate sorting result of each of the subregions for path planning according to the sorting result of the subregions for path planning (the first intermediate sorting result), thereby obtaining the first sorting result.

In some embodiments, the above-mentioned sorting may use any sorting algorithm. For example, the sorting may be performed according to coordinate values of the operation positions along a certain direction, or the like. In some embodiments, the sorting manner may also be user sorting, or may be performed by using a sorting algorithm combined with user sorting.

In some embodiments, the processing device 130 further uses a trained machine learning model for path planning, wherein the model may be trained by a large amount of historical path planning sample data.

In some embodiments, a path planning algorithm used in the present disclosure may be a Joint Linear Interpolation Algorithm MoveJ, or may be a sampling-based search algorithm (for example, a PRM algorithm, an RRT algorithm, or the like), or may be an intelligent bionic path planning algorithm (for example, a neural network algorithm, an ant colony algorithm, a genetic algorithm, or the like), or may be other path planning algorithms (for example, an A* algorithm, a Dijkstra algorithm, a D algorithm, an artificial potential field method, or the like). The present disclosure is not limited thereto.

Merely by way of example, when planning a puncture order of the puncture needles, since the needle insertion points of all needle paths are known, assuming that the coordinates of the needle insertion points are located in the CT bed coordinate system, the processing device 130 may select a needle insertion point having the smallest/largest Z coordinate value. The space is divided into a plurality of parallel regions in the X-axis/Z-axis direction according to the X-axis/Z-axis coordinate value of the needle insertion point. The spacing of the regions may be equal or unequal, and all the needle insertion points are located in the regions. Hereinafter, dividing the regions in the Z-axis direction will be described by way of example. For different needle insertion points in the same region, the needle paths corresponding to the needle insertion points far from the manipulator arm are preferentially planned, that is, in each of the regions, all the needle insertion points are sorted according to X coordinate values from smallest to largest. All the regions are sorted according to the needle insertion points having the smallest/largest Z coordinate values in each of the regions, and the needle paths corresponding to the needle insertion points in the regions having smaller/larger Z coordinate values are preferentially planned. Specifically, the minimum/maximum Z coordinate values of the needle insertion points in each of the regions may be taken as the Z coordinate value of the region, and each of the regions is planned sequentially according to a sorting result of the region coordinate values (from smallest to largest or from largest to smallest).

Hereinafter, how to sort the needle paths will be described by way of example with reference to FIGS. 7A and 7B. In FIG. 7A, a placement position of the manipulator arm 590 installed on the manipulator arm base is on the right side of the CT scanning chamber. In FIG. 7B, the placement position of the manipulator arm 590 is on the left side of the CT scanning chamber. In FIGS. 7A and 7B, the X-axis of the CT bed coordinate system is a short axis of the CT bed, and a positive X-axis direction points to the right. The Z-axis is a long axis of the CT bed, and a positive Z-axis direction is a direction in which the scanning table enters the scanning chamber, that is, an aperture entry direction of the CT bed. The positions 710, 720, 730, 740, 750, 750, 760, 770, and 780 are all needle insertion points, that is, the plurality of operation positions in the surgical planning information.

As shown in FIG. 7A, assuming that a row spacing when dividing regions is set to 'a', first, the needle insertion point 720 having the largest Z coordinate value among all the needle insertion points in the CT bed coordinate system is searched. Then, dividing surfaces of ±a/2 are defined passing through the point 720. The distance between the two dividing surfaces is 'a'. Equidistant subregions for path planning (with a row spacing of 'a') are sequentially divided towards the negative Z-axis direction. Each of the needle insertion points is divided into a subregion for path planning. In the same subregion for path planning, the needle insertion points far from the manipulator arm 790 are preferentially planned; that is, the needle insertion points are sequentially planned according to the distance from the manipulator arm 790 from far to near. In the region where 710 and 720 are located, 710 and 720 are sequentially planned. In the region where 730-750 are located, 730, 740, and 750 are sequentially planned. In the region where 760-580 are located, 760, 770, and 780 are sequentially planned. All the subregions for path planning are sorted according to the needle insertion points having the smallest/largest Z coordinate values in each of the subregions for path planning. The subregions for path planning where the needle insertion points with larger Z coordinate values are located are preferentially planned. That is, the following are sequentially planned in an order from front to back: the region where 710 and 720 are located, the region where 730-750 are located, and the region where 760-780 are located. The final planned order of the needle insertion points from first to last is as follows: 710, 720, 730, 740, 750, 750, 760, 770, and 780.

As shown in FIG. 7B, the main difference between FIG. 7B and FIG. 7A is that the placement position of the manipulator arm 790 is on the left side of the CT scanning chamber. Therefore, after being divided into the same subregions for path planning as in FIG. 7A, the sorting result of the regions is also the same. However, when sorting the needle insertion points in each of the subregions for path planning, the needle insertion points are sequentially planned according to the distance from the manipulator arm 790 from far to near. Then, in the region where 710 and 720 are located, 720 and 710 are sequentially planned. In the region where 730-750 are located, 750, 740, and 730 are sequentially planned. In the region where 760-780 are located, 780, 770, and 760 are sequentially planned. Therefore, the final planned order of the needle insertion points from first to last is as follows: 720, 710, 750, 740, 730, 750, 780, 770, and 760.

In some embodiments, when a scenario is real-time image-guided online puncture (an online mode) or offline puncture (an offline mode), the processing device 130 sorts in an order from far from the manipulator arm to near the manipulator arm to obtain the first sorting result, which increases a plannable space. In some embodiments, when the scenario is offline puncture (the offline mode), the processing device 130 sorts in an order from near the manipulator arm to far from the manipulator arm to obtain the first sorting result, which facilitates doctor operations.

The processing device 130 plans the movement path of the end effector based on the first sorting result. The order of the operation positions on the movement path is the execution order of the operation positions in the surgery. By sorting the operation positions and planning the path based on the first sorting result, a success rate of path planning is improved.

The processing device 130 connects the operation positions with line segments according to the first sorting result, thereby forming the planned movement path of the end effector.

In some embodiments, the planned movement path of the end effector includes one or more subpaths between adjacent operation positions, wherein a path between each two adjacent operation positions may be regarded as a subpath. Adjacent operation positions refer to two sequentially adjacent operation positions in a sorting result (for example, the first sorting result, the second intermediate sorting result). For each set of adjacent operation positions, the processing device 130 plans an avoidance point based on a preceding operation position of the adjacent operation positions, and plans a displacement path segment based on the preceding operation position and the avoidance point. The displacement path segment is a path segment between the preceding operation position and the avoidance point. The processing device 130 further plans an avoidance path segment between the avoidance point and a subsequent operation position of the adjacent operation positions. The avoidance path segment and the displacement path segment constitute the subpath. For more content on how to plan the avoidance path segment and the displacement path segment, refer to FIGS. 11 and 12.

In some embodiments, the movement path of the end effector includes a verification path, and the path that needs to be verified is the verification path. The verification path includes one or more verification subpaths. A verification subpath is constituted by a verification displacement path segment and a verification avoidance path segment. After determining the sorting result of the plurality of operation positions (for example, the first sorting result), the processing device 130 performs a simulation run according to the movement path of the end effector planned based on the sorting result. That is, the planning of the overall movement path is verified. This operation process is referred to as path verification. For the planned movement path, it should be ensured that the overall path can smoothly and sequentially reach all of the operation positions. The overall movement path is only allowed to enter a subsequent intraoperative (i.e., formally entering the interventional surgical procedure) stage after the overall movement path is successfully verified at one time. Planning the verification path may comprise sequentially planning the verification subpaths between each set of adjacent operation positions in the sorting result. The manner of planning the verification subpaths is similar to the manner of planning the subpaths. Refer to FIG. 11 and FIG. 12.

In some embodiments, in addition to verifying the planned movement path of the end effector, the processing device 130 may also perform other verifications related to surgical operations. For example, the processing device 130 may verify at least one of: whether a working channel (for example, a needle path, or the like) interferes with important tissues, whether the working channel is drawn incorrectly, or whether the length of the working channel satisfies corresponding instrument requirements, or the like. In some embodiments, if at least one of these other verifications fails, the processing device 130 may also determine that the planning of the movement path of the end effector fails. In some embodiments, if the planned movement path of the end effector is successfully verified, and the other verifications are also successful, then the processing device 130 may determine that the planning of the movement path of the end effector is successful.

In some embodiments, after the planning of the movement path of the end effector is successful, the processing device 130 determines target surgical planning information, and a sorting result of the plurality of target operation positions in the target surgical planning information, which is the second sorting result. Regarding how to determine the second sorting result, refer to relevant content of step S550. In some embodiments, determining target surgical planning information is performed after the path verification passes.

FIG. 11 is a flowchart illustrating an exemplary process for subpath planning in a system for interventional medicine according to some embodiments of the present disclosure. As shown in FIG. 11, a process 1100 includes the following steps. In some embodiments, the processing device 130 or a path planning module 420 plans the displacement path segment and the avoidance path segment in the subpath of the end effector by executing at least a part of the process 1100. The manner shown in the process 1100 is applicable to the planning of at least one of the verification subpath, the execution subpath, or the like.

Step S1110, for each set of adjacent operation positions, planning an avoidance point based on a preceding operation position of the adjacent operation positions, and planning a displacement path segment based on the preceding operation position and the avoidance point.

The adjacent operation positions refer to two sequentially connected preceding and subsequent operation positions in the sorting result of the plurality of operation positions (for example, the first sorting result, the second sorting result), wherein there are no other operation positions between the two operation positions in the sorting result. The preceding operation position refers to an operation position that is sequentially preceding in the sorting result in a set of adjacent operation positions. Correspondingly, the subsequent operation position refers to an operation position that is sequentially subsequent in the sorting result in a set of adjacent operation positions.

In order to prevent uncontrolled interference and collision between the end effector and surgical instruments (for example, a puncture needle) remaining on a patient's body during an interventional surgical procedure, for an initial path segment when moving from the preceding operation position to the subsequent operation position among the adjacent operation positions, the processing device 130 causes the end effector to retract horizontally for a distance. This operation is referred to as end effector retraction, and this retraction path is a displacement path segment.

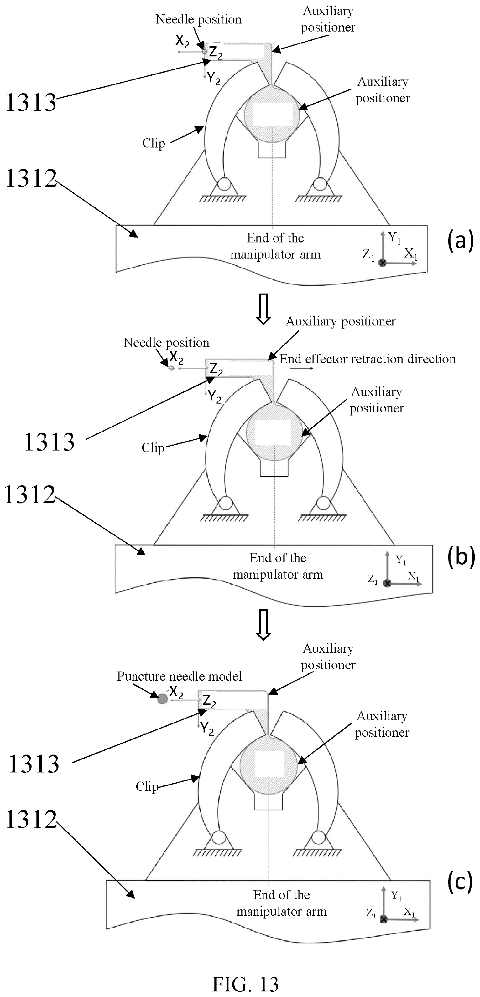

Merely by way of example, FIG. 13 is a schematic diagram of an exemplary end effector retraction of an interventional surgical robot according to some embodiments of the present disclosure. As shown in FIG. 13, Sub-FIG. (a) to Sub-FIG. (c) are schematic diagrams illustrating the end effector retraction process observed from a front view. Sub-FIG. (a) is a state diagram when an end effector 1313 (for example, an auxiliary positioner) reaches a target position. The end effector 1313 is mounted and clamped at the end of a manipulator arm 1312. The end effector 1313 is an auxiliary positioner, and has a positioning groove at the end of the end effector 1313. The positioning groove is used for direction positioning of a needle path when a surgical instrument (for example, a puncture needle) is being punctured. Sub-FIG. (b) is a state diagram when the end effector 1313 performs end effector retraction. After a surgical instrument puncture is completed, the end effector 1313 is caused to retract horizontally for a distance toward an opposite direction of the opening direction of the positioning groove (i.e., the end effector retraction direction in Sub-FIG. (b)), so as to avoid collision between the end effector 1313 and a surgical instrument remaining on a patient's body. This retraction path is the displacement path segment. Sub-FIG. (c) is a state diagram after the end effector 1313 completes end effector retraction, and a puncture needle model (a surgical instrument model) is added at a preceding puncture position (the preceding operation position).

In some embodiments, when the end effector moves in the displacement path segment, the posture of the end effector at the end of the manipulator arm remains unchanged, and the position of the end effector moves horizontally for a distance. For example, as shown in FIG. 13, the end effector 1313 is an auxiliary positioner. In the displacement path segment, the end effector 1313 moves horizontally for a distance toward an opposite direction (i.e., the opposite direction of the opening direction of the positioning groove) of a projection of an X₂ axis in a horizontal plane of an end effector coordinate system (a coordinate system constituted by X₂, Y₂, and Z₂). The posture of the end effector 1313 remains unchanged during the movement.

In some embodiments, the processing device 130 determines an avoidance point on a path between the preceding operation position and the subsequent operation position. The avoidance point refers to a retraction point set in the subpath to avoid interference between the end effector (or a model thereof) and a surgical instrument (or a model thereof) remaining at the preceding operation position. The path between the preceding operation position and the avoidance point is the displacement path segment. Merely by way of example, FIG. 12 is a schematic diagram illustrating an exemplary overall path planning in a system for interventional medicine according to some embodiments of the present disclosure. As shown in FIG. 12, 1214 represents a surgical instrument remaining on a patient's body. The position of an end effector 1213 is an avoidance point, and the position of an end effector 1213-1 is a position after the end effector 1213 is retracted. Path segments represented by arrows 3, 5, 7, and 9 in FIG. 12 are all displacement path segments.

In some embodiments, the processing device 130 presets a retraction distance of the end effector. Then, based on the retraction distance and a conversion relationship before retraction between an end effector coordinate system (a coordinate system constituted by X₂, Y₂, and Z₂ as shown in FIG. 13) and a manipulator arm coordinate system (a manipulator arm coordinate system constituted by X₁, Y₁, and Z₁ as shown in FIG. 12 and FIG. 13), the processing device 130 obtains a conversion relationship after retraction between the end effector coordinate system and the manipulator arm coordinate system. Then, based on the conversion relationship, the processing device 130 obtains a position of a verification avoidance point, namely, coordinates of the verification avoidance point in the manipulator arm coordinate system.

In some embodiments, when planning the displacement path segment of the end effector by using a joint linear interpolation algorithm, it is necessary to determine the position of the avoidance point first.

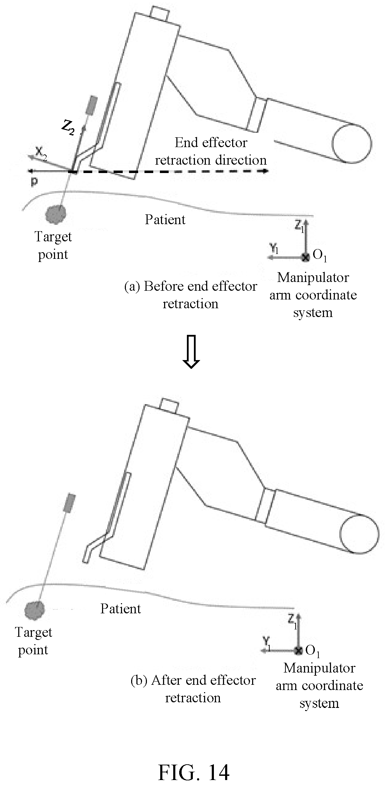

Merely by way of example, FIG. 14 is a schematic diagram of an exemplary front view when an end effector of an interventional surgical robot performs end effector retraction according to some embodiments of the present disclosure. FIG. 15 is a schematic diagram of an exemplary retreat direction when an end effector of an interventional surgical robot performs end effector retraction according to some embodiments of the present disclosure. Referring to FIG. 14 and FIG. 15, an end effector retraction direction of an end effector (for example, an end effector 1513) is an opposite direction of a projection p direction of an X₂ axis in a horizontal plane of an end effector coordinate system (a coordinate system constituted by X₂, Y₂, and Z₂ as shown in FIG. 14 and FIG. 15). An end effector retraction distance (i.e., a distance of a displacement path segment) is defined as dis.

Hereinafter, the determination of the displacement path segment is described by taking as an example the end effector coordinate system constituted by X₂, Y₂, and Z₂ and the manipulator arm coordinate system constituted by X₁, Y₁, and Z₁ as shown in FIG. 14 and FIG. 15. In some embodiments, the principle for determining the displacement path segment is as follows. Assuming that before the end effector performs avoidance, a transformation matrix of the end effector coordinate system relative to the manipulator arm coordinate system is shown in the following Formula (1): ${T}_{BaseToClutch} = \left[\begin{matrix}{a}_{x} & {b}_{x} & {c}_{x} & {p}_{x} \\ {a}_{y} & {b}_{y} & {c}_{y} & {p}_{y} \\ {a}_{z} & {b}_{z} & {c}_{z} & {p}_{z} \\ 0 & 0 & 0 & 1\end{matrix}\right]$ (1), where, the transformation matrix *T*_{BaseToClutch} represents a homogeneous transformation matrix of the end effector coordinate system relative to the manipulator arm coordinate system before the end effector performs a verification displacement path segment. In Formula (1), *a*ₓ, *a*_{y}, and *a*_{z} respectively represent projection components of an X₂ axis unit vector of the end effector coordinate system on the X₁, Y₁, and Z₁ axes of a robot base coordinate system; *b*ₓ, *b*_{y}, and *b*_{z} respectively represent projection components of a Y₂ axis unit vector of the end effector coordinate system on the X₁, Y₁, and Z₁ axes of the robot base coordinate system; *c*ₓ, *c*_{y}, and *c*_{z} respectively represent projection components of a Z₂ axis unit vector of the end effector coordinate system on the X₁, Y₁, and Z₁ axes of the robot base coordinate system; and *p*ₓ, *p*_{y}, and *p*_{z} represent coordinates of an origin O₂ of the end effector coordinate system in the robot base coordinate system.

The horizontal plane refers to a plane constituted by the X₁ axis and the Y₁ axis of the manipulator arm coordinate system. An avoidance distance *Δₓ* of the end effector in an X₁ axis direction and an avoidance distance *Δ_{y}* in a Y1 axis direction are shown in the following Formula (2) and Formula (3): ${Δ}_{x} = dis ∗ \frac{{a}_{x}}{\sqrt{{{a}_{x}}^{2} + {{a}_{y}}^{2}}}$ (2), ${Δ}_{y} = dis ∗ \frac{{a}_{y}}{\sqrt{{{a}_{x}}^{2} + {{a}_{y}}^{2}}}$ (3), where, dis is an end effector retraction distance (i.e., a distance of the displacement path segment).

Based on Formula (1) to Formula (3), after the end effector retracts (that is, after moving along the displacement path segment), a transformation matrix of the end effector coordinate system (where a coordinate system constituted by X'2, Y'2, and Z'2 in FIG. 15 represents the end effector coordinate system after end effector retraction) relative to the manipulator arm coordinate system is shown in the following Formula (4): ${T}_{BaseToCl} ′ = \left[\begin{matrix}{a}_{x} & {b}_{x} & {c}_{x} & {p}_{x} - {Δ}_{x} \\ {a}_{y} & {b}_{y} & {c}_{y} & {p}_{y} - {Δ}_{y} \\ {a}_{z} & {b}_{z} & {c}_{z} & {p}_{z} \\ 0 & 0 & 0 & 1\end{matrix}\right]$ (4), where, the transformation matrix ${T}_{BaseToClutch}^{′}$ represents a homogeneous transformation matrix of the end effector coordinate system relative to the robot base coordinate system after the end effector retracts along the displacement path segment; *a*ₓ, *a*_{y}, *a*_{z}, *b*ₓ, *b*_{y}, *b*_{z}, *c*ₓ, c_{y}, *c*_{z}, *p*ₓ, *p*_{y}, and *p*_{z} have the same meanings as those in Formula (1) to Formula (3).

The processing device 130 obtains an inverse solution of ${T}_{BaseToClutch}^{′}$ after the end effector retracts, to obtain target joint angle data of the end effector. The obtained target joint angle data is the position of the avoidance point. Then, the processing device 130 takes the preceding operation position as a starting point and the avoidance point as a target point, and performs path planning for the displacement path segment.

In some embodiments, the planning of a verification displacement path segment adopts a joint linear interpolation algorithm (i.e., a MoveJ algorithm). The algorithm connects a straight path from a starting point (i.e., the preceding operation position) to a target point (i.e., the avoidance point). If no obstacle exists between the starting point and the target point, planning of the displacement path segment is successful. If an obstacle exists between the starting point and the target point, planning of the displacement path segment fails.

Step S1120, planning the avoidance path segment between the avoidance point and the subsequent operation position of the adjacent operation positions.

After planning the avoidance point, the processing device 130 plans the avoidance path segment between the avoidance point and the subsequent operation position of the adjacent operation positions, wherein the displacement path segment and the avoidance path segment constitute the subpath. For example, as shown in FIG. 12, the avoidance path segment between the avoidance point and the subsequent puncture position of the adjacent puncture positions is a path segment composed of a first avoidance path segment (indicated by arrow 4), a second avoidance path segment (indicated by arrow 6), and a third avoidance path segment (indicated by arrow 8). The avoidance path segment and the displacement path segment constitute the subpath. Therefore, the subpath represents the movement path from the preceding operation position to the subsequent operation position of the adjacent operation positions.

The manner for the processing device 130 to plan the avoidance path segment is similar to the manner for planning the displacement path segment. For example, the processing device 130 may also use a joint linear interpolation algorithm (i.e., the MoveJ algorithm) to plan the avoidance path segment. In this case, the algorithm may connect a straight path from the starting point (i.e., the avoidance point) to the target point (i.e., the subsequent operation position). If no obstacle exists between the starting point and the target point, planning of the avoidance path segment is successful. If an obstacle exists between the starting point and the target point, planning of the avoidance path segment fails.

In some embodiments, after the end effector moves along the displacement path segment, the processing device 130 adds the surgical instrument model corresponding to the preceding operation position to the collision model to update the collision model. The surgical instrument model is a model for simulating surgical instrument entities (for example, a puncture needle, or the like) remaining on the body surface of the patient during an interventional surgical procedure and their movement trajectories. Since the positions of these surgical instruments relative to the patient are relatively fixed, their movement may be equated to the movement of the patient model.

In some embodiments, the processing device 130 plans the avoidance path segment between the avoidance point and the subsequent operation position of the adjacent operation positions based on the updated collision model, wherein the displacement path segment and the avoidance path segment constitute the subpath.

To illustrate with an example of planning a verification subpath, for any verification subpath, the processing device 130 may verify by simulation whether the end effector in the updated collision model will collide with at least one of the surgical instrument model, the patient model, the scanning device model, or the like. If the simulation result is that the end effector will collide with any one or a plurality of the three, then the verification of the verification subpath fails, and the movement path planning of the end effector is determined to be unsuccessful. If the simulation result is that the end effector will not collide with any one of the three, then the verification of the verification subpath is successful, and a next verification subpath is verified. When all verification subpaths are successfully verified, the entire movement path planning of the end effector is successful.

In some embodiments of the present disclosure, by retracting the end effector, the surgical instrument remaining on the body surface of the patient and the gripper of the end effector may be separated first by a distance, so as to avoid interference and collision between the end effector and the surgical instrument remaining on the body surface of the patient, thereby avoiding safety risks. Meanwhile, the surgical instrument model of the preceding operation position may also be added to the collision model at the avoidance point.

In some embodiments, the subpath includes an execution subpath, and the avoidance point includes an execution avoidance point. The displacement path segment includes an execution displacement path segment, the avoidance path segment includes an execution avoidance path segment, and the execution subpath is constituted by the execution displacement path segment and the execution avoidance path segment.

For each set of adjacent operation positions, the processing device 130 plans the execution avoidance point based on the preceding operation position of the adjacent operation positions, and plans the execution displacement path segment based on the preceding operation position and the execution avoidance point. The processing device 130 executes the planned execution displacement path segment, so as to move the end effector to the execution avoidance point. The processing device 130 plans the execution avoidance path segment between the execution avoidance point and the subsequent operation position of the adjacent operation positions. The processing device 130 executes the planned execution avoidance path segment, so as to move the end effector to the subsequent operation position of the adjacent operation positions. The manner for the processing device 130 to plan the execution displacement path segment, the execution avoidance point, and the execution avoidance path segment is similar to the corresponding manner in planning a verification subpath, and details will not be repeated herein.

In some embodiments, the processing device 130 adds the surgical instrument model corresponding to the preceding operation position of the adjacent operation positions to the collision model to update the collision model, and plans the execution avoidance path segment between the execution avoidance point and the subsequent operation position of the adjacent operation positions based on the updated collision model. The operation manner of the step is similar to the corresponding manner in planning a verification subpath, and details will not be repeated herein.

In some embodiments, in the online mode, planning of the execution subpath is performed intraoperatively, that is, the subpath is planned and executed based on a real-time image. For more details on how to plan and execute the execution subpath in the online mode, refer to Step S2070 and Steps S2120 to S2160.

FIG. 16 is a flowchart illustrating an exemplary process for planning an execution subpath in a system for interventional medicine according to some embodiments of the present disclosure. As shown in FIG. 16, the process 1600 includes the following steps. In some embodiments, after the execution subpath planning fails, the processing device 130 or the path execution module 430 re-plans the execution subpath by executing at least a part of the process 1600.

Step S1610, obtaining a first correction position of the end effector.

In some embodiments, during the planning process of the execution subpath, when planning of the execution displacement path segment included therein fails, the processing device 130 obtains the first correction position of the end effector. The processing device 130 automatically or manually by a user drags the end effector to a new relatively safe position, and uses it as the first correction position.

After obtaining the first correction position of the end effector, the processing device 130 executes any one of Steps S1620-S1640 to plan the execution displacement path segment.

Step S1620, planning the execution displacement path segment based on the first correction position and the execution avoidance point.

In some embodiments, after obtaining the first correction position, the processing device 130 directly plans the execution displacement path segment based on the first correction position and the planned execution avoidance point, so as to move the end effector from the first correction position to the planned execution avoidance point along the execution displacement path segment. For example, the processing device 130 uses the first correction position as the preceding operation position, and plans the execution displacement path segment from the first correction position to the planned execution avoidance point.

Step S1630, re-planning the execution avoidance point based on the first correction position, and planning the execution displacement path segment based on the first correction position and the re-planned execution avoidance point.

In some embodiments, after obtaining the first correction position, since the position of the end effector has been changed to the first correction position, to avoid a collision, the processing device 130 re-plans the execution avoidance point, and plans the execution displacement path segment based on the first correction position and the re-planned execution avoidance point, so as to move the end effector from the first correction position to the re-planned execution avoidance point along the execution displacement path segment. For example, the processing device 130 uses the first correction position as the preceding operation position, and plans the execution displacement path segment from the first correction position to the re-planned execution avoidance point.

Step S1640, determining the first correction position as the re-planned execution avoidance point.

In some embodiments, after obtaining the first correction position, the processing device 130 determines whether an available path can be directly planned to move from the first correction position to the subsequent operation position without interference with the surgical instrument of the preceding operation position. When the available path is directly planned to move from the first correction position to the subsequent operation position without interference with the surgical instrument of the preceding operation position, the processing device 130 determines the first correction position as the re-planned execution avoidance point, so as to execute subsequent path planning. In this case, it may not be necessary to plan the execution displacement path segment.

Step S1650, obtaining a second correction position of the end effector.

In some embodiments, during the planning process of the execution subpath, when planning of the execution avoidance path segment included therein fails, the processing device 130 obtains a second correction position of the end effector. The processing device 130 automatically or manually by a user drags the end effector to a new relatively safe position, and uses it as the second correction position.

After obtaining the second correction position of the end effector, the processing device 130 executes Step S1660 to plan the execution avoidance path segment.

Step S1660, planning the execution avoidance path segment based on the second correction position and the subsequent operation position of the adjacent operation positions.

After obtaining the second correction position of the end effector, the processing device 130 uses the second correction position as a new execution avoidance point, and uses the path segment from the second correction position to the subsequent operation position as the execution avoidance path segment.

In an actual operation scenario, failure in planning the execution displacement path segment and failure in planning the execution avoidance path segment may occur simultaneously, or one of them may occur independently. In some embodiments, when the two occur simultaneously, the processing device 130 first handles the case of failure in planning the execution displacement path segment by executing Steps S1610-S1640, so as to plan the execution displacement path segment. Then, the processing device 130 handles the case of failure in planning the execution avoidance path segment by executing Steps S1650-S1660 to plan the execution avoidance path segment. In some embodiments, for the case where one of them occurs independently, the processing device 130 executes Steps S1610-S1640 or Steps S1650-S1660.

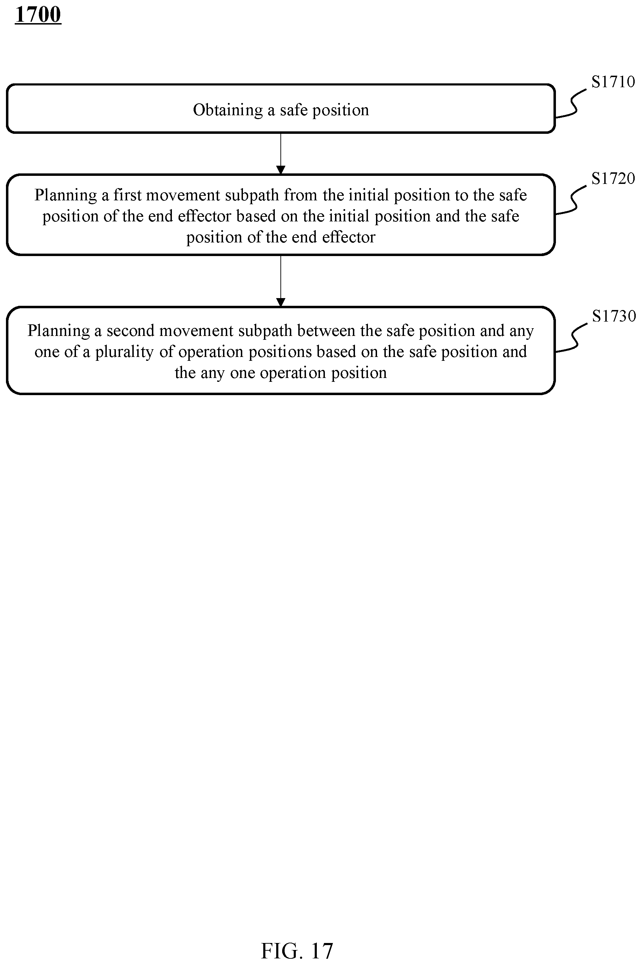

FIG. 17 is a flowchart illustrating an exemplary process for overall path planning in a system for interventional medicine according to some embodiments of the present disclosure. As shown in FIG. 17, the process 1700 includes the following steps. In some embodiments, the processing device 130 or the path planning module 420 implements step S520 by executing at least a part of the process 1700, to plan a movement path of the end effector in an interventional mode based on surgical planning information.

Step S1710: obtaining a safe position.

The safe position is also referred to as an external safe point. The safe position refers to a position where no interference occurs between any plurality of a medical imaging device, a scanning table, a manipulator arm, a surgical instrument, and an end effector. The safe position is a pre-designated point located outside of a scanning chamber of the medical imaging device (for example, the scanning device 111). The safe position is determined based on three-dimensional information of the medical imaging device. In some embodiments, the processing device 130 presets the safe position as an installation point of the end effector. The installation of the end effector refers to an action of connecting and fixing the end effector to an end of the manipulator arm. In some embodiments, in an online mode, the processing device 130 presets the safe position as an installation point of the surgical instrument. The installation of the surgical instrument refers to an action of connecting the end effector and the surgical instrument. In some embodiments, the safe position is set in advance before each interventional surgical procedure and remains fixed and unchanged throughout the entire interventional surgical procedure. In some embodiments, the safe position is a transition position for controlling the movement of the end effector from away from an operation position to close to the operation position, and is referred to as a first transition point. In some embodiments, the safe position is a transition position for controlling the movement of the end effector from close to an operation position to away from the operation position, and is referred to as a second transition point. In some embodiments, the safe position is an initial position of the manipulator arm and the end effector at the start of a surgical procedure. In some embodiments, the safe position is an initial position of the scanning table at the start of a surgical procedure. The safe position in different embodiments may be the same position or may be different positions.

In some embodiments, in the online mode, in a preparation phase of the system for interventional medicine, an end gripper is installed, then a surgical instrument is installed, and the processing device 130 controls the manipulator arm to move to an operation position, that is, an intervention point. For example, the end effector and the surgical instrument may both be installed at the safe position. As another example, the safe position is used as a transition point, and at least one of the end effector and the surgical instrument is installed at other positions than the safe position.

In some embodiments, in an offline mode, an auxiliary positioner is first installed, then the processing device 130 automatically controls or a user manually controls the manipulator arm to move to the intervention point, and then the surgical instrument is installed. For example, the end effector is installed at the safe position, while the surgical instrument is installed after the manipulator arm moves to the operation position. As another example, the safe position is used as a transition point, the end effector is installed at other positions than the safe position, while the surgical instrument is installed after the manipulator arm moves to the operation position.

In some embodiments, the processing device 130 determines the safe position based on pose information of the end effector. In the offline mode or the online mode, the safe position is the first transition point. In steps S1720-S1730, the safe position is consistently the first transition point. However, an external space of the medical imaging device is relatively large, and a selectable range of the safe position is relatively wide. Determining the safe position based on pose information of the end effector can improve the installation convenience of the end effector and the surgical instrument.

Merely by way of example, assume that the end effector corresponding to the safe position is about 1500mm away from an outer wall of the medical imaging device in a horizontal direction, and the safe position is far away from the medical imaging device. A distance in a vertical height between a lowest point of the end effector corresponding to the safe position and the scanning table exceeds 500mm. The distance is much higher than a thickness of a patient lying flat. Therefore, the safe position is a sufficiently safe position for the end effector, which can effectively avoid collisions between the end effector and environmental objects such as a patient, a medical imaging device, or the like, when the end effector is installed to the manipulator arm.

Step S1720: planning a first movement subpath from the initial position to the safe position of the end effector based on the initial position and the safe position of the end effector.

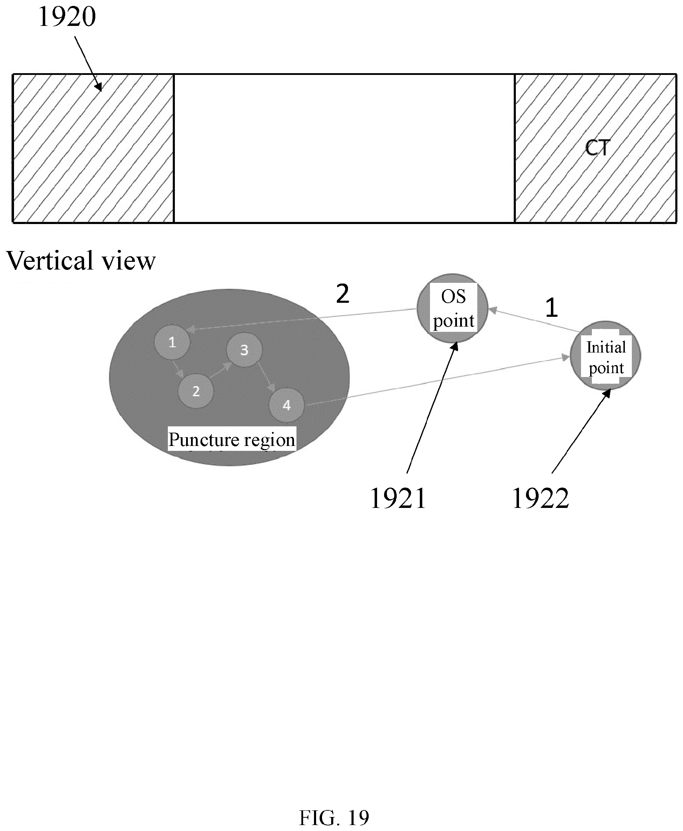

The processing device 130 uses the initial position as a starting point and the safe position as an end point, and plans the first movement subpath from the initial position to the safe position. For example, the first movement subpath may be a path segment indicated by arrow 1 in FIG. 12 and arrow 1 in FIG. 19. The manner of planning the first movement subpath is similar to a manner of planning a subpath, which will not be described in detail herein.

In some embodiments, the processing device 130 plans the first movement subpath from the initial position to the safe position of the end effector based on a collision model. The manner of planning the first movement subpath based on the collision model is similar to the manner of planning a subpath, which will not be described in detail herein.

Step S1730: planning a second movement subpath between the safe position and any one of a plurality of operation positions based on the safe position and the any one operation position.

The processing device 130 uses the safe position as a starting point and any one operation position of the plurality of operation positions as an end point, and plans the second movement subpath between the safe position and the any one operation position (for example, a first operation position in a first sorting result or a second sorting result). For example, the second movement subpath may be a path segment indicated by arrow 2 in FIG. 12 and FIG. 19. The manner of planning the second movement subpath is similar to the manner of planning a subpath, which will not be described in detail herein.

In some embodiments, the processing device 130 plans the second movement subpath of the end effector between the safe position and any one operation position based on the collision model. The manner of planning the second movement subpath based on the collision model is similar to the manner of planning a subpath, which will not be described in detail herein.

Merely by way of example, FIG. 19 is a schematic diagram illustrating an exemplary overall path planning in a system for interventional medicine according to some embodiments of the present disclosure. As shown in FIG. 19, the safe position is 1921, the initial position of the end effector is 1922, the CT machine (a medical imaging device) is 1920, the path segment indicated by arrow 1 is the first movement subpath, the path segment indicated by arrow 2 is the second movement subpath, and 1, 2, 3, and 4 in a puncture region represent four puncture positions. Before an interventional surgical procedure, the manipulator arm may be located at an initial point 1922 (hereinafter referred to as a Home point). When the interventional surgical procedure is performed, the manipulator arm moves from the initial point 1922 to an OS point, that is, the safe position 1921. At the safe position 1921, after the end effector is installed to an end of the manipulator arm, the end effector moves along the second movement subpath to a first puncture position 1 in the puncture region. Then, a physician, under the guidance of the end effector, sequentially performs an interventional operation of a surgical instrument (for example, a puncture needle) along the puncture position 1, the puncture position 2, the puncture position 3, and the puncture position 4. After the interventional operation is completed, the processing device 130 continues to plan and execute a subsequent movement path of the end effector, until interventional actions of the surgical instrument are completed at all puncture positions, and the manipulator arm drives the end effector back to the safe position 1921. Finally, the manipulator arm retreats to the initial position 1922, and the surgical procedure ends.

As another example, as shown in FIG. 12, for a situation of puncture by four puncture needles, planning is performed sequentially from left to right according to a sorting result, the path segment indicated by arrow 1 is the first movement subpath, and the path segment indicated by arrow 2 is the second movement subpath. The manipulator arm first moves along a first movement subpath from an initial position to a safe position. After the end effector is installed at the safe position, the manipulator arm moves along a second movement subpath to the puncture position of the first needle. In the online mode, after the end effector is installed, a surgical instrument may also be installed at the safe position. In the offline mode, after the manipulator arm moves to the puncture position of the first needle, a surgical instrument is installed. After the first needle puncture is completed, the end effector 1213 performs end retraction along the path indicated by arrow 3. The end effector after end retraction is the end effector 1213-1. Next, the processing device 130 plans and executes to the puncture position of the second needle (the path indicated by arrow 4), and then plans the paths of the subsequent three puncture needles based on this (the paths indicated by arrows 5-8). After the puncture of the last puncture needle is completed, the manipulator arm performs end retraction along the path indicated by arrow 9. Finally, the end effector moves along the path indicated by arrow 10 to the initial position. The entire movement path of the end effector is composed of the ten path segments indicated by arrows 1-10. It must be ensured that these ten path segments are all successfully planned at one time before entering an intraoperative execution stage. If any path segment planning fails, a failure will be returned and the user will be prompted to adjust the needle path, and then continue to perform path planning verification on the overall path until successful.

In some embodiments of the present disclosure, by using the safe position for transition of the entire path on the entire movement path of the end effector, the relative fixedness of portions of the movement path is ensured (for example, from the initial position of the end effector to the first operation position, and from the last operation position to the initial position of the end effector), and the path planning is also made faster and more accurate.

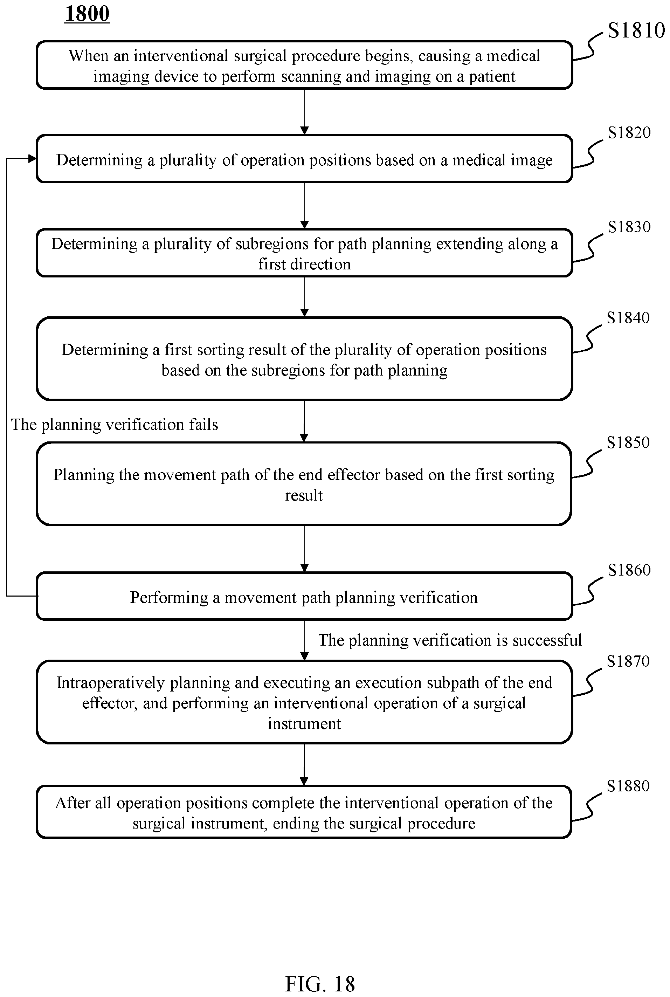

FIG. 18 is a flowchart illustrating an exemplary overall workflow of a system for interventional medicine in an interventional surgical procedure according to some embodiments of the present disclosure. As shown in FIG. 18, a process 1800 includes the following steps. In some embodiments, the processing device 130 or modules of the system 400 implement the process 500 by executing at least a part of the process 1800, to plan the movement path of the end effector in the interventional mode. The manner shown in the process 1800 is applicable to the offline mode and the online mode.

Step S1810, when an interventional surgical procedure begins, causing a medical imaging device (for example, the scanning device 111) to perform scanning and imaging on a patient.

Step S1820, determining a plurality of operation positions based on a medical image. The medical image includes a medical radiation image, an ultrasound scanning image, or the like. The medical radiation image includes CT images, MR images, PET images, or the like.

Step S1830, determining a plurality of subregions for path planning extending along a first direction.

Step S1840, determining a first sorting result of the plurality of operation positions based on the subregions for path planning.

Step S1850, planning the movement path of the end effector based on the first sorting result.

Step S1860, performing a movement path planning verification. If the planning verification fails, the processing device 130 returns to execute Step S1820 until the planning verification is successful, then Step S1870 is executed.

Step S1870, intraoperatively planning and executing an execution subpath of the end effector, and performing an interventional operation of a surgical instrument.

Step S1880, after all operation positions complete the interventional operation of the surgical instrument, ending the surgical procedure.

For specific operation manners in Steps S1820-S1880, refer to FIGS. 5, 6, 11, 16, 17, 20, and 21.

FIG. 20 is a flowchart illustrating an exemplary path planning of an interventional surgical robot in a system for interventional medicine according to some embodiments of the present disclosure. As shown in FIG. 20, a process 2000 includes the following steps. In some embodiments, the processing device 130 or modules of the system 400 implement at least a part of the process 500 by executing at least a part of the process 2000, to plan the movement path of the end effector in the interventional mode based on the surgical planning information. The manner shown in the process 2000 is applicable to the online mode. In the online mode, the surgical planning information further includes a plurality of working channels corresponding to the plurality of operation positions, a data connection exists between the system for interventional medicine and a medical imaging system, and the medical imaging system includes a scanning device and a scanning table. In some embodiments, at least a part of the process 2000 is applicable to the offline mode. For example, some or all of Steps S2010-S2060. Further for example, a preoperative cephalocaudal tilt operation is performed on a gantry in Step S2070.

Step S2010, obtaining first position information of the scanning device, second position information of the scanning table, and the surgical planning information. In some embodiments, Step S2010 is executed by the planning acquisition module 410. For Step S2010, refer to Step S510.

Step S2020, planning the movement path of the end effector based on the first position information, the second position information, and the surgical planning information. In some embodiments, Step S2020 is executed by the path planning module 420.

The processing device 130 sorts the plurality of operation positions in the surgical planning information, determines a first sorting result, and plans the movement path of the end effector based on the first sorting result. For detailed content on how to plan the movement path of the end effector based on the first sorting result, refer to content related to FIG. 6.

In some embodiments, the processing device 130 determines a collision model based on the first position information and the second position information. Specifically, since the patient is placed on the scanning table and is fixed in position relative to the scanning table, the processing device 130 determines relative positions of the scanning device and the scanning table at any time based on real-time positions of the scanning device and the scanning table, that is, relative positions of the scanning device and the patient at any time are also determined. Thus, when a patient model is used to represent the patient and a scanning device model is used to represent the scanning device, the processing device 130 determines the collision model based on the relative positions of the scanning device and the patient at any time.

In some embodiments, in response to an inclination of one or more working channels (for example, the needle paths for puncture in an interventional puncture surgical procedure) relative to a second direction, the processing device 130 determines rotation information of the scanning device toward the second direction, wherein the second direction is parallel to the long axis of the scanning table. Since the cephalocaudal direction of the patient on the scanning table is parallel to the long axis of the scanning table, the rotation of the scanning device toward the second direction is equivalent to a collimation center line of the scanning device tilting toward the cephalocaudal direction, thereby facilitating observation of cephalocaudal tilted needle paths. In the present disclosure, the cephalocaudal tilt refers to tilting relative to the cephalocaudal direction of the patient. The processing device 130 obtains inclination angles of one or more working channels relative to the long axis of the scanning table, which can be referred to as the cephalocaudal inclination angles of the working channels in a puncture surgical procedure. The processing device 130 determines a rotation angle of the scanning device toward the second direction based on a cephalocaudal inclination angle of a working channel, that is, an angle between the collimation center line of the scanning device and the long axis of the scanning table, wherein the angle is referred to as the inclination angle of the scanning device. The inclination angle of the scanning device and the cephalocaudal inclination angle of the working channel may be equal, or a difference between the two satisfies a preset condition, wherein the preset condition is that the working channel is within a scanning range of the scanning device. When the scanning device is a CT, a rotation of the scanning device is equivalent to a rotation of a CT gantry. Therefore, the inclination angle of the scanning device at this time is referred to as a CT gantry inclination angle.

In some embodiments, the scanning table moves (for example, moves along the second direction and/or a third direction), and the processing device 130 determines movement information of the scanning table based on rotation information of the scanning device toward the second direction. Regarding how to determine the movement information of the scanning table, refer to related descriptions of step S2150.

In some embodiments, when planning a path between the operation positions of one or more working channels and the adjacent operation positions, the processing device 130 determines a scanning device model based on the first position information and the rotation information, and determines a patient model based on the second position information and the movement information. Since the position of the scanning device changes after the scanning device rotates toward the second direction, the processing device 130 determines the scanning device model after rotating toward the second direction based on original position information of the scanning device (i.e., the first position information) and change information of the position (i.e., the rotation information). Since the position of the patient changes after the scanning table moves, the processing device 130 determines the patient model after moving based on original position information of the scanning table (i.e., the second position information) and change information of the position (i.e., the movement information).

In some embodiments of the present disclosure, by inclining the gantry of the scanning device (for example, a CT machine, or the like) toward the craniocaudal direction, it is convenient to observe a complete needle path during an interventional surgical procedure, especially a needle path inclined toward the craniocaudal direction, thereby reducing a difficulty of the interventional surgical procedure and shortening a surgery time.

In some embodiments, the processing device 130 plans the movement path of the end effector based on the collision model and a first sorting result. Specifically, the processing device 130 plans an initial movement path of the end effector according to the first sorting result, and then verifies the movement path by simulation operation according to the collision model. If during a verification process, a collision occurs between the end effector and other objects (for example, at least one of the scanning device, the patient, a surgical instrument, or the like), then the verification fails, and the path planning fails; otherwise, the verification succeeds, and the path planning succeeds.

In some embodiments of the present disclosure, by planning the movement path of the end effector based on the collision model and a sorting result of all of the operation positions (for example, needle insertion points, or the like), the path planning is more in line with actual conditions, and the planned path is more reasonable, thereby avoiding a risk of path adjustment during the interventional surgical procedure.

After the scanning table moves, the respective operation positions change positions relative to before the scanning table moves. In some embodiments, the processing device 130 updates one or more operation positions based on the movement information of the scanning table, and then plans the movement path of the end effector according to the updated one or more operation positions.

In some embodiments, after planning the movement path of the end effector based on the first sorting result, the processing device 130 performs path verification on the movement path, that is, performs simulation operation based on the collision model and the planned movement path of the end effector, and determines, through the collision model, whether the manipulator arm end model can sequentially reach the operation positions in an order of the first sorting result. If the path verification succeeds, then the movement path planning is determined to be successful; if the path verification fails, then the movement path planning is determined to be unsuccessful. The entire movement path needs to be entirely verified successfully at once before entering a subsequent intraoperative stage.

In some embodiments, the movement path may comprise a verification path, and the verification path may comprise one or more verification subpaths. The processing device 130 sequentially plans the verification subpaths each of which is between each set of adjacent operation positions in the first sorting result. Regarding detailed content on how to plan the verification subpaths, refer to FIG. 11.

When the planned movement path of the end effector is successfully verified, the processing device 130 determines that the movement path planning of the end effector is successful; when the planned movement path of the end effector fails verification, the processing device 130 determines that the movement path planning of the end effector is unsuccessful.

In some embodiments, in addition to verifying the planned movement path of the end effector, the processing device 130 may also perform other verifications related to interventional surgical procedures, for example, at least one of verifying whether a working channel (for example, a needle path, or the like) interferes with important tissues, whether the working channel is drawn incorrectly, or whether a length of the working channel meets corresponding instrument requirements. If at least one of these other verifications fails, the processing device 130 also determines that the movement path planning of the end effector is unsuccessful; if the planned movement path of the end effector is successfully verified, and all other verifications are also successful, then the processing device 130 determines that the movement path planning of the end effector is successful.

In some embodiments, after the movement path planning of the end effector fails, the processing device 130 performs steps S2030-S2050 to output a prompt message to a user and replan the movement path; after the movement path planning of the end effector succeeds, the processing device 130 performs steps S2060-S2070 to perform an interventional surgical procedure according to the planned movement path.

Step S2030, in response to a failure in planning the movement path of the end effector, outputting a prompt message. In some embodiments, step S2030 is performed by the planning prompt module 440.

When the movement path planning of the end effector fails, the processing device 130 outputs the prompt message to the user (for example, a doctor, or the like) in various manners, wherein the prompt message is determined according to a reason for the planning failure. The reason for the planning failure includes at least one of the following situations: a collision between the end effector and the patient model, a collision between the end effector and the scanning device model, a collision between the end effector and the surgical instrument model, or the like.

In some embodiments, the processing device 130 adjusts the operation positions and corresponding working channels according to the prompt message, and updates the surgical planning information by using the adjusted operation positions and corresponding working channel information, so that the subsequent replanning of the movement path can be successful after adjustment. The adjustment is performed in various manners, for example, based on adjustment suggestions, based on experience, or the like.

Merely by way of example, as shown in Table 1 hereinafter, the prompt message includes the reason for the path planning failure and adjustment suggestions (for example, adjustment suggestions for a needle path, or the like), and the processing device 130 makes adjustments based on at least one of the reason and the adjustment suggestions.

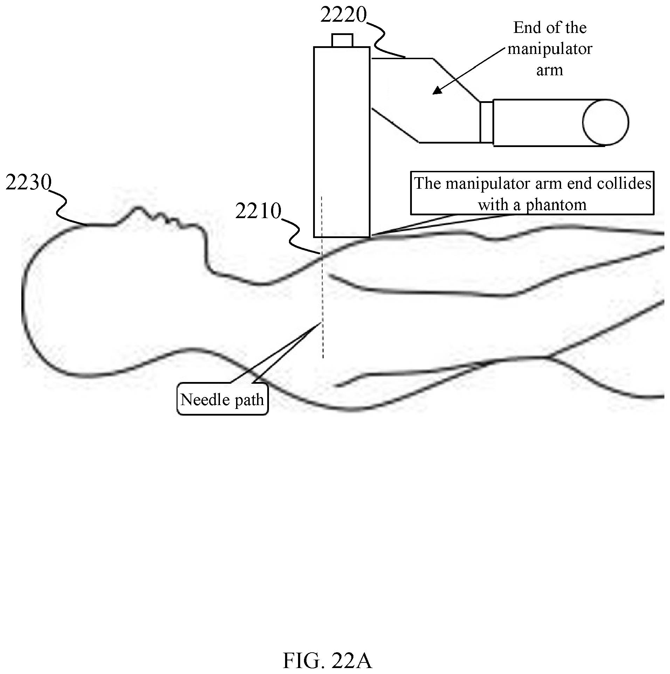

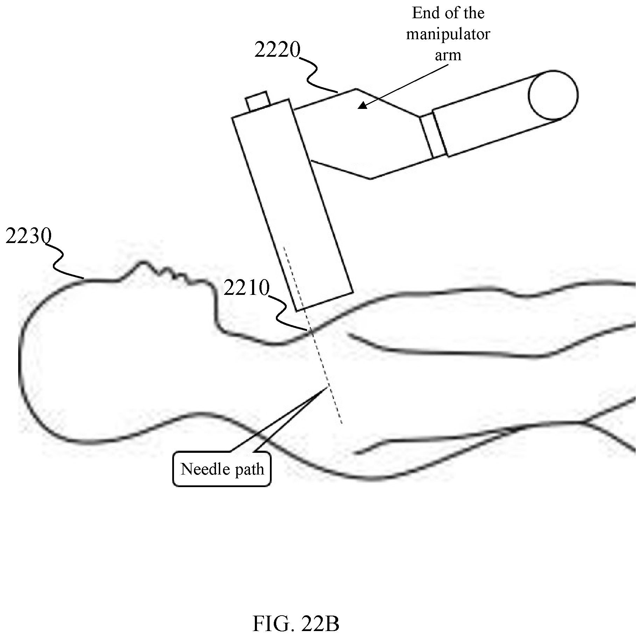

In some embodiments, in response to a path planning failure caused by a collision between the manipulator arm end model and the patient model, the processing device 130 outputs a first prompt message. The first prompt message at least instructs the user to adjust one or more operation positions in the surgical planning information. Merely by way of example, FIG. 22A is a schematic diagram illustrating an exemplary path planning failure caused by a collision between the manipulator arm end model and the patient model according to some embodiments of the present disclosure. As shown in FIG. 22A, when the planned movement path of the end effector is verified by the collision model, it is detected that at a needle insertion point 2210, the manipulator arm end 2220 in the manipulator arm end model (i.e., the end effector) collides with a phantom 2230 (i.e., the patient model), and the verification fails. At this time, the processing device 130 displays the first prompt message to the user. For example, the prompt message corresponding to serial number 1 in Table 1 hereinafter, the prompt message includes the reason for the failure and the adjustment suggestions. In some embodiments, the processing device 130 adjusts a needle insertion point (i.e., an operation position) at a failed verification location and/or a corresponding needle path (i.e., a working channel), or the like, according to the suggestions in the first prompt message. For example, according to suggestion ② of the prompt message corresponding to serial number 1 in Table 1, the needle path may be inclined toward the craniocaudal direction. In some embodiments, the processing device 130 adjusts at least one of the manipulator arm and the scanning table to change a relative position between the manipulator arm end and the patient, thereby adjusting the operation position and/or the corresponding working channel. Merely by way of example, FIG. 22B is a schematic diagram illustrating an exemplary adjustment of a needle path after the path planning failure in FIG. 22A according to some embodiments of the present disclosure. As shown in FIG. 22B, the processing device 130 adjusts a needle insertion point 2010 through movement of the manipulator arm according to the prompt message corresponding to serial number 1 in Table 1, to form a needle path inclined toward the craniocaudal direction, so that at the adjusted needle insertion point 2010, the manipulator arm end 2020 and a phantom 2030 will no longer collide.

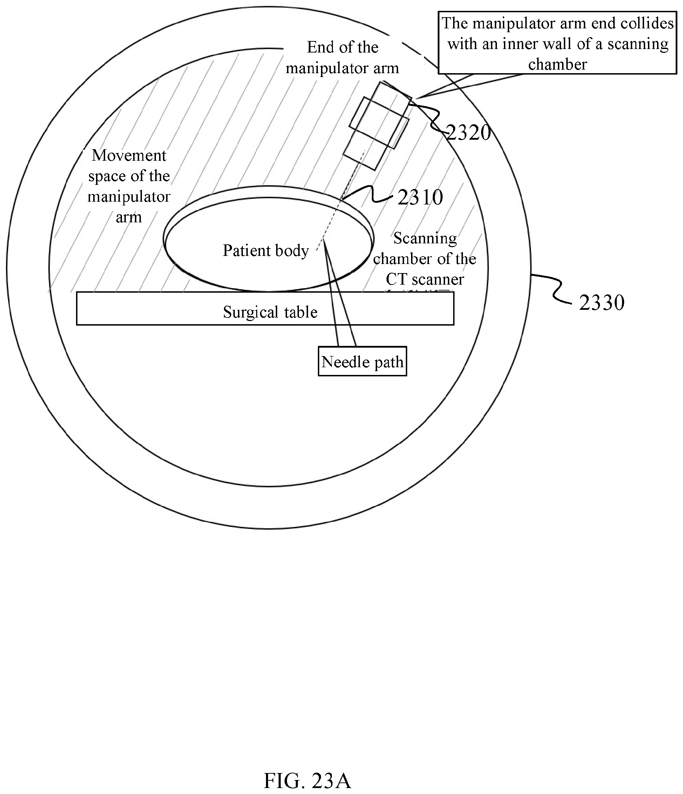

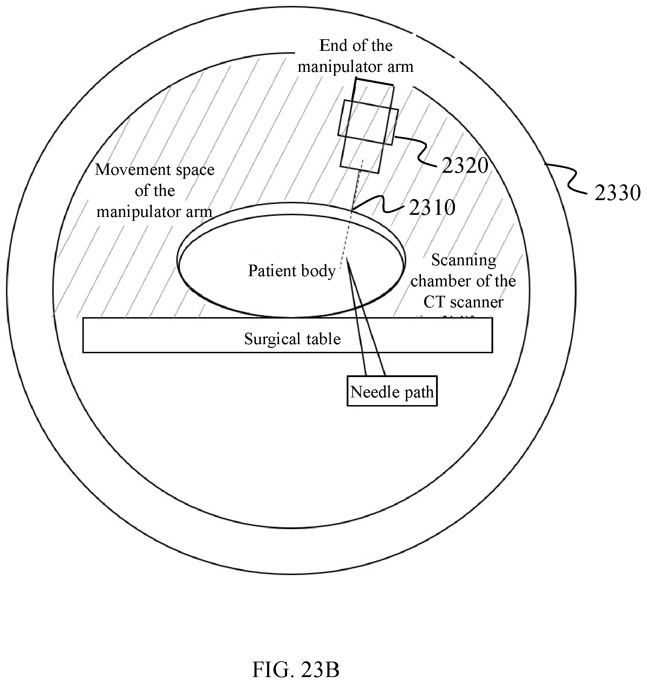

In some embodiments, in response to a path planning failure caused by a collision between the manipulator arm end model and the scanning device model, the processing device 130 outputs a second prompt message. The second prompt message instructs the user to adjust one or more operation positions in the surgical planning information or adjust the position of the scanning table. Merely by way of example, FIG. 23A is a schematic diagram illustrating an exemplary path planning failure caused by a collision between the manipulator arm end model and the scanning device model according to some embodiments of the present disclosure. As shown in FIG. 23A, when the planned movement path of the end effector is verified by the collision model, it is detected that at a needle insertion point 2310, the manipulator arm end 2320 in the manipulator arm end model (i.e., the end effector) collides with an inner wall of a scanning chamber of a CT machine 2330 (i.e., the scanning device model), and the verification fails. At this time, the processing device 130 displays the second prompt message to the user. For example, the prompt message corresponding to serial number 2 in Table 1 hereinafter, the prompt message includes the reason for the failure and the adjustment suggestions. In some embodiments, the processing device 130 adjusts a needle insertion point (i.e., an operation position) at a failed verification location and/or a corresponding needle path (i.e., a working channel), or the like, according to the suggestions in the second prompt message. For example, according to suggestion ① of the prompt message corresponding to serial number 2 in Table 1, a needle insertion point 2310 may be adjusted to a position closer to a middle of the patient's body. In some embodiments, the processing device 130 can adjust the operation position or the position of the scanning table by adjusting at least one of the manipulator arm and the scanning table, so as to change the relative position between the manipulator arm end and the patient. Merely by way of example, FIG. 23B is a schematic diagram of an exemplary adjustment of a needle path after the path planning failure in FIG. 23A according to some embodiments of the present disclosure. As shown in FIG. 23B, the processing device 130 causes the manipulator arm end 2320 to move a distance toward the center of the body of the patient according to the prompt message corresponding to serial number 2 in the table, so that the manipulator arm end 2320 no longer collides with the CT machine 2330 at the adjusted needle insertion point 2310.

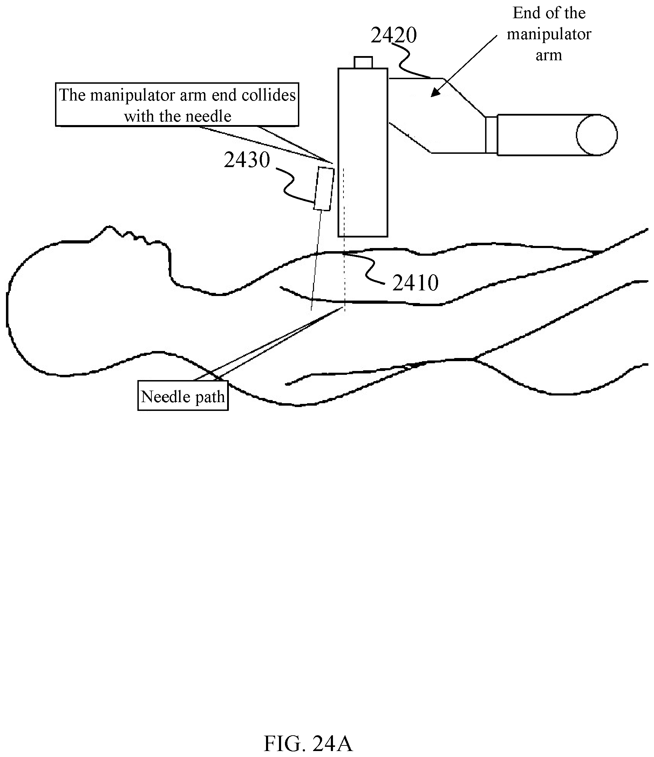

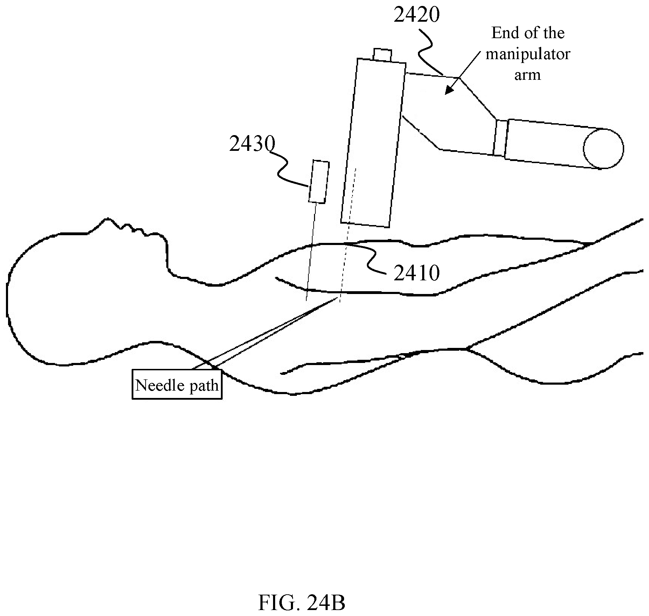

In some embodiments, in response to a path planning failure caused by a collision between the manipulator arm end model and the surgical instrument model, a third prompt message is output. The third prompt message at least instructs a user to adjust one or more operation positions in the surgical planning information along a long axis of the scanning table. Merely by way of example, FIG. 24A is a schematic diagram illustrating an exemplary path planning failure caused by a collision between the manipulator arm end model and the surgical instrument model according to some embodiments of the present disclosure. As shown in FIG. 24A, when the planned movement path of the end effector is verified by the collision model, it is detected that a collision occurs between the manipulator arm end 2420 (i.e., the end effector) of the manipulator arm end model and the puncture needle 2430 (i.e., the surgical instrument model) at a needle insertion point 2410, and the verification fails. At this time, the processing device 130 displays the third prompt message to the user. For example, the prompt message corresponding to serial number 3 in Table 1 described later including a cause of the failure and adjustment suggestions. In some embodiments, the processing device 130 adjusts a needle insertion point (i.e., an operation position) where the verification fails and/or a corresponding needle path (i.e., a working channel), or the like, according to the suggestions in the third prompt message. For example, because a needle path (i.e., the needle path X in the prompt message in Table 1 described later) corresponding to a subsequent operation position (the needle insertion point 2410) and a needle path (i.e., the needle path A in the prompt message in Table 1 described later, wherein an operation sequence of the needle path A is prior to that of needle path X) of the puncture needle 2430 are in different scanning layers, therefore, according to suggestion ② of the prompt message corresponding to serial number 3 in Table 1, a spacing between the operation positions (needle insertion points) of the two needle paths is increased. In some embodiments, the processing device 130 can change the relative position between the manipulator arm end and a surgical instrument on the surface of the patient by adjusting at least one of the manipulator arm and the scanning table, so as to adjust the operation position and/or the corresponding working channel at least along the long axis of the scanning table. For example, FIG. 24B is a schematic diagram of an exemplary adjustment of a needle path after the path planning failure in FIG. 24A according to some embodiments of the present disclosure. As shown in FIG. 24B, the processing device 130, according to the prompt message corresponding to serial number 3 in the table, adjusts a needle path (i.e., needle path X in the prompt message in Table 1 described later) corresponding to the needle insertion point 2410 along the long axis of the scanning table by movement of the manipulator arm, that is, by increasing the left and right inclination angle of the needle path such that the inclination angle is greater than the left and right inclination angle of the needle path of the puncture needle 2430, so that the manipulator arm end 2420 no longer collides with the puncture needle 2430 at the adjusted needle insertion point 2410.

In some embodiments, the causes of the planning failure further include others, for example, at least one of an interference between a working channel and important tissue, an error in drawing the working channel, and the length of the working channel not satisfying corresponding instrument requirements, or the like. The processing device 130 adjusts accordingly based on the prompt messages corresponding to these causes.

The output manner of the prompt message may include a plurality of manners, for example, text, sound, image, video, animation, or the like, or any combination thereof. In some embodiments, the processing device 130 displays text prompt messages to the user via a display device (for example, a display component of the terminal 140). For example, as shown in Table 1:

**Table 1**

| Serial Number | Path Planning Failure Scenario | Prompt Message |
|---|---|---|
| 1 | Collision between Manipulator Arm End Model and Patient Model | Needle Path X Simulation Failure Reason: |
| | | ① The manipulator arm end model may collide with the patient model at a target point; |
| | | ② The planning fails because the manipulator arm end model may collide with the patient model during a path planning computation process; |
| | | It is suggested to adjust the needle path. The following are possibly effective manners for adjusting the needle path: |
| | | ① Increasing the distance between the manipulator arm end model and a surface of the patient model; |
| | | ② Observing the position of the needle path in a three-dimensional space from a plurality of angles, adjusting the needle insertion point, and drawing an inclined needle path; |
| 2 | Collision between Manipulator Arm End Model and Scanning Device Model | Needle Path X Simulation Failure |
| | | Reason: |
| | | ① The manipulator arm end model may collide with the scanning device model at a target point; |
| | | ② The planning fails because the manipulator arm end model may collide with the scanning device model during a path planning computation process; |
| | | It is suggested to adjust the needle path. The following are possibly effective manners for adjusting the needle path: |
| | | ① Adjusting a needle insertion point of the needle path as much as possible to a center position of an image; |
| | | ② Exiting from the current patient, lower a bed height, and re-scan; |
| 3 | Collision between Manipulator Arm End Model and Surgical Instrument Model | Needle Path X Simulation Failure |
| | | Reason: |
| | | ① The manipulator arm end model may collide with puncture needle A at a target point; |
| | | ② The planning fails because the manipulator arm end model may collide with puncture needle A during a path planning computation process; |
| | | It is suggested to adjust the needle path. The following are possibly effective manners for adjusting the needle path: |
| | | ① If the needle insertion points of the needle path X and the needle path A are in the same scanning layer, then trying to increase a spacing between the needle insertion points of the needle path X and the needle path A; |
| | | ② If the needle insertion points of the needle path X and the needle path A are in different scanning layers, then increasing the left and right inclination angle of the needle path X to ensure that the left and right inclination angle of the needle path X is greater than the left and right inclination angle of the needle path A; if the planning still fails to pass, then trying to increase an interlayer spacing between the needle insertion points of the needle path X and the needle path A; |

The number 1 corresponds to a collision between the manipulator arm end model and the patient model, the number 2 corresponds to a collision between the manipulator arm end model and the scanning device model, and the number 3 corresponds to a collision between the manipulator arm end model and the surgical instrument model. The operation order of the needle path A is before the needle path X.

In some embodiments of the present disclosure, by outputting a prompt message after a failure in planning the movement path, it facilitates a user to find a cause of the failure and more effectively select an adjustment strategy.

Step S2040, re-obtaining the second position information of the scanning table and/or the surgical planning information. In some embodiments, Step S2040 is performed by the planning acquisition module 410.

After adjusting the operation positions and the corresponding working channels in the planned movement path, the processing device 130 reacquires the second position information of the scanning table and/or the surgical planning information, wherein the adjustment is performed based on at least one of the prompt message, user experience, or the like. The re-obtained surgical planning information includes at least one adjusted operation position and an adjusted working channel corresponding to the adjusted operation position. The acquisition manner is the same as or similar to Step S2010.

Step S2050, re-planning the movement path of the end effector based on the re-obtained second position information and/or the re-obtained surgical planning information. In some embodiments, Step S2050 is performed by the path planning module 420.

The processing device 130 re-plans the movement path of the end effector based on the re-obtained second position information and/or the re-obtained surgical planning information. The manner of planning the path is the same as or similar to Step S2020.

After the movement path of the end effector is successfully replanned, the processing device 130 performs Step S2060 and Step S2070 to perform a surgical procedure based on the successfully planned movement path. When the replanning fails again, the processing device 130 returns to perform Step S2030 to Step S2050.

Step S2060, in response to successful planning of the movement path of the end effector, determining target surgical planning information, and a second sorting result of a plurality of target operation positions in the target surgical planning information. In some embodiments, Step S2060 is performed by the path execution module 430. Step S2060 refers to the relevant content in Step S550.

Step S2070, sequentially planning and executing an execution subpath between each set of adjacent target operation positions in the second sorting result. In some embodiments, Step S2070 is performed by the path execution module 430.

In a preset environment, the processing device 130 sequentially plans and executes an execution subpath between each set of adjacent target operation positions in the second sorting result based on the acquired first position information, the second position information, and the target surgical planning information. The preset environment includes at least one of a practical surgical stage, a surgical simulation stage, or the like. Specifically, the processing device 130 controls the end effector of the manipulator arm to move along the execution subpath by instructions, or the like. In some embodiments, Steps S2010 to S2060 are performed in a pre-operative stage of a surgical procedure, and Step S2070 is performed in an intra-operative stage of the surgical procedure.

In some embodiments, path planning and path execution are performed by the same or different processors. For example, path planning operations in Steps S2010 to S2070 are performed by a first processor (for example, a processor at a master control end, or the like), and path execution operations in Step S2070 are performed by a second processor different from the first processor (for example, a processor at a slave end, or the like), wherein the first processor and the second processor are included in the processing device 130. For example, path planning and path execution operations in Steps S2010 to S2070 are performed by a processor of the processing device 130.

In the online mode, in order to monitor whether the operations are accurate during a surgical procedure, operations related to the operation positions and the working channels are observed by real-time imaging of the scanning device. For example, in an interventional puncture surgical procedure in the online mode, when the manipulator arm holds the puncture needle and moves to an interventional puncture point, a physician monitors the entire needle insertion process by real-time exposure of a CT machine. In some embodiments, at least a part of each execution subpath is executed within a scanning chamber of the scanning device, which enables the scanning device to perform real-time imaging on a patient and to monitor the surgical procedure, so as to ensure smooth progress of the surgical procedure.

In some embodiments, the processing device 130 tilts a gantry of the scanning device, such that the gantry is tilted towards a craniocaudal direction (i.e., the second direction), to position the target working channel within a scanning range of the scanning device, so as to perform complete and accurate real-time monitoring of the surgical procedure by the scanning device.

In some embodiments, if a target working channel corresponding to a target operation position included in an execution subpath to be executed is inclined relative to the second direction, the processing device 130 controls the scanning device, the scanning table, and the manipulator arm to operate in coordination to execute the execution subpath.

In some embodiments, the foremost target operation position in the second sorting result is referred to as a first target operation position, and the processing device 130 performs the following steps to tilt a gantry of the scanning device and perform a surgical procedure: when the target working channel corresponding to the first target operation position is inclined relative to the second direction, the processing device 130 controls the scanning device to rotate toward the second direction such that a scanning chamber of the scanning device is inclined at a first angle relative to the second direction, wherein the first angle corresponds to an inclination angle of the target working channel corresponding to the first target operation position relative to the second direction, and the second direction is parallel to a long axis of the scanning table. The processing device 130 controls the scanning table to move along the second direction and a third direction, from its initial position at the start of a surgical procedure, to enter the scanning chamber of the scanning device, such that the target working channel corresponding to the first target operation position is within a scanning range of the scanning device, wherein the third direction is a direction perpendicular to the scanning table, and the initial position of the scanning table at the start of the surgical procedure is a position that prevents the scanning table from colliding and interfering with other objects. The processing device 130 plans and controls the manipulator arm and the end effector to execute the execution subpath from the safe position to the first target operation position, wherein the safe position is an initial position of the manipulator arm and the end effector at the start of a surgical procedure, and the initial position is set such that the manipulator arm and the end effector do not collide with the scanning device and the scanning table during the tilt adjustment. In some embodiments, the first angle is equal to or approximately equal to an inclination angle of the target working channel corresponding to the first target operation position relative to the second direction. In some embodiments, when controlling the scanning table to move into the scanning chamber, it may only include movement along the second direction.

In some embodiments, the processing device 130 performs the following steps to tilt a gantry of the scanning device and perform a surgical procedure: in response to an inclination of a subsequent target working channel corresponding to a subsequent target working position of the adjacent target operation positions relative to the second direction, the processing device 130 controls the manipulator arm and the end effector to exit the scanning chamber of the scanning device from a preceding target working position of the adjacent target operation positions to the safe position of the end effector, wherein the second direction is parallel to a long axis of the scanning table, and the safe position is a second transition point, which refers to a transition position for controlling the end effector to move from close to an operation position to away from the operation position. The processing device 130 controls the scanning table to exit the scanning chamber of the scanning device, then controls the scanning device to rotate toward the second direction such that the scanning chamber of the scanning device is inclined at a second angle relative to the second direction, wherein the second angle corresponds to an inclination angle of the subsequent target working channel relative to the second direction. The processing device 130 controls the scanning table to move along the second direction and the third direction to enter the scanning chamber of the scanning device, such that the subsequent target working channel is within a scanning range of the scanning device, wherein the third direction may be a direction perpendicular to the scanning table. The processing device 130 plans and controls the manipulator arm and the end effector to execute the execution subpath from the safe position to the subsequent target working position. The preceding target working position is the target operation position ranked earlier among the adjacent target operation positions, and the subsequent target working position is the target operation position ranked later among the adjacent target operation positions. In some embodiments, the second angle is equal to or approximately equal to an inclination angle of the subsequent target working channel relative to the second direction. In some embodiments, when controlling the scanning table to move into the scanning chamber, it only includes movement along the second direction. For example, if the preceding target working position is the first target operation position, the subsequent target working channel is inclined relative to the second direction, and the preceding target working channel and the subsequent target working channel are located in different scanning layers, then, after tilting the gantry of the scanning device and controlling the manipulator arm and the end effector to execute the execution subpath from the safe position to the preceding target working position, the processing device 130 adjusts the position of the gantry and/or the scanning table in the manner described above, to position the subsequent target working channel within the scanning range of the scanning device, and then plans and controls the manipulator arm and the end effector to execute the execution subpath from the preceding target working position to the subsequent target working position. As another example, if the preceding target working position is not the first target operation position, and the subsequent target working channel is inclined relative to the second direction, then the processing device 130 adjusts the position of the gantry and/or the scanning table in the manner described above, to position the subsequent target working channel within the scanning range of the scanning device, and then plans and controls the manipulator arm and the end effector to execute the execution subpath from the preceding target working position to the subsequent target working position.

In some embodiments, if the subsequent target working channel corresponding to the subsequent target working position among the adjacent target operation positions is inclined relative to the second direction, and the preceding target working channel and the subsequent target working channel are located in different scanning layers, the processing device 130 performs the following steps to tilt a gantry of the scanning device and perform a surgical procedure: the processing device 130 controls the scanning device to rotate toward the second direction such that the scanning chamber of the scanning device is inclined at a second angle relative to the second direction. The processing device 130 controls the scanning table to move along the second direction and the third direction such that the subsequent target working channel is within the scanning range of the scanning device. The processing device 130 plans and controls the manipulator arm and the end effector to execute the execution subpath from the preceding target working position to the subsequent target working position. For example, if the preceding target working position is the first target operation position, then, after tilting the gantry of the scanning device and controlling the manipulator arm and the end effector to execute the execution subpath from the safe position to the preceding target working position, if the preceding target working channel and the subsequent target working channel are located in different scanning layers, the processing device 130 adjusts the position of the gantry and/or the scanning table in the manner described above, to position the subsequent target working channel within the scanning range of the scanning device, and then plans and controls the manipulator arm and the end effector to execute the execution subpath from the preceding target working position to the subsequent target working position.

In some embodiments, if the subsequent target working channel corresponding to the subsequent target working position among the adjacent target operation positions is inclined relative to the second direction, and the preceding target working channel corresponding to the preceding target working position among the adjacent target operation positions and the subsequent target working channel are located in a same scanning layer, the processing device 130 plans and controls the manipulator arm and the end effector to execute the execution subpath from the preceding target working position to the subsequent target working position. For example, if the preceding target working position is the first target operation position, then, after tilting the gantry of the scanning device and controlling the manipulator arm and the end effector to execute the execution subpath from the safe position to the preceding target working position, an inclination angle of the preceding target working channel corresponding to the preceding target working position relative to the second direction is the first angle. If the first angle and the second angle are equal to or not equal to each other, and the preceding target working channel and the subsequent target working channel are located in a same scanning layer, the processing device 130 does not adjust the position of the gantry and the scanning table, and directly plans and controls the manipulator arm and the end effector to execute the execution subpath from the preceding target working position to the subsequent target working position.

In some embodiments, if the subsequent target working channel corresponding to the subsequent target working position among the adjacent target operation positions is inclined relative to the second direction, and the preceding target working channel corresponding to the preceding target working position among the adjacent target operation positions and the subsequent target working channel are located in the same scanning layer, and an inclination angle of the preceding target working channel relative to the second direction is different from an inclination angle of the subsequent target working channel relative to the second direction, the processing device 130 controls the scanning device to rotate toward the second direction, such that the scanning chamber of the scanning device is inclined at the second angle relative to the second direction, and the subsequent target working channel is within the scanning range of the scanning device. The processing device 130 plans and controls the manipulator arm and the end effector to execute the execution subpath from the preceding target operation position to the subsequent target operation position. In some embodiments, before or after controlling the scanning device to rotate toward the second direction, the processing device 130 further controls the scanning table to move along the second direction and a third direction. For example, if the preceding target operation position is a first target operation position, after tilting the gantry of the scanning device and controlling the manipulator arm and the end effector to execute the execution subpath from the safe position to the preceding target operation position, the inclination angle of the preceding target working channel corresponding to the preceding target operation position relative to the second direction is the first angle, if the first angle and the second angle are not equal, and the preceding target working channel and the subsequent target working channel are located in the same scanning layer, the processing device 130 adjusts the position of the gantry and/or the scanning table in the foregoing manner, so that the subsequent target working channel is within the scanning range of the scanning device, and then plans and controls the manipulator arm and the end effector to execute the execution subpath from the preceding target operation position to the subsequent target operation position. In some embodiments, when the processing device 130 controls the scanning table to move, the movement only includes movement along the second direction.

In some embodiments, if the gantry of the scanning device has already been tilted at the preceding target working position among the adjacent target operation positions, the processing device 130 controls the scanning device to perform a gantry homing operation, and then controls the scanning device to rotate, such that the scanning chamber of the scanning device is inclined at the second angle relative to the second direction. In some embodiments, the gantry homing operation includes controlling the scanning device to rotate toward the second direction, such that the scanning chamber of the scanning device is parallel to the second direction. For example, if the preceding target operation position is a first target operation position, after tilting the gantry of the scanning device and controlling the manipulator arm and the end effector to execute the execution subpath from the safe position to the preceding target operation position, the processing device 130 controls the scanning device to rotate a first angle toward the opposite direction of the second direction, such that the scanning chamber of the scanning device is parallel to the second direction, which makes the gantry of the scanning device return to the initial position at the start of the surgical procedure.

In some embodiments, after controlling the scanning device to rotate and/or controlling the scanning table to move, the processing device 130 updates the collision model and position information of the subsequent target working position among the adjacent target operation positions, and plans the execution subpath according to the updated collision model and the updated position information of the subsequent target working position. For details on how to plan the execution subpath according to the updated collision model and the updated position information of the subsequent target working position, refer to the relevant description in step S2020, and will not be repeated herein.

For more details on how to tilt the gantry of the scanning device and perform a surgical procedure through the foregoing steps, refer to the relevant description in steps S2130-S2160, and will not be repeated herein.

In some embodiments of the present disclosure, the accuracy of path planning is improved and ensured by pre-operative planning and simulation verification of the movement path of the end effector of the interventional surgical robot (for example, a puncture needle, or the like). By tilting the gantry of the scanning device during the surgical procedure, complete and accurate real-time monitoring of the surgical procedure is ensured, and the surgical operation difficulty is reduced. The foregoing manner saves the surgical time of a user, reduces the system usage difficulty, and improves the user experience.

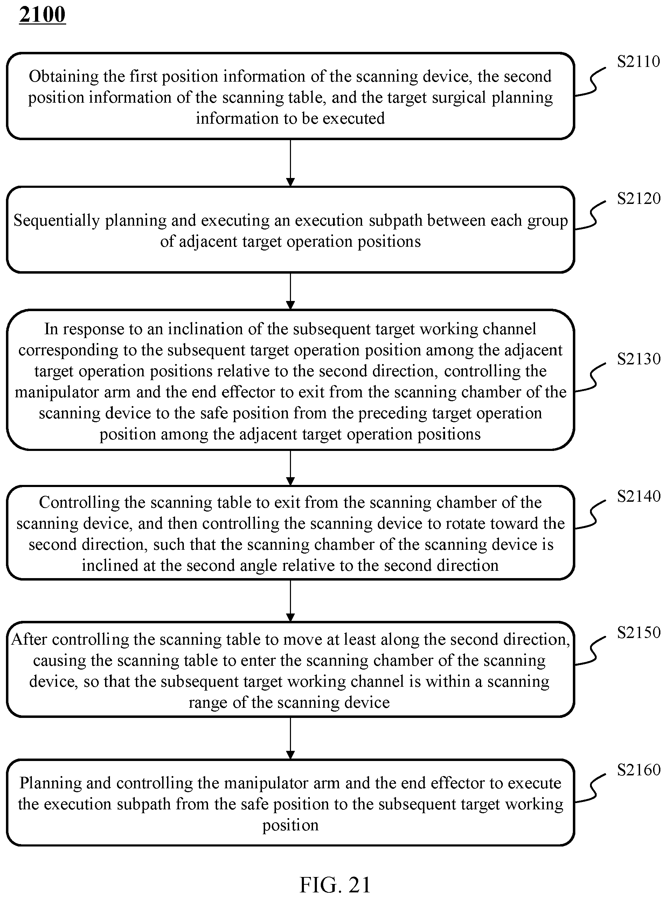

FIG. 21 is a flowchart illustrating an exemplary process for path planning for an interventional surgical robot in a system for interventional medicine according to some embodiments of the present disclosure. As shown in FIG. 21, the process 2100 includes the following steps. In some embodiments, the processing device 130 or the modules of the system 400 implement at least a part of process 500 and at least a part of process 2000 by executing at least a part of the process 2100, to plan the movement path of the end effector in the interventional mode based on the surgical planning information. The manner shown in the process 2100 is applicable to the online mode.

Step S2110, obtaining the first position information of the scanning device, the second position information of the scanning table, and the target surgical planning information to be executed. The target surgical planning information includes a plurality of target operation positions and a plurality of target working channels corresponding to the plurality of target operation positions.

The operation of step S2110 is similar to that of step S2010, wherein the target surgical planning information to be executed may be equivalent to the surgical planning information in step S2010. The difference lies in that the surgical planning corresponding to the target surgical planning information to be executed includes the movement path of the end effector that can be directly executed, while the surgical planning information in step S2010 needs path planning and verification. For more details on the operation of step S2110, refer to step S2010.

Step S2120, sequentially planning and executing an execution subpath between each group of adjacent target operation positions. wherein at least a portion of each execution subpath is executed within a scanning chamber of the scanning device. For details on the operation of step S2120, refer to step S2070.

Step S2130, in response to an inclination of the subsequent target working channel corresponding to the subsequent target operation position among the adjacent target operation positions relative to the second direction, controlling the manipulator arm and the end effector to exit from the scanning chamber of the scanning device to the safe position from the preceding target operation position among the adjacent target operation positions. The second direction is parallel to the long axis of the scanning table. The safe position is a second transition point, which refers to a transition position for controlling the end effector to move from close to the operation position to away from the operation position.

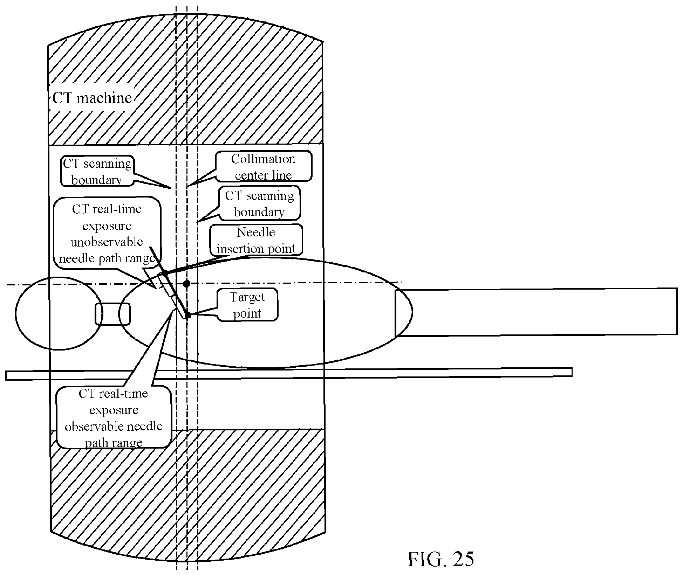

When performing surgical procedures such as interventional puncture in the chest or the abdomen, for a needle path inclined in the craniocaudal direction, it is not possible to see a puncture needle insertion trajectory (i.e., a complete needle path) of the puncture needle under real-time exposure, and the surgical difficulty is high. For example, FIG. 25 is a schematic diagram illustrating an exemplary needle path inclined in a craniocaudal direction without tilting a CT gantry according to some embodiments of the present disclosure. As shown in FIG. 25, the CT gantry is not tilted, and the needle path in FIG. 25 is a needle path inclined in the craniocaudal direction. When the CT scanner performs real-time exposure, a complete needle path cannot be seen because the exposure direction is not parallel to the layer where the needle path is located.

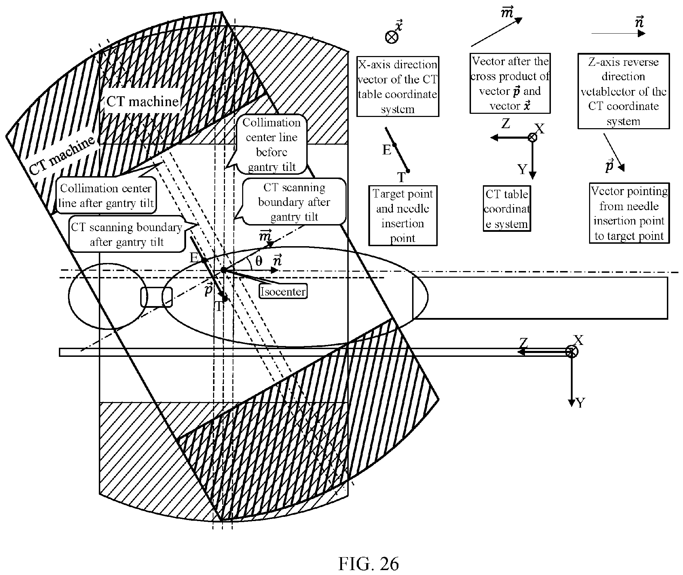

The processing device 130 tilts the scanning device by tilting the gantry of the scanning device (for example, a CT machine, an MRI device, or the like), so that the puncture needle insertion trajectory of the puncture needle can be seen under real-time exposure, thereby reducing the surgical difficulty to a certain extent. For example, as shown in FIGS. 26-28, based on the CT gantry shown in FIG. 25, the CT gantry is tilted toward the craniocaudal direction (i.e., the second direction), such that the exposure direction of the CT scanner is parallel to the layer where the needle path is located, so that a complete needle path inclined in the craniocaudal direction can be seen.

In some embodiments, tilting the scanning device may also be applicable to other surgical procedures, for example, vascular surgery, or the like.

During the process of tilting the scanning device, the manipulator arm and the end effector first need to be moved out from the scanning chamber of the scanning device. In response to an inclination of the subsequent target working channel corresponding to the subsequent target operation position among the adjacent target operation positions relative to the second direction, the processing device 130 controls the manipulator arm and the end effector to exit the scanning chamber of the scanning device from the preceding target operation position among the adjacent target operation positions to the safe position, wherein the second direction is parallel to the long axis of the scanning table. Taking an interventional multi-needle puncture surgical procedure as an example, the preceding target working position may correspond to a current needle insertion point, and the subsequent target working position may correspond to a next needle insertion point to be executed. The inclination of the target working channel corresponding to the subsequent target operation position among the adjacent target operation positions toward the second direction indicates that the target working channel is a needle path inclined in the craniocaudal direction, that is, the next needle insertion point corresponds to a needle path inclined in the craniocaudal direction. The processing device 130 controls the manipulator arm and the puncture needle to exit the scanning chamber of the CT scanner from the current needle insertion point to the safe position.

The processing device 130 tilts the scanning device by executing step S2140 and moves the scanning table by executing step S2150, so that the target operation position is within the scanning range of the scanning device. Taking a CT machine as an example, the processing device 130 rotates the CT gantry to tilt it by executing step S2140, and moves the scanning table by executing step S2150, so that a target point and a needle insertion point are located within the scanning range enclosed by the CT scanning boundary. In some embodiments, the target point and the needle insertion point are located on the collimation centerline of the CT machine.

Step S2140, controlling the scanning table to exit from the scanning chamber of the scanning device, and then controlling the scanning device to rotate toward the second direction, such that the scanning chamber of the scanning device is inclined at the second angle relative to the second direction. wherein the second angle corresponds to an inclination angle of the subsequent target working channel relative to the second direction.

After controlling the manipulator arm and the end effector to exit to the safe position, the processing device 130 controls the scanning table to exit from the scanning chamber of the scanning device, so that the tilting of the scanning device has sufficient space.

After the manipulator arm, the end effector, and the scanning table have all exited from the scanning chamber of the scanning device, the processing device 130 controls the scanning device to tilt, that is, controls the scanning device to rotate toward the second direction (i.e., the craniocaudal direction) to tilt the scanning chamber of the scanning device relative to the second direction.

The rotation angle of the scanning device rotating toward the second direction (for example, the first angle and the second angle) is equal to or similar to the inclination angle of the target working channel toward the second direction. The processing device 130 determines the rotation angle in the following manner: determining a first vector of the target working channel based on the target surgical planning information, and determining the rotation angle based on the first vector. The first vector is a vector representing a spatial direction of the target working channel. For example, in a puncture surgical procedure, the first vector may be a vector pointing from a needle insertion point to a target point. In some embodiments, the tilting of the scanning device is equivalent to gantry tilting. Therefore, the gantry tilting angle of the scanning device is taken as the rotation angle of the scanning device rotating toward the second direction.

FIG. 26 is a schematic diagram illustrating an exemplary calculation of a CT gantry inclination angle according to some embodiments of the present disclosure. As shown in FIG. 26, the scanning device is a CT gantry, and the scanning table is a CT table. During a pre-operative scanning stage, when a puncture point is localized in the scanning chamber of the CT scanner, by moving the scanning table, the plane where the target point is located is positioned at the collimation centerline. At this time, the needle insertion point is recorded as *E*(*xₑ, yₑ, zₑ*) and the target point is recorded as *T*(*xₜ, yₜ, zₜ*), which are the needle insertion point and target point values in the target surgical planning information. The needle insertion point and the target point are all represented under a CT table coordinate system (also referred to as a patient coordinate system). In some embodiments, a vector *̅p̅*̅ pointing from the needle insertion point E to the target point T is shown in Formula (5) below: $\vec{p} = \left\{{x}_{t}-{x}_{e}; {y}_{t}-{y}_{e}; {z}_{t}-{z}_{e}\right\}$ (5).

When the X-axis direction vector of the CT table coordinate system is denoted as *̅x̅*̅ = [1; 0; 0], then the vector after the cross product of vectors *̅p̅*̅ and *̅x̅*̅ is shown in the following Formula (6): $\vec{m} = \vec{p} \times \vec{x} = \left\{0; {z}_{t}-{z}_{e}; {y}_{e}-{y}_{t}\right\}$ (6),

If the Z-axis reverse direction vector of the CT table coordinate system is denoted as *n̅ =* [0; 0; 1], then the angle *θ* between vectors *̅m̅*̅ and *̅n̅*̅ is the inclination angle of the needle path in the craniocaudal direction (which is equal to the CT gantry inclination angle), and the angle *θ* is shown in the following Formula (7): $θ = arc cos \left(\frac{\vec{m} ⋅ \vec{n}}{\left|\vec{m}\right| \left|\vec{n}\right|}\right) = \frac{{y}_{t} - {y}_{e}}{\sqrt{{\left({z}_{t}-{z}_{e}\right)}^{2} + {\left({y}_{e}-{y}_{t}\right)}^{2}}}$ (7),

Step S2150, after controlling the scanning table to move at least along the second direction, causing the scanning table to enter the scanning chamber of the scanning device, so that the subsequent target working channel is within a scanning range of the scanning device.

After the scanning device completes the craniocaudal tilting, the processing device 130 controls the scanning table to move along the second direction and a third direction, so that the scanning table enters the scanning chamber of the scanning device and the subsequent target working channel is within a scanning range of the scanning device. The third direction is a direction perpendicular to the scanning table, namely, perpendicular to the plane where the first direction and the second direction are located. Therefore, the second direction is the horizontal direction, and the third direction is the vertical direction. Controlling the scanning table to move along the second direction and the third direction means controlling the scanning table to perform horizontal table movement and vertical table movement, thereby re-entering the scanning chamber of the scanning device. In some embodiments, when there is no possibility of interference between the scanning table and the scanning device after the scanning device is tilted, the processing device 130 controls the scanning table to only perform movement along the second direction, namely, only perform horizontal table movement.

After the scanning device is tilted, an inclination angle of the gantry of the scanning device corresponds to a minimum scanning table height. The processing device 130 avoids interference between the scanning table and the scanning device by moving the scanning table up and down, and makes the target scanning position fall within the scanning range enclosed by the scanning boundary of the scanning device by moving the scanning table left and right.

The table movement amount of the scanning table is determined in the following manner: the processing device 130 obtains the minimum scanning table height of the scanning device at the first angle, and determines the vertical table movement amount of the scanning table based on the minimum scanning table height. After the vertical table movement, the processing device 130 determines the relative position of the target point relative to the isocenter of the scanning device, and determines the horizontal table movement direction and the horizontal table movement amount of the scanning table based on the relative position.

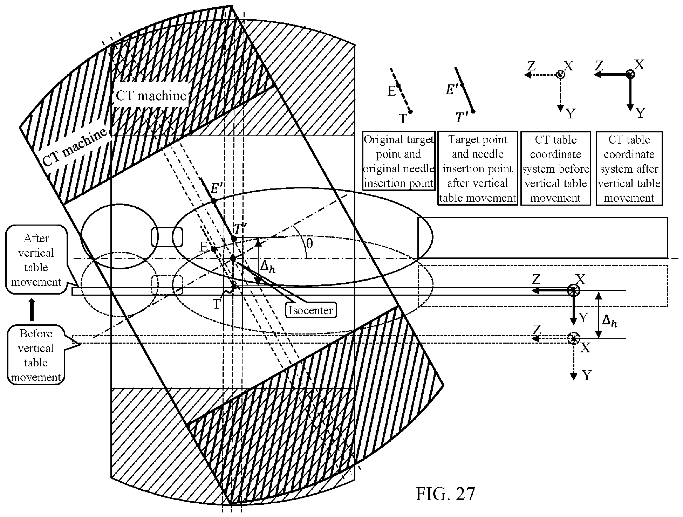

FIG. 27 is a schematic diagram illustrating an exemplary calculation of a vertical table movement amount of a CT table, according to some embodiments of the present disclosure. As shown in FIG. 27, the scanning device is a CT gantry, and the scanning table is a CT table. FIG. 27 illustrates the vertical table movement of the CT table based on the CT gantry completing tilting as shown in FIG. 26. For a specific model of CT, each inclination angle corresponds to a minimum scanning table height, and the minimum scanning table height can be obtained by querying parameters of the CT. As shown in FIG. 27, let the gantry inclination angle be *θ̂* (which is equal to the inclination angle of the needle path *θ* in the craniocaudal direction in FIG. 26), and the minimum scanning table height of the CT in FIG. 27 corresponding to the inclination angle be *hₘᵢₙ*.

Let the table height in an untilted state of the gantry be *h*₀, then the vertical table movement amount Δ*ₕ* is shown in the following formula: ${Δ}_{h} = \left\{\begin{matrix}0 , \hat{θ} = 0 \\ {h}_{min} \left(\hat{θ}\right) - {h}_{0} , \hat{θ} > 0\end{matrix}\right.$ (8), then the table height *ĥ* after the gantry is tilted needs to be set to *ĥ* = *h*₀ + Δ*ₕ*.

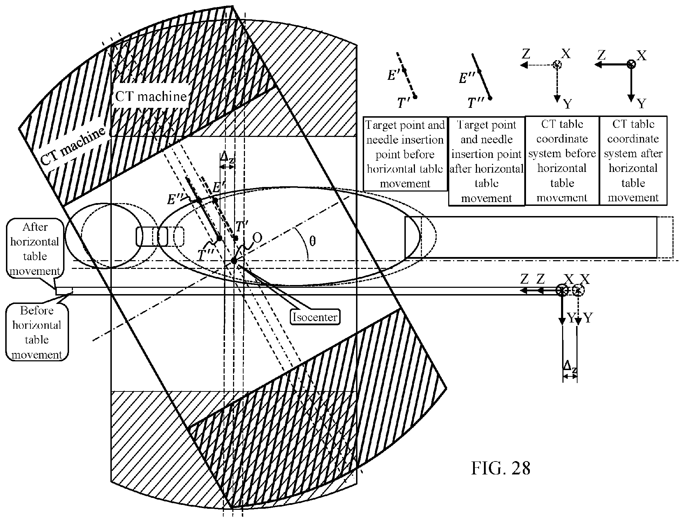

FIG. 28 is a schematic diagram illustrating an exemplary calculation of a horizontal table movement amount of a CT table according to some embodiments of the present disclosure. FIG. 28 illustrates the horizontal table movement of the CT table based on the CT table completing the vertical table movement as shown in FIG. 27. As shown in FIG. 27, after the vertical table movement, the CT table coordinate system moves upward by Δ*ₕ*. Let the insertion point after moving upward be *E*'(*xₑ, yₑ*, *zₑ*) and the target point be *T*'(*xₜ*, *yₜ*, *zₜ*). Then, in FIG. 28, the insertion point after horizontal table movement is *E"*, and the target point is *T"*. The coordinate of the isocenter O in the target surgical planning information in the CT table coordinate system during preoperative scanning is *O*(*xₒ*, *yₒ*, *zₒ*). Then the coordinate of the isocenter O in the CT table coordinate system after vertical table movement is *O*(*xₒ, yₒ +* Δ*ₕ, zₒ*). The isocenter is the rotation center of the CT gantry, and it will not change during gantry tilting and scanning table movement.

When the Y coordinate *yₜ* of the target point *T'* after vertical table movement is greater than the Y coordinate (*yₒ +* Δ*ₕ*) of the isocenter O in the CT table coordinate system after vertical table movement, the scanning table moves horizontally toward the scanning chamber; otherwise, the scanning table moves horizontally out of the scanning chamber. Let the horizontal table movement direction be *horDir*, then the horizontal table movement direction *horDir* is shown in the following Formula (9): $horDir = \left\{\begin{matrix}- 1 , {y}_{t} < \left({y}_{o}+{Δ}_{h}\right) \\ 1 , {y}_{t} \geq \left({y}_{o}+{Δ}_{h}\right)\end{matrix}\right.$ (9),

The horizontal table movement amount Δ*_{z}* is shown in the following Formula (10):${Δ}_{z} = horDir ∗ OT ' ∗ tan θ = horDir ∗ abs \left({y}_{t}-\left({y}_{o}+{Δ}_{h}\right)\right) ∗ tan θ$ (10) where *OT'* represents the distance between the isocenter O and the target point *T'* ; *abs* represents the absolute value operation; *θ* has the same meaning as in Formula (7), representing the inclination angle of the needle path in the craniocaudal direction, which is equal to the gantry inclination angle *θ̂*.

As shown in FIG. 28, the Y coordinate *yₜ* of the target point *T'* after vertical table movement is greater than the Y coordinate (*yₒ +* Δ*ₕ*) of the isocenter O in the CT table coordinate system after vertical table movement. Therefore, the scanning table moves horizontally toward the scanning chamber. At this time, *horDir*=1. Substituting *horDir*=1 into Formula (10) obtains the horizontal table movement amount Δ*_{z}*.

Step S2160, planning and controlling the manipulator arm and the end effector to execute the execution subpath from the safe position to the subsequent target working position.

After the scanning table moves in the vertical and horizontal directions, a position where the manipulator arm arrives also needs to be changed. After step 2150, the processing device 130 plans and controls the manipulator arm and the end effector to execute the execution subpath from the safe position to the subsequent target working position. Specifically, the processing device 130 repositions the manipulator arm and plans its arrival position so that the manipulator arm can continue to execute the execution subpath from the safe position to the subsequent target working position (namely, the next operation position).

Since the relative position between the scanning table and the scanning device changes after gantry tilting, the position information of the needle insertion point and the target point also changes. The position information of the needle insertion point and the target point may be represented by coordinates in the manipulator arm coordinate system. The processing device 130 determines the position information of the needle insertion point and the target point after gantry tilting in the following manner: determining a first transformation matrix based on the horizontal table movement amount and the vertical table movement amount of the scanning table; obtaining the transformation relationship of the coordinate systems at the needle insertion point and the target point in the manipulator arm coordinate system; and determining the transformation relationship of the coordinate systems at the needle insertion point and the target point in the manipulator arm coordinate system based on the transformation relationship and the first transformation matrix.

In some embodiments, the positive Y-coordinate axis direction of the CT table is vertically downward, and the positive Z-axis direction is from the foot side of the CT table to the head side of the CT table. After gantry tilting, the CT table coordinate system, relative to preoperative scanning, moves along the Y-coordinate axis by -Δ*ₕ*, namely, moves upward by Δ*ₕ* in the vertical direction, and moves along the Z-coordinate axis by -Δ*_{z}*, namely, moves by Δ*_{z}* from the head side of the CT table to the foot side of the CT table. Then the transformation relationship (the transformation matrix ${Transl}_{cou _ p}^{couch _ p ′}$) of the CT table coordinate system before and after movement is shown in the following Formula (11): ${Transl}_{couch _ p}^{couch _ p ′} = \left[\begin{matrix}0 \\ - {Δ}_{z} \\ {Δ}_{h} \\ 1\end{matrix}\right]$ (11),

Let the transformation relationships of the coordinate systems at the needle insertion point and the target point in the manipulator arm coordinate system during preoperative scanning be ${M}_{robot}^{E}$ and ${M}_{robot}^{T}$. After gantry tilting, the positions of the needle insertion point and the target point in the manipulator arm coordinate system need to consider the above transformation relationships. The needle insertion point and the target point converted to the manipulator arm coordinate system are shown in the following Formula (12) and Formula (13): ${E}_{robot}^{′} = {M}_{robot}^{E} ∗ {Transl}_{couc _ p}^{couch _ p ′}$ (12), ${T}_{robot}^{′} = {M}_{robot}^{T} ∗ {Transl}_{couc _ p}^{co _ p ′}$ (13), where ${E}_{robot}^{′}$ represents the coordinates of the needle insertion point in the manipulator arm coordinate system after gantry tilting; ${T}_{robot}^{′}$ represents the coordinates of the target point in the manipulator arm coordinate system after gantry tilting.

In some embodiments, the processing device 130 determines the collision model based on the first position information and the second position information obtained in step S2110. For specific content regarding how to determine the collision model, refer to the relevant description in step S2020.

After the scanning device is tilted, the scanning device model also needs to be updated. Due to the vertical and horizontal movement of the scanning table, the patient model (including the surgical instrument model that already exists on the patient's body) also needs to be updated in real time. In some embodiments, after step S2150, the processing device 130 updates the collision model and the position information of the subsequent target working position.

The processing device 130 updates the scanning device model in the following manner. The processing device 130 obtains the transformation relationship of the scanning table coordinate system relative to the manipulator arm coordinate system before the scanning device is tilted. The processing device 130 determines a second transformation matrix of the scanning table coordinate system based on a rotation angle of the scanning device towards the second direction. The processing device 130 updates the transformation relationship of the scanning table coordinate system relative to the manipulator arm coordinate system based on the second transformation matrix to update the scanning device model.

Assume that during a pre-operative scan, the transformation relationship of the CT table coordinate system relative to the manipulator arm coordinate system is ${M}_{robot}^{CTCenter}$. Assume that after the gantry is tilted by *θ̃*, the transformation relationship (the transformation matrix ${Transl}_{CT}^{CT _ θ}$) of the CT table coordinate system relative to that during the pre-operative scan is shown in the following formula (14): ${Transl}_{CT}^{CT _ θ} = \left[\begin{matrix}1 & 0 & 0 & 0 \\ 0 & cos \left(-\hat{θ}\right) & - sin \left(-\hat{θ}\right) & 0 \\ 0 & sin \left(-\hat{θ}\right) & cos \left(-\hat{θ}\right) & 0 \\ 0 & 0 & 0 & 1\end{matrix}\right]$ (14),

The pose of the scanning device model (i.e., the CT collision detection model) in the manipulator arm coordinate system after the CT gantry is tilted needs to consider the transformation relationship. The pose is updated to ${M}_{robot}^{CTCenter} ∗ {Transl}_{CT}^{CT _ θ}$.

The processing device 130 updates the patient model in the following manner. The processing device 130 obtains the transformation relationship of the patient coordinate system relative to the manipulator arm coordinate system before the scanning device is tilted. The processing device 130 determines a third transformation matrix of the patient coordinate system based on a movement amount of the scanning table. The processing device 130 updates the transformation relationship of the patient coordinate system relative to the manipulator arm coordinate system based on the third transformation matrix to update the patient model.

Assume that during a pre-operative scan, the transformation relationship of the patient coordinate system relative to the manipulator arm coordinate system is ${M}_{robot}^{patient}$. After the patient moves by Δ*ₕ* in a vertical direction and by Δ*_{z}* in a horizontal direction, the transformation relationship (the transformation matrix ${Transl}_{couc _ p}^{couch _ p ′}$) of the patient model (i.e., the patient collision detection model) relative to that during the pre-operative scan is shown in the following formula (15): ${Transl}_{couc _ p}^{co _ p ′} = \left[\begin{matrix}0 \\ - {Δ}_{z} \\ {Δ}_{h} \\ 1\end{matrix}\right]$ (15),

The pose of the patient collision detection model (i.e., the patient model) in the manipulator arm coordinate system after the CT gantry is tilted needs to consider the transformation relationship. The pose is updated to ${M}_{robot}^{patient} ∗ {Transl}_{couc _ p}^{co _ p ′}$.

The processing device 130 plans the execution subpath based on the updated collision model and the updated position information of the subsequent target working position. Regarding the content of how to plan the execution subpath based on the collision model and the position information of the subsequent target working position, refer to the relevant description in step S2020.

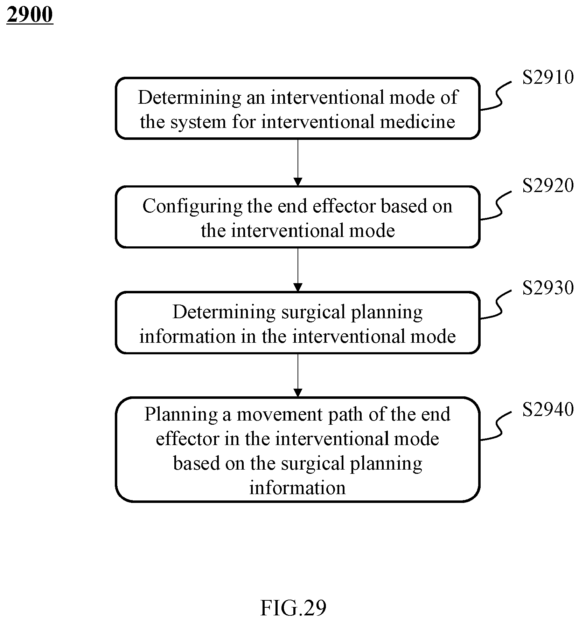

FIG. 29 is a flowchart illustrating an exemplary control method for a system for interventional medicine according to some embodiments of the present disclosure. As shown in FIG. 29, a process 2900 includes the following steps. The method shown in the process 2900 is applicable to a system for interventional medicine (for example, the system 100), wherein the system for interventional medicine includes an interventional surgical robot and a processor, and the interventional surgical robot includes a manipulator arm and an end effector. In some embodiments, the processing device 130 or the modules in the system 400 plan the movement path of the end effector in the interventional mode by executing at least a part of the process 2900.

Step S2910, determining an interventional mode of the system for interventional medicine. In some embodiments, steps S2910-S2930 are executed by the plan acquisition module 410.

The processing device 130 determines the interventional mode based on a selection of a user (for example, a doctor), or the like. For example, the doctor sends instructions through a terminal (for example, the terminal 140) and selects the offline mode or the online mode as the interventional mode for a multi-needle puncture surgery. For more content regarding how to determine the interventional mode, refer to step S510.

Step S2920, configuring the end effector based on the interventional mode.

The processing device 130 configures the end effector according to the specific interventional mode. For example, for a plurality of puncture surgeries, the end effector is configured as an end gripper to facilitate holding a puncture needle. Further for example, to assist with puncture of the puncture needle, the end effector is configured as an auxiliary positioner. For more content regarding configuring the end effector, refer to step S510.

Step S2930, determining surgical planning information in the interventional mode. Step S2910 refers to step S510.

Step S2940, planning a movement path of the end effector in the interventional mode based on the surgical planning information. In some embodiments, step S2940 is executed by the path planning module 420. Step S2910 refers to step S520.

It should be noted that the above descriptions related to process 500, process 600, process 1100, process 1600-process 1800, process 2000, process 2100, and process 2900 are merely for illustration and explanation, and do not limit the scope of applicability of the present disclosure. For a person skilled in the art, various modifications and alterations may be made to process 500, process 600, process 1100, process 1600-process 1800, process 2000, process 2100, and process 2900 under the guidance of the present disclosure. However, these modifications and alterations are still within the scope of the present disclosure. For example, in process 2000 and process 2100, the CT gantry tilt may be performed only when a cephalic-caudal inclined needle path is detected during surgery. Alternatively, the movement path of the end effector may be planned according to the CT gantry tilt from the beginning, such that adaptive planning may be performed for situations such as cephalic-caudal inclined needle path from the beginning, thereby avoiding adjustment during surgery and reducing the surgery time.

Basic concepts have been described above. Obviously, for a person skilled in the art, the detailed disclosure above is merely by way of example and does not constitute a limitation on the present disclosure. Although not explicitly stated herein, a person skilled in the art may make various modifications, improvements, and amendments to the present disclosure. Such modifications, improvements, and amendments are suggested in the present disclosure, and therefore such modifications, improvements, and amendments are still within the spirit and scope of the exemplary embodiments of the present disclosure.

Meanwhile, the present disclosure uses specific words to describe the embodiments of the present disclosure. Such as 'one embodiment', 'a certain embodiment', and/or 'some embodiments' mean a certain feature, structure, or characteristic related to at least one embodiment of the present disclosure. Therefore, it should be emphasized and noted that 'one embodiment' or 'a certain embodiment' or 'an alternative embodiment' mentioned twice or more in different places in the present disclosure do not necessarily refer to the same embodiment. Furthermore, certain features, structures, or characteristics in one or more embodiments of the present disclosure may be appropriately combined.

Furthermore, unless explicitly stated in the claims, the order of processing elements and sequences, the use of numerals and letters, or the use of other names described in the present disclosure is not intended to limit the order of processes and methods of the present disclosure. Although some inventive embodiments currently considered useful have been discussed in the above disclosure through various examples, it should be understood that such details are merely for illustrative purposes. The appended claims are not limited to the disclosed embodiments. Instead, the claims are intended to cover all modifications and equivalent combinations that fall within the spirit and scope of the embodiments of the present disclosure. For example, although the implementation of various components described above may be embodied in a hardware device, it may also be implemented as a software only solution, e.g., an installation on an existing server or mobile device.

Similarly, it should be noted that to simplify the statements of the present disclosure and to assist in understanding one or more inventive embodiments, various features are sometimes grouped into one embodiment, a figure, or the description thereof in the preceding descriptions of the embodiments of the present disclosure. However, this disclosure manner does not mean that the features required by the subject matter of the present disclosure are more than the features mentioned in the claims. In practice, the features of an embodiment are fewer than all the features of the single embodiment disclosed above.

In some embodiments, numbers describing components and quantities of attributes are used. It should be understood that such numbers for embodiment descriptions use the modifiers 'about', 'approximately', or 'substantially' in some examples to qualify them. Unless otherwise specified, 'about', 'approximately', or 'substantially' indicate that the stated numbers allow for a variation of ±20%. Accordingly, in some embodiments, the numerical parameters used in the present disclosure and the claims are approximate values, and the approximate values may be changed according to the characteristics required by an individual embodiment. In some embodiments, the numerical parameters should consider specified significant digits and adopt a general manner of digit retention. Although numerical ranges and parameters used in some embodiments of the present disclosure for confirming a breadth of scope may be approximate values, in specific embodiments, such values may be set as precisely as possible within a feasible range.

Regarding each patent, patent application, patent application publication, and other materials cited in the present disclosure, such as articles, books, specifications, publications, documents, or the like, the entirety of their content is hereby incorporated by reference into the present disclosure. However, excluded are prosecution history files that are inconsistent with or conflict with the content of the present disclosure. Also excluded are documents (currently or subsequently appended to the present disclosure) that limit the broadest scope of the claims of the present disclosure. It should be noted that, if the descriptions, definitions, and/or usages of terms in materials attached to the present disclosure are inconsistent with or conflict with the content described in the present disclosure, the descriptions, definitions, and/or usages of terms of the present disclosure shall prevail.

Finally, it should be understood that the embodiments described in the present disclosure are merely used to illustrate the principles of the embodiments of the present disclosure. Other variations may also fall within the scope of the present disclosure. Therefore, by way of example and not limitation, alternative configurations of the embodiments of the present disclosure may be considered consistent with the teachings of the present disclosure. Accordingly, the embodiments of the present disclosure are not limited to the embodiments explicitly introduced and described in the present disclosure.

## Claims

1. A system for interventional medicine, comprising: an interventional surgical robot and a processor, wherein the interventional surgical robot includes a manipulator arm and an end effector; and the processor is configured to perform operations including:
obtaining surgical planning information in an interventional mode, wherein the interventional mode is an online mode or an offline mode, and the surgical planning information at least includes a plurality of operation positions; and
planning a movement path of the end effector in the interventional mode based on the surgical planning information.

2. The system of claim 1, wherein the planning the movement path of the end effector in the interventional mode based on the surgical planning information includes:
determining a plurality of subregions for path planning extending along a first direction, wherein the plurality of subregions for path planning cover the plurality of operation positions, and each of the subregions for path planning covers at least a part of the plurality of operation positions; and
planning the movement path of the end effector based on the subregions for path planning.

3. The system of claim 2, wherein the planning the movement path of the end effector based on the subregions for path planning includes:
determining a first sorting result of the plurality of operation positions based on the subregions for path planning; and
planning the movement path of the end effector based at least on the first sorting result.

4. The system of claim 3, wherein the determining the first sorting result of the plurality of operation positions based on the subregions for path planning includes:
sorting the subregions for path planning along a preset direction, wherein the preset direction is parallel to a horizontal plane and perpendicular to the first direction; and
sorting operation positions covered by each of the subregions for path planning.

5. The system of claim 4, wherein the end effector includes an end gripper for holding a puncture needle, and the determining the first sorting result of the plurality of operation positions based on the subregions for path planning further includes:
determining opening directions of the end gripper at the plurality of operation positions; and
determining the preset direction based on the opening directions.

6. The system of claim 4, wherein the sorting the operation positions covered by each of the subregions for path planning includes:
for each of the subregions for path planning, sorting the operation positions covered by the subregion for path planning along the first direction.

7. The system of claim 4, wherein the end effector includes an end gripper for holding a puncture needle, and the sorting the operation positions covered by each of the subregions for path planning includes:
determining opening directions of the end gripper at the operation positions; and
sorting the operation positions covered by the subregion for path planning based on the opening directions.

8. The system of any one of claims 1 to 7, wherein the movement path includes at least one subpath between adjacent operation positions, and the planning the movement path of the end effector in the interventional mode based on the surgical planning information includes:
for each set of adjacent operation positions, planning an avoidance point based on a preceding operation position of the adjacent operation positions, and planning a displacement path segment based on the preceding operation position and the avoidance point, wherein the displacement path segment is a path segment between the preceding operation position and the avoidance point; and
planning an avoidance path segment between the avoidance point and a subsequent operation position of the adjacent operation positions, wherein the avoidance path segment and the displacement path segment constitute the subpath.

9. The system of claim 8, wherein the subpath includes an execution subpath, the avoidance point includes an execution avoidance point, the displacement path segment includes an execution displacement path segment, the avoidance path segment includes an execution avoidance path segment, the execution subpathconsists of the execution displacement path segment and the execution avoidance path segment, and the processor is further configured to perform operations including:
in response to unsuccessful planning of the execution displacement path segment, obtaining a first correction position of the end effector;
planning the execution displacement path segment based on the first correction position and the execution avoidance point; or
re-planning the execution avoidance point based on the first correction position, and planning the execution displacement path segment based on the first correction position and the re-planned execution avoidance point; or
determining the first correction position as the re-planned execution avoidance point; and/or
in response to unsuccessful planning of the execution avoidance path segment, obtaining a second correction position of the end effector;
planning the execution avoidance path segment based on the second correction position and the subsequent operation position of the adjacent operation positions.

10. The system of claim 1, wherein the planning the movement path of the end effector in the interventional mode based on the surgical planning information further includes:
planning a first movement subpath from an initial position of the end effector to a safe position of the end effector based on the initial position and the safe position; and
planning a second movement subpath between the safe position and any one of the plurality of operation positions based on the safe position and the any one operation position.

11. The system of any one of claims 1 to 10, wherein the planning the movement path of the end effector in the interventional mode based on the surgical planning information includes:
planning the movement path based on a collision model, wherein the collision model includes a patient model, a scanning device model, and a manipulator arm end model; or the collision model includes the patient model, the scanning device model, the manipulator arm end model, and a surgical instrument model of a surgical instrument inserted into a body of a patient.

12. The system of any one of claims 1 to 11, wherein in the online mode, the surgical planning information further includes a plurality of working channels corresponding to the plurality of operation positions, the system for interventional medicine is in communication with a medical imaging system including a scanning device and a scanning table, and the planning the movement path of the end effector in the interventional mode based on the surgical planning information includes:
obtaining first position information of the scanning device and second position information of the scanning table;
planning the movement path based on the first position information, the second position information, and the surgical planning information, wherein the movement path includes a verification path; and
in response to successful planning of the movement path of the end effector, determining target surgical planning information, wherein the target surgical planning information includes a plurality of target operation positions.

13. The system of claim 12, wherein the movement path further includes an execution path, the execution path includes at least one execution subpath, and the processor is further configured to perform operations including:
determining a second sorting result of the plurality of target operation positions;
planning and executing an execution subpath between each set of adjacent target operation positions in the second sorting result in sequence, wherein at least a part of each execution subpath is executed within a scanning chamber of the scanning device.

14. The system of claim 13, wherein the planning and executing the execution subpath between each set of adjacent target operation positions in the second sorting result in sequence includes:
in response to an inclination of a target operation channel corresponding to a target operation position included in the execution subpath relative to a second direction,
controlling the scanning device, the scanning table, and the manipulator arm to operate in coordination to execute the execution subpath.

15. The system of claim 14, wherein the controlling the scanning device, the scanning table, and the manipulator arm to operate in coordination to execute the execution subpath includes:
in response to an inclination of a subsequent target working channel corresponding to a subsequent target operation position of the adjacent target operation positions relative to the second direction, controlling the manipulator arm and the end effector to exit the scanning chamber of the scanning device from a preceding target operation position of the adjacent target operation positions to a safe position of the end effector, wherein the second direction is parallel to a longitudinal direction of the scanning table;
controlling the scanning table to exit the scanning chamber of the scanning device, then controlling the scanning device to rotate toward the second direction, such that the scanning chamber of the scanning device tilts at a second angle relative to the second direction, wherein the second angle is an inclination angle of the subsequent target working channel relative to the second direction;
controlling the scanning table to move at least along the second direction to enter the scanning chamber of the scanning device, such that the subsequent target working channel is within a scanning range of the scanning device; and
planning and controlling the manipulator arm and the end effector to execute the execution subpath from the safe position to the subsequent target operation position.

16. The system of claim 14, wherein the controlling the scanning device, the scanning table, and the manipulator arm to operate in coordination to execute the execution subpath includes:
in response to an inclination of a target working channel corresponding to a first target operation position in the second sorting result relative to the second direction, controlling the scanning device to rotate toward the second direction, such that the scanning chamber of the scanning device tilts at a first angle relative to the second direction, wherein the first angle corresponds to an inclination angle of the target working channel corresponding to the first target operation position relative to the second direction, and the second direction is parallel to a longitudinal direction of the scanning table;
controlling the scanning table to move at least along the second direction to enter the scanning chamber of the scanning device, such that the target working channel corresponding to the first target operation positin is within a scanning range of the scanning device; and
planning and controlling the manipulator arm and the end effector to execute the execution subpath from a safe position of the end effector to the first target operation position.

17. The system of claim 14, wherein the controlling the scanning device, the scanning table, and the manipulator arm to operate in coordination to execute the execution subpath includes:
in response to an inclination of a subsequent target working channel corresponding to a subsequent target operation position of the adjacent target operation positions relative to the second direction, and that a preceding target working channel corresponding to a preceding target operation position of the adjacent target operation positions and the subsequent target working channel are located in a same scanning layer, and an inclination angle of the preceding target working channel relative to the second direction is different from an inclination angle of the subsequent target working channel relative to the second direction,
controlling the scanning device to rotate toward the second direction, such that the scanning chamber of the scanning device tilts at a second angle relative to the second direction, and the subsequent target working channel is within a scanning range of the scanning device, the second angle is the inclination angle of the subsequent target working channel relative to the second direction, and the second direction is parallel to a longitudinal direction of the scanning table; and
planning and controlling the manipulator arm and the end effector to execute the execution subpath from the preceding target working position to the subsequent target working position.

18. The system of claim 15 or 17, wherein the controlling the scanning device, the scanning table, and the manipulator arm to operate in coordination to execute the execution subpath further includes:
before the controlling the scanning device to rotate toward the second direction, such that the scanning chamber of the scanning device tilts at the second angle relative to the second direction, controlling the scanning device to perform a gantry homing operation.

19. The system of claim 14, wherein the controlling the scanning device, the scanning table, and the manipulator arm to operate in coordination to execute the execution subpath includes:
in response to an inclination of a subsequent target working channel corresponding to a subsequent target operation position of the adjacent target operation positions relative to the second direction, and that a preceding target working channel corresponding to a preceding target operation position of the adjacent target operation positions and the subsequent target working channel are located in a same scanning layer,
planning and controlling the manipulator arm and the end effector to execute the execution subpath from the preceding target working position to the subsequent target working position, wherein the second direction is parallel to a longitudinal direction of the scanning table.

20. The system of any of claims 15-17 and claim 11, wherein the controlling the scanning device, the scanning table, and the manipulator arm to operate in coordination to execute the execution subpath includes:
after controlling the scanning device to rotate and/or controlling the scanning table to move, updating a collision model and position information of the subsequent target working position; and
planning the execution subpath based on the updated collision model and the updated position information of the subsequent target working position.

21. The system of any one of claims 1-11, wherein in the online mode, the surgical planning information further includes a plurality of working channels corresponding to the plurality of operation positions, the system for interventional medicine is in communication with a medical imaging system including a scanning device and a scanning table, and the planning the movement path of the end effector in the interventional mode based on the surgical planning information includes:
obtaining first position information of the scanning device and second position information of the scanning table; and
planning and executing, based on the first position information, the second position information, and the surgical planning information, an execution subpath between each set of adjacent target operation positions in sequence, wherein one or more execution subpaths constitute an execution path, and the planning and executing the execution subpath between each set of the adjacent target operation positions includes:
in response to an inclination of a target working channel corresponding to a target operation position included in the execution subpath relative to a second direction, controlling the scanning device, the scanning table, and the manipulator arm to operate in coordination to execute the execution subpath.

22. The system of claim 21, wherein the controlling the scanning device, the scanning table, and the manipulator arm to operate in coordination to execute the execution subpath includes:
in response to an inclination of a subsequent target working channel corresponding to a subsequent target operation position of the adjacent target operation positions relative to the second direction, controlling the manipulator arm and the end effector to exit a scanning chamber of the scanning device from a preceding target operation position of the adjacent target operation positions to a safe position of the end effector, wherein the second direction is parallel to a longitudinal direction of the scanning table;
controlling the scanning table to exit the scanning chamber of the scanning device, then controlling the scanning device to rotate toward the second direction, such that the scanning chamber of the scanning device tilts at a second angle relative to the second direction, wherein the second angle is an inclination angle of the subsequent target working channel relative to the second direction;
controlling the scanning table to move at least along the second direction to enter the scanning chamber of the scanning device, such that the subsequent target working channel is within a scanning range of the scanning device; and
planning and controlling the manipulator arm and the end effector to execute the execution subpath from the safe position to the subsequent target operation position.

23. The system of claim 21, wherein the controlling the scanning device, the scanning table, and the manipulator arm to operate in coordination to execute the execution subpath includes:
in response to an inclination of a target working channel corresponding to a first target operation position in the target surgical planning information relative to the second direction, controlling the scanning device to rotate toward the second direction, such that the scanning chamber of the scanning device tilts at a first angle relative to the second direction, wherein the first angle is an inclination angle of the target working channel relative to the second direction, and the second direction is parallel to a longitudinal direction of the scanning table;
controlling the scanning table to move at least along the second direction to enter the scanning chamber of the scanning device, such that the target working channel is within a scanning range of the scanning device; and
planning and controlling the manipulator arm and the end effector to execute the execution subpath from a safe position of the end effector to the first target operation position.

24. The system of claim 21, wherein the controlling the scanning device, the scanning table, and the manipulator arm to operate in coordination to execute the execution subpath includes:
in response to an inclination of a subsequent target working channel corresponding to a subsequent target operation position of the adjacent target operation positions relative to the second direction, and that a preceding target working channel corresponding to a preceding target operation position of the adjacent target operation positions and the subsequent target working channel are located in a same scanning layer, and an inclination angle of the preceding target working channel relative to the second direction is different from an inclination angle of the subsequent target working channel relative to the second direction,
controlling the scanning device to rotate toward the second direction, such that a scanning chamber of the scanning device tilts at a second angle relative to the second direction, and the subsequent target working channel is within a scanning range of the scanning device, the second angle is the inclination angle of the subsequent target working channel relative to the second direction, and the second direction is parallel to a longitudinal direction of the scanning table; and
planning and controlling the manipulator arm and the end effector to execute the execution subpath from the preceding target working position to the subsequent target working position.

25. The system of claim 22 or 24, wherein the controlling the scanning device, the scanning table, and the manipulator arm to operate in coordination to execute the execution subpath includes:
before the controlling the scanning device to rotate toward the second direction, such that the scanning chamber of the scanning device tilts at the second angle relative to the second direction, controlling the scanning device to perform a gantry homing operation.

26. The system of claim 21, wherein the controlling the scanning device, the scanning table, and the manipulator arm to operate in coordination to execute the execution subpath includes:
in response to an inclination of a subsequent target working channel corresponding to a subsequent target operation position of the adjacent target operation positions relative to the second direction, and that a preceding target working channel corresponding to a preceding target operation position of the adjacent target operation positions and the subsequent target working channel are located in a same scanning layer,
planning and controlling the manipulator arm and the end effector to execute the execution subpath from the preceding target working position to the subsequent target working position, wherein the second direction is parallel to a longitudinal direction of the scanning table.

27. The system of claims 11 and 21, wherein the controlling the scanning device, the scanning table, and the manipulator arm to operate in coordination to execute the execution subpath includes:
determining a collision model based on the first position information and the second position information.

28. The system of any one of claims 22-24 and claim 27, wherein the controlling the scanning device, the scanning table, and the manipulator arm to operate in coordination to execute the execution subpath includes:
after controlling the scanning device to rotate and/or controlling the scanning table to move, updating a collision model and position information of the subsequent target working position; and
planning the execution subpath based on the updated collision model and the updated position information of the subsequent target working position.

29. The system of claim 13 or claim 21, wherein the online mode includes a real-time control mode, the execution path is planned further based on a real-time image, and the real-time image is an image obtained by keeping the scanning device continuously turned on during an interventional surgical procedure.

30. The system of claim 13 or claim 21, wherein the online mode includes a breakpoint control mode, the execution path is planned further based on a breakpoint image, and the breakpoint image is an image obtained by intermittently turning on the scanning device during an interventional surgical procedure.

31. The system of claim 13 or claim 21, wherein the online mode includes a real-time control mode and a breakpoint control mode, and the real-time control mode and the breakpoint control mode are switchable.

32. The system of claim 12, wherein the processor is further configured to perform operations including:
in response to a failure in planning the movement path of the end effector, outputting a prompt message;
re-obtaining at least one of the second position information of the scanning table or the surgical planning information, wherein the re-obtained surgical planning information includes at least one adjusted operation position and an adjusted working channel corresponding to the at least one adjusted operation position; and
re-planning the movement path of the end effector based on the re-obtained second position information and/or the re-obtained surgical planning information.

33. The system of claim 1, wherein in the offline mode, the processor is further configured to perform operations including:
in response to a failure in planning the movement path of the end effector, outputting a prompt message;
re-obtaining the surgical planning information, wherein the re-obtained surgical planning information includes at least one adjusted operation position and an adjusted working channel corresponding to the at least one adjusted operation position; and
re-planning the movement path of the end effector based on the re-obtained surgical planning information.

34. The system of claim 32 or 33, wherein the prompt message includes at least one of following prompt messages:
a first prompt message, wherein the first prompt message is output in response to a path planning failure caused by a collision between a manipulator arm end model and a patient model, and the first prompt message at least indicates to a user to adjust the plurality of operation positions;
a second prompt message, wherein the second prompt message is output in response to a path planning failure caused by a collision between the manipulator arm end model and a scanning device model, and the second prompt message indicates to the user to adjust the plurality of operation positions or adjust a position of a scanning table;
a third prompt message, wherein the third prompt message is output in response to a path planning failure caused by a collision between the manipulator arm end model and a surgical instrument model, and the third prompt message at least indicates to the user to adjust the plurality of operation positions along a longitudinal direction or a radial direction of the scanning table.

35. A method, implemented on a system for interventional medicine including an interventional surgical robot and a processor, the interventional surgical robot including a manipulator arm and an end effector, the method comprising:
obtaining surgical planning information in an interventional mode, wherein the interventional mode is an online mode or an offline mode, and the surgical planning information at least includes a plurality of operation positions; and
planning a movement path of the end effector in the interventional mode based on the surgical planning information.

36. A controlling method implemented on a system for interventional medicine including an interventional surgical robot and a processor, the interventional surgical robot including a manipulator arm and an end effector, the method comprising:
determining an interventional mode of the system for interventional medicine;
configuring the end effector based on the interventional mode;
determining surgical planning information in the interventional mode; and
planning a movement path of the end effector in the interventional mode based on the surgical planning information.

37. A method for path planning of an interventional surgical robot, the interventional surgical robot including a manipulator arm and an end effector connected to an end of the manipulator arm, the method comprising:
obtaining a plurality of operation positions from surgical planning information;
determining a plurality of subregions for path planning extending along a first direction, wherein the plurality of subregions for path planning fully cover the plurality of operation positions, and each of the subregions for path planning covers at least a part of the plurality of operation positions; and
planning a movement path of the end effector based on the plurality of subregions for path planning.

38. A system for interventional medicine, in communication with a medical imaging system including a scanning device and a scanning table, the system for interventional medicine comprising:
an interventional surgical robot and a processor, wherein the interventional surgical robot includes a manipulator arm and an end effector, and the processor is configured to perform operations including:
obtaining first position information of the scanning device, second position information of the scanning table, and surgical planning information, wherein the surgical planning information includes a plurality of operation positions and a plurality of working channels corresponding to the plurality of operation positions;
planning a movement path of the end effector based on the first position information, the second position information, and the surgical planning information; and
determining target surgical planning information in response to successful planning of the movement path of the end effector.

39. A system for interventional medicine, in communication with a medical imaging system including a scanning device and a scanning table, the system for interventional medicine comprising:
an interventional surgical robot and a processor, wherein the interventional surgical robot includes a manipulator arm and an end effector, and the processor is configured to perform operations including:
obtaining first position information of the scanning device, second position information of the scanning table, and target surgical planning information to be executed, wherein the target surgical planning information includes a plurality of target operation positions and a plurality of target working channels corresponding to the plurality of target operation positions;
planning and executing an execution subpath between each set of adjacent target operation positions in sequence, wherein at least a portion of each execution subpath is executed within a scanning chamber of the scanning device, and the planning and executing the execution subpath between each set of the adjacent target operation positions in sequence includes:
in response to an inclination of a target working channel corresponding to a target operation position included in the execution subpath relative to a second direction,
controlling the scanning device, the scanning table, and the manipulator arm to operate in coordination to execute the execution subpath.
